# EUROPEAN PATENT SPECIFICATION

(11) **EP 1 523 372 B1**
(45) Date of publication and mention of the grant of the patent: **02.09.2009**
(21) Application number: 03765046.2
(22) Date of filing: 18.07.2003
(51) Int. Cl.: A61K 38/25, A61K 39/395, A61P 3/04

(54) **GHRELIN-CARRIER CONJUGATES**
GHRELIN-TRÄGER-KONJUGATE
CONJUGUES DE PORTEURS DE GHRELIN

(30) Priority: 19.07.2002 US 396638 P
(43) Date of publication of application: 20.04.2005
(73) Proprietor: Cytos Biotechnology AG, 8952 Schlieren (CH)
(72) Inventor: BACHMANN, Martin, F., CH-8472 Seuzach (CH); FULURIJA, Alma, CH-8952 Schlieren (CH)
(74) Representative: Wichmann, Hendrik
(86) International application number: PCT/EP2003/007849
(87) International publication number: WO 2004/009124

(56) References cited:
- EP-A- 1 197 496
- WO-A-00/32227
- WO-A-01/85208
- WO-A-01/92292
- WO-A-03/024481
- WO-A-03/031466
- WO-A-03/039225
- WO-A-03/051389
- US-A1- 2001 038 862
- CHACKERIAN B ET AL: "Induction of autoantibodies to mouse CCR5 with recombinant papillomavirus particles" PROCEEDINGS OF THE NATIONAL ACADEMY OF SCIENCES OF USA, NATIONAL ACADEMY OF SCIENCE. WASHINGTON, US, vol. 96, no. 5, 2 March 1999 (1999-03-02), pages 2373-2378, XP002133522 ISSN: 0027-8424
- LECHNER FRANZISKA ET AL: "Virus-like particles as a modular system for novel vaccines." INTERVIROLOGY. SWITZERLAND 2002, vol. 45, no. 4-6, 2002, pages 212-217, XP008023830 ISSN: 0300-5526
- MURAKAMI N ET AL: "Role for central ghrelin in food intake and secretion profile of stomach ghrelin in rats." THE JOURNAL OF ENDOCRINOLOGY. ENGLAND AUG 2002, vol. 174, no. 2, August 2002 (2002-08), pages 283-288, XP009022559 ISSN: 0022-0795
- JEGERLEHNER A ET AL: "A molecular assembly system that renders antigens of choice highly repetitive for induction of protective B cell responses" VACCINE, BUTTERWORTH SCIENTIFIC. GUILDFORD, GB, vol. 20, no. 25-26, 19 August 2002 (2002-08-19), pages 3104-3112, XP004374549 ISSN: 0264-410X
- TSCHOP M ET AL: "GHRELIN INDUCES ADIPOSITY IN RODENTS" NATURE, MACMILLAN JOURNALS LTD. LONDON, GB, vol. 407, 19 October 2000 (2000-10-19), pages 908-913, XP002951587 ISSN: 0028-0836 cited in the application
- MASAMITSU NAKAZATO ET AL: "A role for ghrelin in the central regulation of feeding" NATURE, MACMILLAN JOURNALS LTD. LONDON, GB, vol. 409, 11 January 2001 (2001-01-11), pages 194-198, XP002901678 ISSN: 0028-0836 cited in the application
- BEDNAREK ET AL: "Structure-Function Studies on the New Growth Hormone-Releasing Peptide, Ghrelin: Minimal Sequence of Ghrelin Necessary for Activation of Growth Hormone Secretagogue Receptor 1a" JOURNAL OF MEDICINAL CHEMISTRY, AMERICAN CHEMICAL SOCIETY. WASHINGTON, US, vol. 43, no. 23, 26 October 2000 (2000-10-26), pages 4370-4376, XP002166937 ISSN: 0022-2623
- KOJIMA MASAYASU ET AL: "Ghrelin is a growth-hormone-releasing acylated peptide from stomach" NATURE, MACMILLAN JOURNALS LTD. LONDON, GB, vol. 402, 9 December 1999 (1999-12-09), pages 656-660, XP002166936 ISSN: 0028-0836 cited in the application
- CUMMINGS DAVID E ET AL: "Elevated plasma ghrelin levels in Prader Willi syndrome." NATURE MEDICINE. UNITED STATES JUL 2002, vol. 8, no. 7, July 2002 (2002-07), pages 643-644, XP002265242 ISSN: 1078-8956
- HINNEY ANKE ET AL: "Ghrelin gene: identification of missense variants and a frameshift mutation in extremely obese children and adolescents and healthy normal weight students." THE JOURNAL OF CLINICAL ENDOCRINOLOGY AND METABOLISM. UNITED STATES JUN 2002, vol. 87, no. 6, June 2002 (2002-06), pages 2716-2719, XP002265243 ISSN: 0021-972X
- WREN A M ET AL: "Ghrelin enhances appetite and increases food intake in humans." THE JOURNAL OF CLINICAL ENDOCRINOLOGY AND METABOLISM. UNITED STATES DEC 2001, vol. 86, no. 12, December 2001 (2001-12), pages 5992-5995, XP002265244 ISSN: 0021-972X
- HOSODA H ET AL: "PURIFICATION AND CHARACTERIZATION OF RAT DES-GLN14-GHRELIN, A SECOND ENDOGENOUS LIGAND FOR THE GROWTH HORMONE SECRETAGOGUE RECEPTOR" JOURNAL OF BIOLOGICAL CHEMISTRY, AMERICAN SOCIETY OF BIOLOGICAL CHEMISTS, BALTIMORE, MD, US, vol. 275, no. 29, July 2000 (2000-07), pages 21995-22000, XP002932624 ISSN: 0021-9258 cited in the application

## Description

### BACKGROUND OF THE INVENTION

### Field of the Invention

The present invention is related to the fields of molecular biology, virology, immunology and medicine. The invention provides a composition comprising an ordered and repetitive antigen or antigenic determinant array, and in particular a ghrelin or ghrelin-derived peptide-array. More specifically, the invention provides a composition comprising a virus-like particle and at least one ghrelin or a ghrelin-derived peptide bound thereto.

The invention also provides a process for producing the conjugates and the ordered and repetitive arrays, respectively. The compositions of the invention are useful in the production of vaccines for the treatment of obesity and other disease associated with increased food-uptake or increased body weight and to efficiently induce immune responses, in particular antibody responses. Furthermore, the compositions of the invention are particularly useful to efficiently induce self-specific immune responses within the indicated context.

### Related Art

Obesity is a disease afflicting millions of people world-wide. Although the underlying causes for obesity may be manifold, a common reason for almost all forms of obesity is increased food-uptake. Many factors regulate hunger and feeding behaviour, including leptin, growth-hormone (GH), neuropeptide Y (NPY), agouti-related protein (AGRP) and others. A recently identified key regulator of feeding behaviour is ghrelin, an acylated peptide produced in the stomach and also some parts of the brain (hypothalamus) (Kojima et al. Nature 402: 656-660 (1999)). Ghrelin is derived by encymatic cleavage from a prepro form encompassing 117 amino acids resulting in a 28 amino acid long peptide with a n-octanoylation at serine 3. Biologically active ghrelin needs to be n-octanyolated at this position. A second, 27 aa isoform of ghrelin (Ghrelin-desQ14), lacking a glutamine (Q) at position 14, has been identified, however, this isoform represents only a minor component of circulating ghrelin. Like full length ghrelin, the biological activity of Ghrelin-des-Q14 is dependant on the n-octanoyl group on serine 3. Nevertheless, most functional activity is derived from the 28 aa ghrelin isoform (Hosoda et al. Biochem. Biophys. Res. Commun. 279(3): 909-913 (2000)). Ghrelin is highly conserved, since human and rat ghrelin differ by only 2 amino-acids (GSSFLSPEHQRVQQRKESKKPPAKLQPR (SEQ ID NO: 31) versus GSSFLSPEHQKAQQ-RKESKKPPAKLQPR (SEQ ID NO: 32)) (Kojima et al. Nature 402: 656-660 (1999)).

Receptors for ghrelin (GHS-R) are expressed in various regions of the brain, including the arcuate nucleus (Arc) and ventromedial nucleus of the hypothalamus and in the pituitary gland (Howard et al. Science 273:974-977 (1996)); McKee et al. Mol Endokrin. 11:415-423 (1997); Guan et al., Mol brain research 48:23-29 (1997)), indicating that ghrelin primarily acts in the brain. In addition to stimulating release of GH from the pituitary gland (Kojima et al. Nature 402: 656-660 (1999)), ghrelin has more recently been identified as a key central regulator of feeding (Nakazato et al., Nature 409: 194-198 (2001)). Specifically, upon intracerebroventricular application, ghrelin was shown to stimulate feeding. Moreover, intracerebroventricular application of anti-ghrelin antibodies inhibited feeding. Ghrelin injection induced upregulated release of NPY and anti-NPY antibodies together with AGRP antagonists blocked ghrelin induced feeding, suggesting that ghrelin modulates feeding via enhancing expression of NPY and AGRP (Nakazato et al., Nature 409: 194-198 (2001)). Moreover, peripheral daily administration of ghrelin induced body weight gain in mice and rats and serum ghrelin concentrations were increased in fasting rats and reduced by feeding, further suggestin that ghrelin plays a key role in regulating feeding (Tschop et al, Nature 407:908-912). Transgenic rats expressing anti-sense GHS-R RNA in the Arc exhibited lower body weight and less adipose tissue, supporting the notion that ghrelin regulates body weight (Shuto et al., JCI 109:14291436 (2002)).There is also evidence for a key role for ghrelin in human feeding behaviour. Peripheral administration of ghrelin in humans enhanced appetite and increased food uptake in humans (Wren et al., J Clin Endocrinol Metab 86: 5992-5998 (2001)). Humans with Prader-Willi syndrome, the most common form of human syndromic obesity, exhibit highly increased ghrelin levels (Cummings et al. Nat Med 8:643-644 (2002)). In addition, plasma ghrelin levels in humans are strongly increased after diet-induced weight loss, correlating with rapid regain of weight when people stop the diet. In contrast, in patients with gastric bypass surgery, ghrelin levels remained low during and after diet and patients do not usually regain their weight under these conditions ((Cummings et al., N Engl J Med 21: 1623-1630 (2002)). Hence, ghrelin appears to be a key regulator of food uptake and body weight in humans.

Since peripheral administration of ghrelin was able to increase food uptake leading to increased body weight (Tschop et al, Nature 407:908-912), it is likely that ghrelin produced in the stomach reaches the brain through the blood stream and triggers feeding. Thus, it may be possible to block migration of ghrelin from the blood to the brain to stop food uptake in animals and humans. As it has been shown that specific antibodies can block ghrelin action in the brain (Nakazato et al., Nature 409: 194-198 (2001)) it is likely that peripheral antibodies will also be able to block the action of peripheral ghrelin. In addition, since antibodies inefficiently penetrate the blood brain barrier, ghrelin-specific antibodies would probably be able to seclude ghrelin from the brain but would not act on ghrelin within the brain. This would be a particularly attractive possibility, since ghrelin is also produced in the brain where it probably exerts functions different from regulating food uptake (Nakazato et al., Nature 409: 194-198 (2001)). Therefore, a potential therapy for obesity would be to induce ghrelin-specific antibodies in the host, leading to the long-term blockage obstruction of ghrelin resulting in reduced food-uptkake, similarly to that observed in gastric bypass patients.

WO98/42840 discloses the influence of ghrelin and ghrelin-derived fragments on the gastrointestinal tract and hereby in particular their effect on gastric motility and gastric emptying. Moreover, US 6'420'521 discloses the use of short ghrelin peptides for effects on gastric function, including gastric emptying, gastric contractility and glucose absorption.

However, it is usually difficult to induce antibody responses against peptides, in particular against self-peptides. One way to improve the efficiency of vaccination is to increase the degree of repetitiveness of the antigen applied. Unlike isolated proteins, viruses induce prompt and efficient immune responses in the absence of any adjuvants both with and without T -cell help (Bachmann and Zinkernagel, Ann. Rev. Immunol: 15:235-270 (1991)). Although viruses often consist of few proteins, they are able to trigger much stronger immune responses than their isolated components. For B-cell responses, it is known that one crucial factor for the immunogenicity of viruses is the repetitiveness and order of surface epitopes. Many viruses exhibit a quasi-crystalline surface that displays a regular array of epitopes which efficiently crosslinks epitope-specific immunoglobulins on B cells (Bachmann and Zinkernagel, Immunol. Today 17:553-558 (1996)). This crosslinking of surface immunoglobulins on B cells is a strong activation signal that directly induces cell-cycle progression and the production of IgM antibodies. Further, such triggered B cells are able to activate T helper cells, which in turn induce a switch from IgM to IgG antibody production in B cells and the generation of long-lived B cell memory - the goal of any vaccination (Bachmann and Zinkernagel, Ann. Rev. Immunol. 15:235-270 (1997)). Viral structure is even linked to the generation of anti-antibodies in autoimmune disease and as a part of the natural response to pathogens (see Fehr, T., et al., J Exp. Med. 185:1785-1792 (1997)). Thus, antibodies presented by a highly organized viral surface are able to induce strong anti-antibody responses.

As indicated, however, the immune system usually fails to produce antibodies against self-derived structures. For soluble antigens present at low concentrations, this is due to tolerance at the Th cell level. Under these conditions, coupling the self-antigen to a carrier that can deliver T help may break tolerance. For soluble proteins present at high concentrations or membrane proteins at low concentration, B and Th cells may be tolerant. However, B cell tolerance may be reversible (anergy) and can be broken by administration of the antigen in a highly organized fashion coupled to a foreign carrier (Bachmann and Zinkernagel, Ann. Rev. Immunol. 15:235-270 (1997)).

### BRIEF SUMMARY OF THE INVENTION

We have found that ghrelin or ghrelin peptides, which are bound to a core particle having a structure with an inherent repetitive organization, and hereby in particular to virus-like-particles (VLPs) and subunits of VLPs, respectively, leading to highly ordered and repetitive conjugates represent potent immunogens for the induction of specific antibodies. The present invention provides a prophylactic and therapeutic mean for the treatment of obesity and related diseases, which is based on an ordered and repetitive ghrelin or ghrelin-derived peptide core particle array and in particular on a VLP-ghrelin/ghrelin peptide-conjugate and -array, respectively. This prophylactic and therapeutic composition is able to induce high titers of anti-ghrelin antibodies in a vaccinated animal or human. Therefore, the present invention focuses on ghrelin and its brain-related properties. The present invention, moreover, focuses on the central effects of ghrelin in the brain, more importantly the regulation of appetite, growth hormone secretion and energy homeostasis. Hence, it is preferred that the antibodies induced by our vaccination strategy are able to bind the n-octanoylated form(s) of ghrelin. As indicated, ghrelin or shorter ghrelin peptide fragments could be used, when coupled to a core particle, and alternatively or preferably administered in adjuvant, to induce ghrelin-specific antibodies in humans and in animals.

Therefore, short peptide fragments of ghrelin, particularly the shorter peptides of residues 24-33, 42-51, 31-41 and 28-37, coupled either C- or N-terminally to a core particle or a virus-like particle, respectively, are capable of inducing highly specific anti-ghrelin antibodies being capable of neutralizing peripheral circulating ghrelin before it entered the CNS and exerted an effect on growth hormone and hence, food intake.

In a preferred embodiment of the present invention, thus, the antigen or antigenic determinant is selected from the group of ghrelin peptides corresponding to residues of 24-33, 42-51, 31-41 and 28-37 of any of the sequences set forth in SEQ ID NO: 144 to 146, wherein said preferred ghrelin peptide fragments are selected from the group consisting of human ghrelin; (b) bovine ghrelin; (c) sheep ghrelin; (d) dog ghrelin; (e) cat ghrelin; (f) mouse ghrelin; (g) pig ghrelin; and (h) horse ghrelin.

More specifically, in the present invention, we were able to induce high levels of antibodies that recognize, surprisingly, the *n*-octanoylated form of ghrelin as shown herein, and in particular in example 17. Furthermore, generated antibodies also recognized the alternative isorform, Ghrelin-desQ14. As a result, antibodies generated from vaccination with C- or N-terminally linked ghrelin and ghrelin peptide, respectively, to a core particle or, preferably to a VLP, were able to block the entry of n-octanoylated ghrelin into the brain and modulated food intake in mice. Therefore, the present invention focuses on vaccination strategies against active ghrelin as a treatment for obesity and other related diseases.

As shown herein, and in particular in example 18, vaccination with C- or N-terminally linked ghrelin and ghrelin peptide, respectively, to a core particle or, preferably to a VLP, leads to reduced food uptake in mice, suggesting that ghrelin and ghrelin peptide, respectively, is a key regulator of food intake and antibodies that target ghrelin and ghrelin peptide, respectively, is supposed to be a potential therapy for obesity and other related diseases.

The present invention, thus, provides for a composition comprising: (a) a core particle with at least one first attachment site wherein said core particle is a virus-like particle of an RNA-phage; and (b) at least one antigen or antigenic determinant with at least one second attachment site, wherein said antigen or antigenic determinant is ghrelin or a ghrelin peptide wherein the ghrelin peptide contains at least five consecutive amino acids of ghrelin, and wherein said second attachment site being selected from the group consisting of (i) an attachment site not naturally occurring with said antigen or antigenic determinant; and (ii) an attachment site naturally occurring with said antigen or antigenic determinant, wherein said second attachment site is capable of association to said first attachment site; and wherein said antigen or antigenic determinant and said core particle interact through said association to form an ordered and repetitive antigen array. Alternative preferred embodiments of core particles suitable for use in the present invention are recombinant proteins, a bacteriophage, a virus-like particle of a RNA-phage, a bacterial pilus or flagella or any other core particle having an inherent repetitive structure capable of forming an ordered and repetitive antigen array in accordance with the present invention.

More specifically, the invention provides a composition comprising an ordered and repetitive antigen or antigenic determinant array, and hereby in particular ghrelin or ghrelin-derived peptide VLP conjugates. More specifically, the invention provides a composition comprising a virus-like particle and at least one ghrelin or ghrelin-derived peptide bound thereto. The invention also provides a process for producing the conjugates and the ordered and repetitive arrays, respectively. The compositions of the invention are useful in the production of vaccines for the treatment of obesity and related diseases and as a pharmaccine to prevent or cure obesity and related diseases and to efficiently induce immune responses, in particular antibody responses. Furthermore, the compositions of the invention are particularly useful to efficiently induce self-specific immune responses within the indicated context

In the present invention, ghrelin or a ghrelin peptide is bound to a core particle and VLP, respectively, typically in an oriented manner, yielding an ordered and repetitive ghrelin or ghrelin-derived peptide antigen array. Furthermore, the highly repetitive and organized structure of the core particles and VLPs, respectively, mediates the display of the ghrelin or ghrelin peptide in a highly ordered and repetitive fashion leading to a highly organized and repetitive antigen array. Furthermore, binding of the ghrelin or ghrelin peptide to the core particle and VLP, respectively, provides T helper cell epitopes, since the core particle and VLP is foreign to the host immunized with the core particle-ghrelin or ghrelin-derived peptide array and VLP-ghrelin or ghrelin peptide array, respectively. Those arrays differ from prior art conjugates, in particular, in their highly organized structure, dimensions, and in the repetitiveness of the antigen on the surface of the array.

In one aspect of the invention, the ghrelin or ghrelin peptide is expressed in a suitable expression host, or synthesized, while the core particle and the VLP, repespectively, is expressed and purified from an expression host suitable for the folding and assembly of the core particle and the VLP, respectively. Ghrelin or ghrelin peptides may be chemically synthesized. Since biologically active ghrelin contains a n-octanyolated serine at position three, chemical synthesis will be the preferred way of producing ghrelin for a vaccine formulation containing biologically active forms of ghrelin. The ghrelin or ghrelin peptide array is then assembled by binding the ghrelin or ghrelin peptide to the core particle and the VLP, respectively.

In another aspect, the present invention provides for a composition comprising (a) a virus-like particle, and (b) at least one antigen or antigenic determinant, wherein said antigen or said antigenic determinant is ghrelin or a ghrelin peptide, and wherein said at least one antigen or antigenic determinant is bound to said virus-like particle.

In a further aspect, the present invention provides for a pharmaceutical composition comprising (a) the inventive composition, and (b) an acceptable pharmaceutical carrier.

In still a further aspect, the present invention provides for a vaccine composition comprising a composition comprising: (a) a core particle with at least one first attachment site; and (b) at least one antigen or antigenic determinant with at least one second attachment site, wherein said antigen or antigenic determinant is ghrelin or a ghrelin peptide, and wherein said second attachment site being selected from the group consisting of (i) an attachment site not naturally occurring with said antigen or antigenic determinant; and (ii) an attachment site naturally occurring with said antigen or antigenic determinant, wherein said second attachment site is capable of association to said first attachment site; and wherein said antigen or antigenic determinant and said core particle interact through said association to form an ordered and repetitive antigen array.

In a further aspect, the present invention provides for a vaccine composition comprising a composition, wherein said composition comprising (a) a virus-like particle; and (b) at least one antigen or antigenic determinant, wherein said antigen or said antigenic determinant is ghrelin or a ghrelin peptide; and wherein said at least one antigen or antigenic determinant is bound to said virus-like particle.

In still a further aspect, the present invention provides for a process for producing a composition of claim 1 comprising (a) providing a virus-like particle; and (b) providing at least one antigen or antigenic determinant, wherein said antigen or said antigenic determinant is ghrelin or a ghrelin peptide; (c) combining said virus-like particle and said at least one antigen or antigenic determinant so that said at least one said antigen or antigenic determinant is bound to said virus-like particle.

Analogously, the present invention provides a process for producing an inventive composition comprising: (a) providing a core particle with at least one first attachment site; (b) providing at least one antigen or antigenic determinant with at least one second attachment site, wherein said antigen or antigenic determinant is ghrelin or a ghrelin peptide, and wherein said second attachment site being selected from the group consisting of (i) an attachment site not naturally occurring with said antigen or antigenic determinant; and (ii) an attachment site naturally occurring with said antigen or antigenic determinant; and wherein said second attachment site is capable of association to said first attachment site; and (c) combining said core particle and said at least one antigen or antigenic determinant, wherein said antigen or antigenic determinant and said core particle interact through said association to form an ordered and repetitive antigen array.

In a further aspect, the present invention provides for a use of a composition of claim 1 for the manufacture of a medicament for treatment of obesity or a related disease.

In a still further aspect, the present invention provides for a use of a composition of claim 1 for the preparation of a medicament for the therapeutic or prophylactic treatment of obesity or a related disease. Furthermore, in a still further aspect, the present invention provides for a use of a composition of claim 1, either in isolation or in combination with other agents, for the manufacture of a composition, vaccine, drug or medicament for therapy or prophylaxis of obesity or a related disease, and/or for stimulating the mammalian immune system.

Therefore, the invention provides, in particular, vaccine compositions which are suitable for preventing and/or reducing or curing obesity or conditions related thereto. The invention further provides immunization and vaccination methods, respectively, for preventing and/or reducing or curing obesity or conditions related thereto, in animals, and in particular in pets such as cats or dogs as well as in humans. The inventive compositions may be used prophylactically or therapeutically.

In specific embodiments, the invention provides methods for preventing, curing and/or attenuating obesity or conditions related thereto which are caused or exacerbated by "self" gene products, i.e. "self antigens" as used herein. In related embodiments, the invention provides methods for inducing immunological responses in animals and individuals, respectively, which lead to the production of antibodies that prevent, cure and/or attenuate obesity or conditions related thereto, which are caused or exacerbated by "self" gene products.

As would be understood by one of ordinary skill in the art, when compositions of the invention are administered to an animal or a human, they may be in a composition which contains salts, buffers, adjuvants, or other substances which are desirable for improving the efficacy of the composition. Examples of materials suitable for use in preparing pharmaceutical compositions are provided in numerous sources including Remington's Pharmaceutical Sciences (Osol, A, ed., Mack Publishing Co. (1990)).

Compositions of the invention are said to be "pharmacologically acceptable" if their administration can be tolerated by a recipient individual. Further, the compositions of the invention will be administered in a "therapeutically effective amount" (*i*.*e*., an amount that produces a desired physiological effect).

The compositions of the present invention may be administered by various methods known in the art, but will normally be administered by injection, infusion, inhalation, oral administration or other suitable physical methods. The compositions may alternatively be administered intramuscularly, intravenously, or subcutaneously. Components of compositions for administration include sterile aqueous (*e*.*g*., physiological saline) or non-aqueous solutions and suspensions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption.

Other embodiments of the present invention will be apparent to one of ordinary skill in light of what is known in the art, the following description of the invention, and the claims.

### BRIEF DESCRIPTION OF THE DRAWINGS/FIGURES

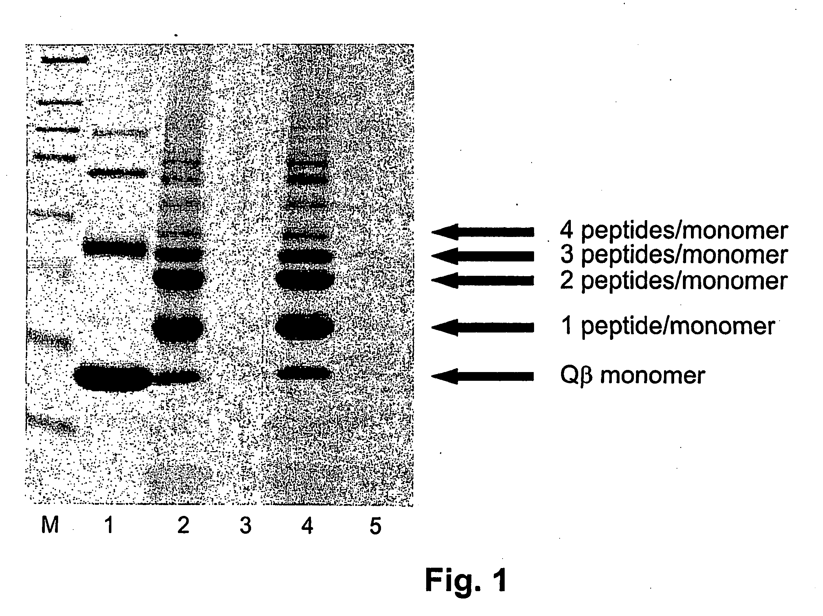

Figure 1 shows the coupling products from the reaction of murine C-Ghrelin (SEQ ID: No. 77) or murine Ghrelin-GC (SEQ ID: No. 105) to Qβ capsid protein. Lane M is the marker, lane 1 shows derivatized Qβ VLP, lane 2 shows Qβ-C-Ghrelin in soluble fraction, lane 3 shows Qβ-C-Ghrelin in insoluble fraction, lane 4 shows Ghrelin-GC-Qβ in soluble fraction, and lane 5 shows Ghrelin-GC-Qβ in insoluble fraction. Very little product is in the insoluble fraction.

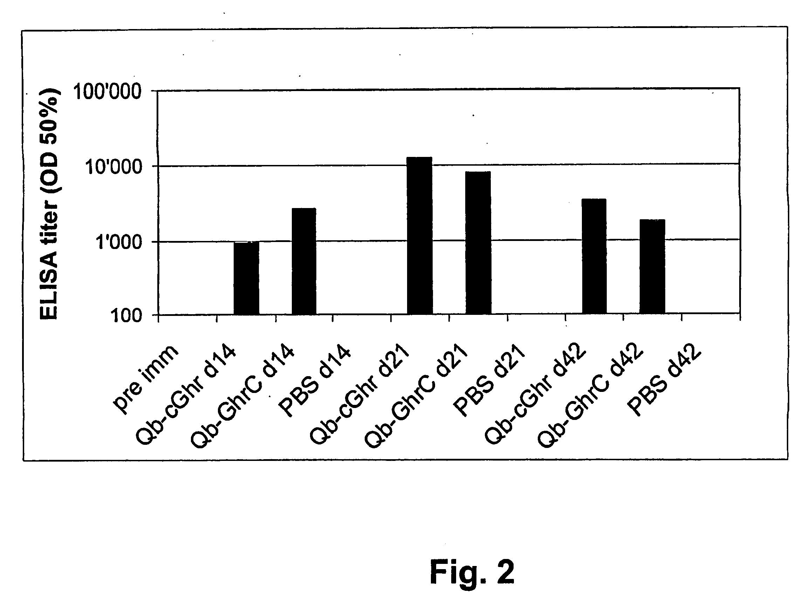

Figure 2 shows the average titers of ghrelin-specific IgG antibodies that were detected in pooled sera from mice, which had been immunized with murine Qβ-C-Ghrelin (Qb-cGhr), murine Ghrelin-GC-Qβ (Qb-GhrC) or PBS on day 0, 14, 21 and 42. ELISA titers are expressed as serum dilutions which lead to half maximal OD in the ELISA assay. ELISA plates were coated with serine-octanylated, murine ghrelin (Bachem, Product No. H-4862) at a concentration of 20µg/ml. The plates were blocked and then incubated with serially diluted mouse sera from day 14, 21 and 42. Bound antibodies were detected with enzymatically labeled anti-mouse IgG antibody. As a control, pre-immune serum of the same mice was also tested.

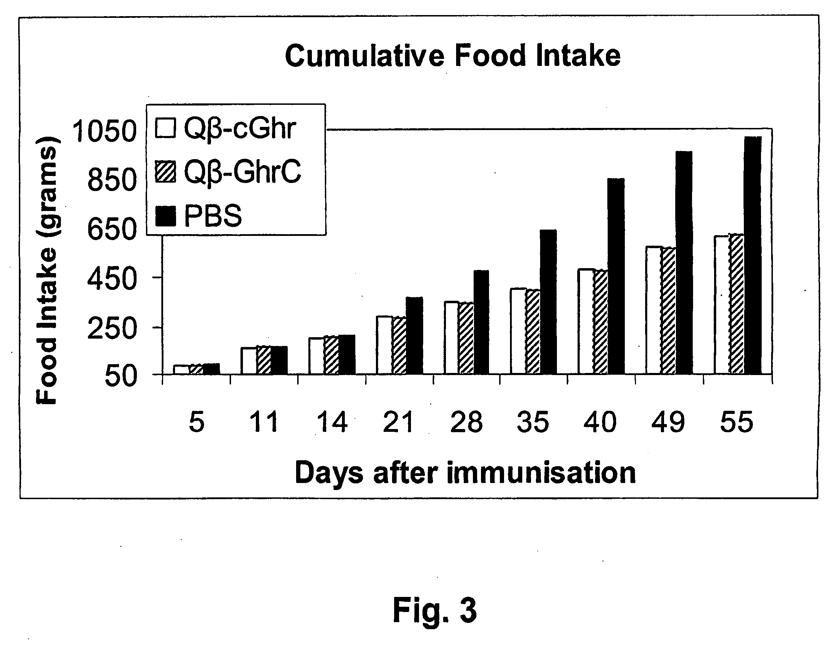

Figure 3 shows the cumulative food consumption of mice, which had been immunized with murine Qβ-C-Ghrelin (Qb-cGhr), murine Ghrelin-GC-Qβ (Qb-GhrC) or PBS. As a control, mice were immunized with PBS. All mice were placed on a normal diet between days 0 and 14. Subsequently, all mice were given a high fat (45%) diet to facilitate the development of diet-induced obesity. Food and water was administered *ad libitum.* Individual mice were monitored for body weight changes and food and water consumption per cage (i.e. group) was also monitored at regular intervals after immunization (at day 5, 11, 14, 21, 28, 35, 40, 49, and 55 after immunization).

### DETAILED DESCRIPTION OF THE INVENTION

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are hereinafter described.

### 1. Definitions:

Adjuvant: The term "adjuvant" as used herein refers to non-specific stimulators of the immune response or substances that allow generation of a depot in the host which when combined with the vaccine and pharmaceutical composition, respectively, of the present invention may provide for an even more enhanced immune response. A variety of adjuvants can be used. Examples include complete and incomplete Freund's adjuvant, aluminum hydroxide and modified muramyldipeptide. Further adjuvants are mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum*. Such adjuvants are also well known in the art. Further adjuvants that can be administered with the compositions of the invention include, but are not limited to, Monophosphoryl lipid immunomodulator, AdjuVax 100a, QS-21, QS-18, CRL1005, Aluminum salts (Alum), MF-59, OM-174, OM-197, OM-294, and Virosomal adjuvant technology. The adjuvants can also comprise a mixture of these substances.

Immunologically active saponin fractions having adjuvant activity derived from the bark of the South American tree Quillaja Saponaria Molina are known in the art. For example QS21, also known as QA21, is an Hplc purified fraction from the Quillaja Saponaria Molina tree and it's method of its production is disclosed (as QA21) in U.S. Pat. No. 5,057,540. Quillaja saponin has also been disclosed as an adjuvant by Scott et al, Int. Archs. Allergy Appl. Immun., 1985, 77, 409. Monosphoryi lipid A and derivatives thereof are known in the art. A preferred derivative is 3 de-o-acylated monophosphoryl lipid A, and is known from British Patent No. 2220211. Further preferred adjuvants are described in WO00/00462.

However, an advantageous feature of the present invention is the high immunogenicty of the inventive compositions. As already outlined herein or will become apparent as this specification proceeds, vaccines and pharmaceutical compositions devoid of adjuvants are provided, in further alternative or preferred embodiments, leading to vaccines and pharmaceutical compositions for treating AD being devoid of adjuvants and, thus, having a superior safety profile since adjuvants may cause side-effects. The term "devoid" as used herein in the context of vaccines and pharmaceutical compositions for treating AD refers to vaccines and pharmaceutical compositions that are used without adjuvants.

Amino acid linker: An "amino acid linker", or also just termed "linker" within this specification, as used herein, either associates the antigen or antigenic determinant with the second attachment site, or more preferably, already comprises or contains the second attachment site, typically - but not necessarily - as one amino acid residue, preferably as a cysteine residue. The term "amino acid linker" as used herein, however, does not intend to imply that such an amino acid linker consists exclusively of amino acid residues, even if an amino acid linker consisting of amino acid residues is a preferred embodiment of the present invention. The amino acid residues of the amino acid linker are, preferably, composed of naturally occuring amino acids or unnatural amino acids known in the art, all-L or all-D or mixtures thereof. However, an amino acid linker comprising a molecule with a sulfhydryl group or cysteine residue is also encompassed within the invention. Such a molecule comprise preferably a C1-C6 alkyl-, cycloalkyl (C5, C6), aryl or heteroaryl moiety. However, in addition to an amino acid linker, a linker comprising preferably a C1-C6 alkyl-, cycloalkyl- (C5, C6), aryl- or heteroaryl- moiety and devoid of any amino acid(s) shall also be encompassed within the scope of the invention. Association between the antigen or antigenic determinant or optionally the second attachment site and the amino acid linker is preferably by way of at least one covalent bond, more preferably by way of at least one peptide bond.

Animal: As used herein, the term "animal" is meant to include, for example, humans, sheep, elks, deer, mule deer, minks, mammals, monkeys, horses, cattle, pigs, goats, dogs, cats, rats, mice, birds, chicken, reptiles, fish, insects and arachnids.

Antibody: As used herein, the term "antibody" refers to molecules which are capable of binding an epitope or antigenic determinant. The term is meant to include whole antibodies and antigen-binding fragments thereof, including single-chain antibodies. Most preferably the antibodies are human antigen binding antibody fragments and include, but are not limited to, Fab, Fab' and F(ab')2, Fd, single-chain Fvs (scFv), single-chain antibodies, disulfide-linked Fvs (sdFv) and fragments comprising either a V_{L} or V_{H} domain. The antibodies can be from any animal origin including birds and mammals. Preferably, the antibodies are human, murine, rabbit, goat, guinea pig, camel, horse or chicken. As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described, for example, in U.S. Patent No. 5,939,598 by Kucherlapati et al.

Antigen: As used herein, the term "antigen" refers to a molecule capable of being bound by an antibody or a T cell receptor (TCR) if presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a Th cell epitope and is given in adjuvant. An antigen can have one or more epitopes (B- and T- epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

Antigenic determinant: As used herein, the term "antigenic determinant" is meant to refer to that portion of an antigen that is specifically recognized by either B- or T-lymphocytes. B-lymphocytes responding to antigenic determinants produce antibodies, whereas T-lymphocytes respond to antigenic determinants by proliferation and establishment of effector functions critical for the mediation of cellular and/or humoral immunity.

Association: As used herein, the term "association" as it applies to the first and second attachment sites, refers to the binding of the first and second attachment sites that is preferably by way of at least one non-peptide bond. The nature of the association may be covalent, ionic, hydrophobic, polar or any combination thereof, preferably the nature of the association is covalent.

Attachment Site, First: As used herein, the phrase "first attachment site" refers to an element of non-natural or natural origin, to which the second attachment site located on the antigen or antigenic determinant may associate. The first attachment site may be a protein, a polypeptide, an amino acid, a peptide, a sugar, a polynucleotide, a natural or synthetic polymer, a secondary metabolite or compound (biotin, fluorescein, retinol, digoxigenin, metal ions, phenylmethylsulfonylfluoride), or a combination thereof, or a chemically reactive group thereof. The first attachment site is located, typically and preferably on the surface, of the core particle such as, preferably the virus-like particle. Multiple first attachment sites are present on the surface of the core and virus-like particle, respectively, typically in a repetitive configuration.

Attachment Site, Second: As used herein, the phrase "second attachment site" refers to an element associated with the antigen or antigenic determinant to which the first attachment site located on the surface of the core particle and virus-like particle, respectively, may associate. The second attachment site of the antigen or antigenic determinant may be a protein, a polypeptide, a peptide, a sugar, a polynucleotide, a natural or synthetic polymer, a secondary metabolite or compound (biotin, fluorescein, retinol, digoxigenin, metal ions, phenylmethylsulfonylfluoride), or a combination thereof, or a chemically reactive group thereof. At least one second attachment site is present on the antigen or antigenic determinant. The term "antigen or antigenic determinant with at least one second attachment site" refers, therefore, to an antigen or antigenic construct comprising at least the antigen or antigenic determinant and the second attachment site. However, in particular for a second attachment site, which is of non-natural origin, i.e. not naturally occurring within the antigen or antigenic determinant, these antigen or antigenic constructs comprise an "amino acid linker".

Bound: As used herein, the term "bound" refers to binding or attachment that may be covalent, *e*.*g*., by chemically coupling, or non-covalent, *e*.*g*., ionic interactions, hydrophobic interactions, hydrogen bonds, etc. Covalent bonds can be, for example, ester, ether, phosphoester, amide, peptide, imide, carbon-sulfur bonds, carbon-phosphorus bonds, and the like. The term "bound" is broader than and includes terms such as "coupled," "fused" and "attached".

Coat protein(s): As used herein, the term "coat protein(s)" refers to the protein(s) of a bacteriophage or a RNA-phage capable of being incorporated within the capsid assembly of the bacteriophage or the RNA-phage. However, when referring to the specific gene product of the coat protein gene of RNA-phages the term "CP" is used. For example, the specific gene product of the coat protein gene of RNA-phage Qβ is referred to as "Qβ CP", whereas the "coat proteins" of bacteriophage Qβ comprise the "Qβ CP" as well as the A1 protein. The capsid of Bacteriophage Qβ is composed mainly of the Qβ CP, with a minor content of the A1 protein. Likewise, the VLP Qβ coat protein contains mainly Qβ CP, with a minor content of A 1 protein.

Core particle: As used herein, the term "core particle" refers to a rigid structure with an inherent repetitive organization. A core particle as used herein may be the product of a synthetic process or the product of a biological process.

Coupled: The term "coupled", as used herein, refers to attachment by covalent bonds or by strong non-covalent interactions, typically and preferably to attachment by covalent bonds. Any method normally used by those skilled in the art for the coupling of biologically active materials can be used in the present invention.

Effective Amount: As used herein, the term "effective amount" refers to an amount necessary or sufficient to realize a desired biologic effect. An effective amount of the composition would be the amount that achieves this selected result, and such an amount could be determined as a matter of routine by a person skilled in the art. For example, an effective amount for treating an immune system deficiency could be that amount necessary to cause activation of the immune system, resulting in the development of an antigen specific immune response upon exposure to antigen. The term is also synonymous with "sufficient amount."

The effective amount for any particular application can vary depending on such factors as the disease or condition being treated, the particular composition being administered, the size of the subject, and/or the severity of the disease or condition. One of ordinary skill in the art can empirically determine the effective amount of a particular composition of the present invention without necessitating undue experimentation.

Epitope: As used herein, the term "epitope" refers to continuous or discontinuous portions of a polypeptide having antigenic or immunogenic activity in an animal, preferably a mammal, and most preferably in a human. An epitope is recognized by an antibody or a T cell through its T cell receptor in the context of an MHC molecule. An "immunogenic epitope," as used herein, is defined as a portion of a polypeptide that elicits an antibody response or induces a T-cell response in an animal, as determined by any method known in the art. (*See*, for example, Geysen et al., Proc. Natl. Acad. Sci. USA 81:3998-4002 (1983)). The term "antigenic epitope," as used herein, is defined as a portion of a protein to which an antibody can immunospecifically bind its antigen as determined by any method well known in the art. Immunospecific binding excludes non-specific binding but does not necessarily exclude cross-reactivity with other antigens. Antigenic epitopes need not necessarily be immunogenic. Antigenic epitopes can also be T-cell epitopes, in which case they can be bound immunospecifically by a T-cell receptor within the context of an MHC molecule.

An epitope can comprise 3 amino acids in a spatial conformation which is unique to the epitope. Generally, an epitope consists of at least about 5 such amino acids, and more usually, consists of at least about 8-10 such amino acids. If the epitope is an organic molecule, it may be as small as Nitrophenyl.

Fusion: As used herein, the term "fusion" refers to the combination of amino acid sequences of different origin in one polypeptide chain by in-frame combination of their coding nucleotide sequences. The term "fusion" explicitly encompasses internal fusions, *i*.*e*., insertion of sequences of different origin within a polypeptide chain, in addition to fusion to one of its termini.

Ghrelin: The term "ghrelin" as used herein refers to a peptide derived from a protein encoded by a ghrelin gene. As used herein ghrelin includes all forms of ghrelins known in humans,, cats, dogs and all domesticated animals as well as of other animals. Ghrelin, as used herein, includes ghrelin with or without a n-octanoyl-modification. Moreover, ghrelin also includes all splice variants that exist of ghrelin. In addition, due to high sequence homology between ghrelins of different species (only 2 aa exchanged between rat and human ghrelin (Kojima et al. Nature 402: 656-660 (1999)), all natural variants of ghrelin with more than 80% identity, preferably more than 90%, more preferably more than 95%, and even more preferably more than 99% with human ghrelin are referred to as "ghrelin" herein.

As used herein, the term "ghrelin-peptides" are broadly defined as any peptide which represents a fraction of ghrelin and containing at least two, preferably at least three, more preferably at least four, more preferably at least five, or at least six, and even more preferably at least eight or nine or even more preferably at least ten consecutive amino acids of the original ghrelin peptide. Typically, "ghrelin peptide" and "ghrelin-fragment" are used interchangeably. Moreover, the terms "ghrelin peptide and fragments thereof" as used herein, shall encompass beside the ghrelin peptide, any fraction of said ghrelin peptide, wherein said fraction may be, preferably, derived by deletion of one or more amino acids at the N and/or C terminus. The ghrelin peptide can be obtained by recombinant expression in eukaryotic or prokaryotic expression systems as ghrelin peptide alone or as a fusion with other amino acids or proteins, e.g. to facilitate folding, expression or solubility of the ghrelin peptide or to facilitate purification of the ghrelin peptide. To facilitate or enable correct folding of fusion proteins between ghrelin peptides and subunit proteins of VLPs or capsids, one or more amino acids may be added N- or C-terminally to ghrelin peptides. To enable coupling of ghrelin peptides to subunit proteins of VLPs or capsids or core particles, at least one second attachment site may be added to the ghrelin peptide. Alternatively ghrelin peptides may be synthesized using methods known to the art. Such peptides may even contain amino acids which are not present in the corresponding ghrelin protein. The peptides may be modified by n-octanoylation.

Residue: As used herein, the term "residue" is meant to mean a specific amino acid in a polypeptide backbone or side chain.

Immune response: As used herein, the term "immune response" refers to a humoral immune response and/or cellular immune response leading to the activation or proliferation of B- and/or T-lymphocytes and/or and antigen presenting cells. In some instances, however, the immune responses may be of low intensity and become detectable only when using at least one substance in accordance with the invention. "Immunogenic" refers to an agent used to stimulate the immune system of a living organism, so that one or more functions of the immune system are increased and directed towards the immunogenic agent. An "immunogenic polypeptide" is a polypeptide that elicits a cellular and/or humoral immune response, whether alone or linked to a carrier in the presence or absence of an adjuvant. Preferably, antigen presenting cell may be activated.

A substance which "enhances" an immune response refers to a substance in which an immune response is observed that is greater or intensified or deviated in any way with the addition of the substance when compared to the same immune response measured without the addition of the substance. For example, the lytic activity of cytotoxic T cells can be measured, *e*.*g*. using a ⁵¹Cr release assay, in samples obtained with and without the use of the substance during immunization. The amount of the substance at which the CTL lytic activity is enhanced as compared to the CTL lytic activity without the substance is said to be an amount sufficient to enhance the immune response of the animal to the antigen. In a preferred embodiment, the immune response in enhanced by a factor of at least about 2, more preferably by a factor of about 3 or more. The amount or type of cytokines secreted may also be altered. Alternatively, the amount of antibodies induced or their subclasses may be altered.

Immunization: As used herein, the terms "immunize" or "immunization" or related terms refer to conferring the ability to mount a substantial immune response (comprising antibodies and/or cellular immunity such as effector CTL) against a target antigen or epitope. These terms do not require that complete immunity be created, but rather that an immune response be produced which is substantially greater than baseline. For example, a mammal may be considered to be immunized against a target antigen if the cellular and/or humoral immune response to the target antigen occurs following the application of methods of the invention.

Natural origin: As used herein, the term "natural origin" means that the whole or parts thereof are not synthetic and exist or are produced in nature.

Non-natural: As used herein, the term generally means not from nature, more specifically, the term means from the hand of man.

Non-natural origin: As used herein, the term "non-natural origin" generally means synthetic or not from nature; more specifically, the term means from the hand of man.

Ordered and repetitive antigen or antigenic determinant array: As used herein, the term "ordered and repetitive antigen or antigenic determinant array" generally refers to a repeating pattern of antigen or antigenic determinant, characterized by a typically and preferably uniform spacial arrangement of the antigens or antigenic determinants with respect to the core particle and virus-like particle, respectively. In one embodiment of the invention, the repeating pattern may be a geometric pattern. Typical and preferred examples of suitable ordered and repetitive antigen or antigenic determinant arrays are those which possess strictly repetitive paracrystalline orders of antigens or antigenic determinants, preferably with spacings of 1 to 30 nanometers, preferably 5 to 15 nanometers.

Pili: As used herein, the term "pili" (singular being "pilus") refers to extracellular structures of bacterial cells composed of protein monomers (*e*.*g*., pilin monomers) which are organized into ordered and repetitive patterns. Further, pili are structures which are involved in processes such as the attachment of bacterial cells to host cell surface receptors, inter-cellular genetic exchanges, and cell-cell recognition. Examples of pili include Type-1 pili, P-pili, F1C pili, S-pili, and 987P-pili. Additional examples of pili are set out below.

Pilus-like structure: As used herein, the phrase "pilus-like structure" refers to structures having characteristics similar to that of pili and composed of protein monomers. One example of a "pilus-like structure" is a structure formed by a bacterial cell which expresses modified pilin proteins that do not form ordered and repetitive arrays that are identical to those of natural pili.

Polypeptide: As used herein, the term "polypeptide" refers to a molecule composed of monomers (amino acids) linearly linked by amide bonds (also known as peptide bonds). It indicates a molecular chain of amino acids and does not refer to a specific length of the product. Thus, peptides, dipeptides, tripeptides, oligopeptides and proteins are included within the definition of polypeptide. This term is also intended to refer to post-expression modifications of the polypeptide, for example, glycosylations, acetylations, phosphorylations, and the like. A recombinant or derived polypeptide is not necessarily translated from a designated nucleic acid sequence. It may also be generated in any manner, including chemical synthesis.

Self antigen: As used herein, the tem "self antigen" refers to proteins encoded by the host's DNA and products generated by proteins or RNA encoded by the host's DNA are defined as self. In addition, proteins that result from a combination of two or several self-molecules or that represent a fraction of a self-molecule and proteins that have a high homology two self-molecules as defined above (>95%, preferably >97%, more preferably >99%) may also be considered self.

Treatment: As used herein, the terms "treatment", "treat", "treated" or "treating" refer to prophylaxis and/or therapy. When used with respect to an infectious disease, for example, the term refers to a prophylactic treatment which increases the resistance of a subject to infection with a pathogen or, in other words, decreases the likelihood that the subject will become infected with the pathogen or will show signs of illness attributable to the infection, as well as a treatment after the subj ect has become infected in order to fight the infection, *e*.*g*., reduce or eliminate the infection or prevent it from becoming worse. When used with respect to obesity or related diseases, the term "treatment" refers to a prophylactic or therapeutic treatment which increases the resistance of a subject against, and/or which reverts obesity.

Vaccine: As used herein, the term "vaccine" refers to a formulation which contains the composition of the present invention and which is in a form that is capable of being administered to an animal. Typically, the vaccine comprises a conventional saline or buffered aqueous solution medium in which the composition of the present invention is suspended or dissolved. In this form, the composition of the present invention can be used conveniently to prevent, ameliorate, or otherwise treat a condition. Upon introduction into a host, the vaccine is able to provoke an immune response including, but not limited to, the production of antibodies and/or cytokines and/or the activation of cytotoxic T cells, antigen presenting cells, helper T cells, dendritic cells and/or other cellular responses.

Optionally, the vaccine of the present invention additionally includes an adjuvant which can be present in either a minor or major proportion relative to the compound of the present invention.

Virus-like particle (VLP): As used herein, the term "virus-like particle" refers to a structure resembling a virus particle. Moreover, a virus-like particle in accordance with the invention is non-replicative and noninfectious since it lacks all or part of the viral genome, in particular the replicative and infectious components of the viral genome. A virus-like particle in accordance with the invention may contain nucleic acid distinct from their genome. A typical and preferred embodiment of a virus-like particle in accordance with the present invention is a viral capsid such as the viral capsid of the corresponding virus, bacteriophage, or RNA-phage. The terms "viral capsid" or "capsid", as interchangeably used herein, refer to a macromolecular assembly composed of viral protein subunits. Typically and preferably, the viral protein subunits assemble into a viral capsid and capsid, respectively, having a structure with an inherent repetitive organization, wherein said structure is, typically, spherical or tubular. For example, the capsids of RNA-phages or HBcAgs have a spherical form of icosahedral symmetry. The term "capsid-like structure" as used herein, refers to a macromolecular assembly composed of viral protein subunits resembling the capsid morphology in the above defined sense but deviating from the typical symmetrical assembly while maintaining a sufficient degree of order and repetitiveness.

Virus-like particle of a bacteriophage: As used herein, the term "virus-like particle of a bacteriophage" refers to a virus-like particle resembling the structure of a bacteriophage, being non replicative and noninfectious, and lacking at least the gene or genes encoding for the replication machinery of the bacteriophage, and typically also lacking the gene or genes encoding the protein or proteins responsible for viral attachment to or entry into the host. This definition should, however, also encompass virus-like particles of bacteriophages, in which the aforementioned gene or genes are still present but inactive, and, therefore, also leading to non-replicative and noninfectious virus-like particles of a bacteriophage.

VLP of RNA phage coat protein: The capsid structure formed from the self-assembly of 180 subunits of RNA phage coat protein and optionally containing host RNA is referred to as a "VLP of RNA phage coat protein." A specific example is the VLP of Qβ coat protein. In this particular case, the VLP of Qβ coat protein may either be assembled exclusively from Qβ CP subunits (generated by expression of a Qβ CP gene containing, for example, a TAA stop codon precluding any expression of the longer A1 protein through suppression, see Kozlovska, T.M., et al., Intervirology 39: 9-15 (1996)), or additionally contain A1 protein subunits in the capsid assembly.

Virus particle: The term "virus particle" as used herein refers to the morphological form of a virus. In some virus types it comprises a genome surrounded by a protein capsid; others have additional structures (*e*.*g*., envelopes, tails, etc.).

One, a, or an: When the terms "one," "a," or "an" are used in this disclosure, they mean "at least one" or "one or more," unless otherwise indicated.

As will be clear to those skilled in the art, certain embodiments of the invention involve the use of recombinant nucleic acid technologies such as cloning, polymerase chain reaction, the purification of DNA and RNA, the expression of recombinant proteins in prokaryotic and eukaryotic cells, etc. Such methodologies are well known to those skilled in the art and can be conveniently found in published laboratory methods manuals (*e*.*g*., Sambrook, J. et al., eds., Molecular Cloning, A Laboratory Manual, 2nd. edition, Cold Spring Harbor Laboratory Press, Cold Spring Harbor, N.Y. (1989); Ausubel, F. et al., eds., Current Protocols in Molecular Biology, John H. Wiley & Sons, Inc. (1997)). Fundamental laboratory techniques for working with tissue culture cell lines (Celis, J., ed., Cell Biology, Academic Press, 2nd edition, (1998)) and antibody-based technologies (Harlow, E. and Lane, D., Antibodies: A Laboratory Manual, Cold Spring Harbor Laboratory, Cold Spring Harbor, N.Y. (1988); Deutscher, M.P., "Guide to Protein Purification," Meth. Enzymol. 128, Academic Press San Diego (1990); Scopes, R.K., Protein Purification Principles and Practice, 3rd ed., Springer-Verlag, New York (1994)) are also adequately described in the literature.

### 2. Compositions and Methods for Enhancing an Immune Response

The disclosed invention provides compositions and methods for enhancing an immune response against ghrelin or a ghrelin peptide in an animal. Compositions of the invention comprise, or alternatively consist of (a) a core particle with at least one first attachment site; and (b) at least one antigen or antigenic determinant with at least one second attachment site, wherein said antigen or antigenic determinant is ghrelin or a ghrelin peptide, and wherein said second attachment site being selected from the group consisting of (i) an attachment site not naturally occurring with said antigen or antigenic determinant; and (ii) an attachment site naturally occurring with said antigen or antigenic determinant, wherein said second attachment site is capable of association to said first attachment site; and wherein said antigen or antigenic determinant and said core particle interact through said association to form an ordered and repetitive antigen array. More specifically, compositions of the invention comprise, or alternatively consist of, a virus-like particle and at least one antigen or antigenic determinant, wherein the antigen or antigenic determinant is ghrelin or a ghrelin peptide and wherein the at least one antigen or antigenic determinant is bound to the virus-like particle so as to form an ordered and repetitive antigen-VLP-array. Furthermore, the invention conveniently enables the practitioner to construct such a composition, inter alia, for treatment and/or prophylactic prevention of obesity.

In one embodiment, the core particle comprises, or is selected from a group consisting of, a virus, a bacterial pilus, a structure formed from bacterial pilin, a bacteriophage, a virus-like particle, a virus-like particle of a RNA phage, a viral capsid particle or a recombinant form thereof. Any virus known in the art having an ordered and repetitive coat and/or core protein structure may be selected as a core particle of the invention; examples of suitable viruses include sindbis and other alphaviruses, rhabdoviruses (*e*.*g*. vesicular stomatitis virus), picornaviruses (*e*.*g*., human rhino virus, Aichi virus), togaviruses (*e*.*g*., rubella virus), orthomyxoviruses (*e*.*g*., Thogoto virus, Batken virus, fowl plague virus), polyomaviruses (*e*.*g*., polyomavirus BK, polyomavirus JC, avian polyomavirus BFDV), parvoviruses, rotaviruses, Norwalk virus, foot and mouth disease virus, a retrovirus, Hepatitis B virus, Tobacco mosaic virus, Flock House Virus, and human Papilomavirus, and preferably a RNA phage, bacteriophage Qβ, bacteriophage R17, bacteriophage M11, bacteriophage MX1, bacteriophage NL95, bacteriophage fr, bacteriophage GA, bacteriophage SP, bacteriophage MS2, bacteriophage f2, bacteriophage PP7 (for example, *see* Table 1 in Bachmann, M.F. and Zinkernagel, R.M., Immunol. Today 17:553-558 (1996)).

In a further embodiment, the invention utilizes genetic engineering of a virus to create a fusion between an ordered and repetitive viral envelope protein and a first attachment site being comprised by, or alternatively or preferably being a heterologous protein, peptide, antigenic determinant or a reactive amino acid residue of choice. Other genetic manipulations known to those in the art may be included in the construction of the inventive compositions; for example, it may be desirable to restrict the replication ability of the recombinant virus through genetic mutation. Furthermore, the virus used for the present invention is replication incompetent due to chemical or physical inactivation or, as indicated, due to lack of a replication competent genome. The viral protein selected for fusion to the first attachment site should have an organized and repetitive structure. Such an organized and repetitive structure includes paracrystalline organizations with a spacing of 5-30 nm, preferably 5-15 nm, on the surface of the virus. The creation of this type of fusion protein will result in multiple, ordered and repetitive first attachment sites on the surface of the virus and reflect the normal organization of the native viral protein. As will be understood by those in the art, the first attachment site may be or be a part of any suitable protein, polypeptide, sugar, polynucleotide, peptide (amino acid), natural or synthetic polymer, a secondary metabolite or combination thereof that may serve to specifically attach the antigen or antigenic determinant leading an ordered and repetitive antigen array.

In another embodiment of the invention, the core particle is a recombinant alphavirus, and more specifically, a recombinant Sinbis virus. Alphaviruses are positive stranded RNA viruses that replicate their genomic RNA entirely in the cytoplasm of the infected cell and without a DNA intermediate (Strauss, J. and Strauss, E., Microbiol. Rev. 58:491-562 (1994)). Several members of the alphavirus family, Sindbis (Xiong, C. et al., Science 243:1188-1191 (1989); Schlesinger, S., Trends Biotechnol. 11:18-22 (1993)), Semliki Forest Virus (SFV) (Liljeström, P. & Garoff, H., Bio/Technology 9:1356-1361 (1991)) and others (Davis, N.L. et al., Virology 171:189-204 (1989)), have received considerable attention for use as virus-based expression vectors for a variety of different proteins (Lundstrom, K., Curr. Opin. Biotechnol. 8:578-582 (1997); Liljeström, P., Curr. Opin. Biotechnol. 5:495-500 (1994)) and as candidates for vaccine development. Recently, a number of patents have issued directed to the use of alphaviruses for the expression of heterologous proteins and the development of vaccines (*see* U.S. Patent Nos. 5,766,602; 5,792,462; 5,739,026; 5,789,245 and 5,814,482). The construction of the alphaviral core particles of the invention may be done by means generally known in the art of recombinant DNA technology, as described by the aforementioned articles.

A variety of different recombinant host cells can be utilized to produce a viral-based core particle for antigen or antigenic determinant attachment. For example, alphaviruses are known to have a wide host range; Sindbis virus infects cultured mammalian, reptilian, and amphibian cells, as well as some insect cells (Clark, H., J. Natl. Cancer Inst. 51:645 (1973); Leake, C., J. Gen. Virol. 35:335 (1977); Stollar, V. in THE TOGA VIRUSES, R.W. Schlesinger, Ed., Academic Press, (1980), pp.583-621). Thus, numerous recombinant host cells can be used in the practice of the invention. BHK, COS, Vero, HeLa and CHO cells are particularly suitable for the production of heterologous proteins because they have the potential to glycosylate heterologous proteins in a manner similar to human cells (Watson, E. et al., Glycobiology 4:227, (1994)) and can be selected (Zang, M. et al., Bio/Technology 13:389 (1995)) or genetically engineered (Renner W. et al., Biotech. Bioeng. 4:476 (1995); Lee K. et al. Biotech. Bioeng. 50:336 (1996)) to grow in serum-free medium, as well as in suspension.

Introduction of the polynucleotide vectors into host cells can be effected by methods described in standard laboratory manuals (*see*, *e*.*g*., Sambrook, J. et al., eds., MOLECULAR CLONING, A LABORATORY MANUAL, 2nd. edition, Cold Spring Harbor Laboratory Press, Cold Spring Harbor, N.Y. (1989), Chapter 9; Ausubel, F. et al., eds., CURRENT PROTOCOLS IN MOLECULAR BIOLOGY, John H. Wiley & Sons, Inc. (1997), Chapter 16), including methods such as electroporation, DEAE-dextran mediated transfection, transfection, microinjection, cationic lipid-mediated transfection, transduction, scrape loading, ballistic introduction, and infection. Methods for the introduction of exogenous DNA sequences into host cells are discussed in Felgner, P. et al., U.S. Patent No. 5,580,859.

Packaged RNA sequences can also be used to infect host cells. These packaged RNA sequences can be introduced to host cells by adding them to the culture medium. For example, the preparation of non-infective alphaviral particles is described in a number of sources, including "Sindbis Expression System", Version C (*Invitrogen* Catalog No. K750-1).

When mammalian cells are used as recombinant host cells for the production of viral-based core particles, these cells will generally be grown in tissue culture. Methods for growing cells in culture are well known in the art (*see*, *e.g.,* Celis, J., ed., CELL BIOLOGY, Academic Press, 2nd edition, (1998); Sambrook, J. et al., eds., MOLECULAR CLONING, A LABORATORY MANUAL, 2nd. edition, Cold Spring Harbor Laboratory Press, Cold Spring Harbor, N.Y. (1989); Ausubel, F. et al., eds., CURRENT PROTOCOLS IN MOLECULAR BIOLOGY, John H. Wiley & Sons, Inc. (1997); Freshney, R., CULTURE OF ANIMAL CELLS, Alan R. Liss, Inc. (1983)).

Further examples of RNA viruses suitable for use as core particle in the present invention include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picomaviridae, including the genus Enterovirus (poliovirus, Coxsackie virus A and B, enteric cytopathic human orphan (ECHO) viruses, hepatitis A, C, D, E and G viruses, Simian enteroviruses, Murine encephalomyelitis (ME) viruses, Poliovirus muris, Bovine enteroviruses, Porcine enteroviruses, the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest *virus),* the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial *virus* and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses and, filoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that may be used as core particles include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A, B, C, D and E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc.). Finally, DNA viruses may include viruses such as chronic infectious neuropathic agents (CHINA virus).

In other embodiments, a bacterial pilin, a subportion of a bacterial pilin, or a fusion protein which contains either a bacterial pilin or subportion thereof is used to prepare compositions and vaccine compositions, respectively, of the invention. Examples of pilin proteins include pilins produced by *Escherichia coli, Haemophilus influenzae, Neisseria meningitidis, Neisseria gonorrhoeae, Caulobacter crescentus, Pseudomonas stutzeri*, and *Pseudomonas aeruginosa*. The amino acid sequences of pilin proteins suitable for use with the present invention include those set out in GenBank reports AJ000636, AJ132364, AF229646, AF051814, AF051815), and X00981.

Bacterial pilin proteins are generally processed to remove N-terminal leader sequences prior to export of the proteins into the bacterial periplasm. Further, as one skilled in the art would recognize, bacterial pilin proteins used to prepare compositions and vaccine compositions, respectively, of the invention will generally not have the naturally present leader sequence.

One specific example of a pilin protein suitable for use in the present invention is the P-pilin of *E. coli* (GenBank report AF237482 (SEQ ID NO:1)). An example of a Type-1 *E. coli* pilin suitable for use with the invention is a pilin having the amino acid sequence set out in GenBank report P04128 (SEQ ID NO:2), which is encoded by nucleic acid having the nucleotide sequence set out in GenBank report M27603 (SEQ ID NO:3). The entire disclosures of these GenBank reports are incorporated herein by reference. Again, the mature form of the above referenced protein would generally be used to prepare compositions and vaccine compositions, respectively, of the invention.

Bacterial pilins or pilin subportions suitable for use in the practice of the present invention will generally be able to associate to form ordered and repetitive antigen arrays.

Methods for preparing pili and pilus-like structures *in vitro* are known in the art. Bullitt et al., Proc. Natl. Acad. Sci. USA 93:12890-12895 (1996), for example, describe the *in vitro* reconstitution of *E. coli* P-pili subunits. Furthermore, Eshdat et al., J. Bacteriol. 148:308-314 (1981) describe methods suitable for dissociating Type-1 pili of *E. coli* and the reconstitution of pili. In brief, these methods are as follows: pili are dissociated by incubation at 37°C in saturated guanidine hydrochloride. Pilin proteins are then purified by chromatography, after which pilin dimers are formed by dialysis against 5 mM tris(hydroxymethyl) aminomethane hydrochloride (pH 8.0). Eshdat *et al.* also found that pilin dimers reassemble to form pili upon dialysis against the 5 mM tris(hydroxymethyl) aminomethane (pH 8.0) containing 5 mM MgCl₂.

Further, using, for example, conventional genetic engineering and protein modification methods, pilin proteins may be modified to contain a first attachment site to which an antigen or antigenic determinant is linked through a second attachment site. Alternatively, antigens or antigenic determinants can be directly linked through a second attachment site to amino acid residues which are naturally resident in these proteins. These modified pilin proteins may then be used in vaccine compositions of the invention.

Bacterial pilin proteins used to prepare compositions and vaccine compositions, respectively, of the invention may be modified in a manner similar to that described herein for HBcAg. For example, cysteine and lysine residues may be either deleted or substituted with other amino acid residues and first attachment sites may be added to these proteins. Further, pilin proteins may either be expressed in modified form or may be chemically modified after expression. Similarly, intact pili may be harvested from bacteria and then modified chemically.

In another embodiment, pili or pilus-like structures are harvested from bacteria (*e*.*g*., *E. coli*) and used to form compositions and vaccine compositions of the invention. One example of pili suitable for preparing compositions and vaccine compositions is the Type-1 pilus of *E. coli,* which is formed from pilin monomers having the amino acid sequence set out in SEQ ID NO:2.

A number of methods for harvesting bacterial pili are known in the art. Bullitt and Makowski (Biophys. J. 74:623-632 (1998)), for example, describe a pilus purification method for harvesting P-pili from *E. coli.* According to this method, pili are sheared from hyperpiliated *E. coli* containing a P-pilus plasmid and purified by cycles of solubilization and MgCl₂ (1.0 M) precipitation.

Once harvested, pili or pilus-like structures may be modified in a variety of ways. For example, a first attachment site can be added to the pili to which antigens or antigen determinants may be attached through a second attachment site. In other words, bacterial pili or pilus-like structures can be harvested and modified to lead to ordered and repetitive antigen arrays.

Antigens or antigenic determinants could be linked to naturally occurring cysteine resides or lysine residues present in Pili or pilus-like structures. In such instances, the high order and repetitiveness of a naturally occurring amino acid residue would guide the coupling of the antigens or antigenic determinants to the pili or pilus-like structures. For example, the pili or pilus-like structures could be linked to the second attachment sites of the antigens or antigenic determinants using a heterobifunctional cross-linking agent

When structures which are naturally synthesized by organisms (*e*.*g*., pili) are used to prepare compositions and vaccine compositions of the invention, it will often be advantageous to genetically engineer these organisms so that they produce structures having desirable characteristics. For example, when Type-1 pili of *E. coli* are used, the *E. coli* from which these pili are harvested may be modified so as to produce structures with specific characteristics. Examples of possible modifications of pilin proteins include the insertion of one or more lysine residues, the deletion or substitution of one or more of the naturally resident lysine residues, and the deletion or substitution of one or more naturally resident cysteine residues (*e*.*g*., the cysteine residues at positions 44 and 84 in SEQ ID NO:2).

Further, additional modifications can be made to pilin genes which result in the expression products containing a first attachment site other than a lysine residue (*e*.*g*., a *FOS* or *JUN* domain). Of course, suitable first attachment sites will generally be limited to those which do not prevent pilin proteins from forming pili or pilus-like structures suitable for use in vaccine compositions of the invention.

Pilin genes which naturally reside in bacterial cells can be modified *in vivo* (*e*.*g*., by homologous recombination) or pilin genes with particular characteristics can be inserted into these cells. For examples, pilin genes could be introduced into bacterial cells as a component of either a replicable cloning vector or a vector which inserts into the bacterial chromosome. The inserted pilin genes may also be linked to expression regulatory control sequences (*e*.*g*., a *lac* operator).

In most instances, the pili or pilus-like structures used in compositions and vaccine compositions, respectively, of the invention will be composed of single type of a pilin subunit. Pili or pilus-like structures composed of identical subunits will generally be used because they are expected to form structures which present highly ordered and repetitive antigen arrays.

However, the compositions of the invention also include compositions and vaccines comprising pili or pilus-like structures formed from heterogenous pilin subunits. The pilin subunits which form these pili or pilus-like structures can be expressed from genes naturally resident in the bacterial cell or may be introduced into the cells. When a naturally resident pilin gene and an introduced gene are both expressed in a cell which forms pili or pilus-like structures, the result will generally be structures formed from a mixture of these pilin proteins. Further, when two or more pilin genes are expressed in a bacterial cell, the relative expression of each pilin gene will typically be the factor which determines the ratio of the different pilin subunits in the pili or pilus-like structures.

When pili or pilus-like structures having a particular composition of mixed pilin subunits is desired, the expression of at least one of the pilin genes can be regulated by a heterologous, inducible promoter. Such promoters, as well as other genetic elements, can be used to regulate the relative amounts of different pilin subunits produced in the bacterial cell and, hence, the composition of the pili or pilus-like structures.

In additional, the antigen or antigenic determinant can be linked to bacterial pili or pilus-like structures by a bond which is not a peptide bond, bacterial cells which produce pili or pilus-like structures used in the compositions of the invention can be genetically engineered to generate pilin proteins which are fused to an antigen or antigenic determinant. Such fusion proteins which form pili or pilus-like structures are suitable for use in vaccine compositions of the invention.

Virus-like particles in the context of the present application refer to structures resembling a virus particle but which are not pathogenic. In general, virus-like particles lack the viral genome and, therefore, are noninfectious. Also, virus-like particles can be produced in large quantities by heterologous expression and can be easily purified.

In a preferred embodiment, the core particle is a virus-like particle, wherein the virus-like particle is a recombinant virus-like particle. The skilled artisan can produce VLPs using recombinant DNA technology and virus coding sequences which are readily available to the public. For example, the coding sequence of a virus envelope or core protein can be engineered for expression in a baculovirus expression vector using a commercially available baculovirus vector, under the regulatory control of a virus promoter, with appropriate modifications of the sequence to allow functional linkage of the coding sequence to the regulatory sequence. The coding sequence of a virus envelope or core protein can also be engineered for expression in a bacterial expression vector, for example.

Examples of VLPs include, but are not limited to, the capsid proteins of Hepatitis B virus (Ulrich, et al., Virus Res. 50:141-182 (1998)), measles virus (Warnes, et al., Gene 160:173-178 (1995)), Sindbis virus, rotavirus (US 5,071,651 and US 5,374,426), foot-and-mouth-disease virus (Twomey, et al., Vaccine 13:1603-1610, (1995)), Norwalk virus (Jiang, X., et al., Science (1990); Matsui, S.M., et al., J. Clin. Invest. 87:1456-1461 (1991)), the retroviral GAG protein (WO 96/30523), the retrotransposon Ty protein pl, the surface protein of Hepatitis B virus (WO 92/11291), human papilloma virus (WO 98/15631), RNA phages, Ty, fr-phage, GA-phage, AP205-phage and Qβ-phage.

As will be readily apparent to those skilled in the art, the VLP of the invention is not limited to any specific form. The particle can be synthesized chemically or through a biological process, which can be natural or non-natural. By way of example, this type of embodiment includes a virus-like particle or a recombinant form thereof.

In a more specific embodiment, the VLP can comprise, or alternatively essentially consist of, or alternatively consist of recombinant polypeptides, or fragments thereof, being selected from recombinant polypeptides of Rotavirus, recombinant polypeptides of Norwalk virus, recombinant polypeptides of Alphavirus, recombinant polypeptides of Foot and Mouth Disease virus, recombinant polypeptides of measles virus, recombinant polypeptides of Sindbis virus, recombinant polypeptides of Polyoma virus, recombinant polypeptides of Retrovirus, recombinant polypeptides of Hepatitis B virus (*e*.*g*., a HBcAg), recombinant polypeptides of Tobacco mosaic virus, recombinant polypeptides of Flock House Virus, recombinant polypeptides of human Papillomavirus, recombinant polypeptides of bacteriophages, recombinant polypeptides of RNA phages, recombinant polypeptides of Ty, recombinant polypeptides of fr-phage, recombinant polypeptides of GA-phage and recombinant polypeptides of Qβ-phage. The virus-like particle can further comprise, or alternatively essentially consist of, or alternatively consist of, one or more fragments of such polypeptides, as well as variants of such polypeptides. Variants of polypeptides can share, for example, at least 80%, 85%, 90%, 95%, 97%, or 99% identity at the amino acid level with their wild-type counterparts.

In a preferred embodiment, the virus-like particle comprises, consists essentially of, or alternatively consists of recombinant proteins, or fragments thereof, of a RNA-phage. Preferably, the RNA-phage is selected from the group consisting of a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7, and m) bacteriophage AP205.

In another preferred embodiment of the present invention, the virus-like particle comprises, or alternatively consists essentially of, or alternatively consists of recombinant proteins, or fragments thereof, of the RNA-bacteriophage Qβ or of the RNA-bacteriophage fr, or of the RNA-bacteriophage AP205.

In a further preferred embodiment of the present invention, the recombinant proteins comprise, or alternatively consist essentially of, or alternatively consist of coat proteins of RNA phages.

RNA-phage coat proteins forming capsids or VLPs, or fragments of the bacteriophage coat proteins compatible with self-assembly into a capsid or a VLP, are, therefore, further preferred embodiments of the present invention. Bacteriophage Qβ coat proteins, for example, can be expressed recombinantly in *E*. *coli.* Further, upon such expression these proteins spontaneously form capsids. Additionally, these capsids form a structure with an inherent repetitive organization.

Specific preferred examples of bacteriophage coat proteins which can be used to prepare compositions of the invention include the coat proteins of RNA bacteriophages such as bacteriophage Qβ (SEQ ID NO:4; PIR Database, Accession No. VCBPQβ referring to Qβ CP and SEQ ID NO: 5; Accession No. AAA16663 referring to Qβ A1 protein), bacteriophage R17 (SEQ ID NO:6; PIR Accession No. VCBPR7), bacteriophage fr (SEQ ID NO:7; PIR Accession No. VCBPFR), bacteriophage GA (SEQ II7 NO:8; GenBank Accession No. NP-040754), bacteriophage SP (SEQ II7 NO:9; GenBank Accession No. CAA30374 referring to SP CP and SEQ ID NO: 10; Accession No. NP 695026 referring to SP A1 protein), bacteriophage MS2 (SEQ ID NO: 11; PIR Accession No. VCBPM2), bacteriophage M11 (SEQ ID NO:12; GenBank Accession No. AAC06250), bacteriophage MX1 (SEQ ID NO:13; GenBank Accession No. AAC14699), bacteriophage NL95 (SEQ ID NO:14; GenBank Accession No. AAC14704), bacteriophage f2 (SEQ ID NO: 15; GenBank Accession No. P03611), bacteriophage PP7 (SEQ ID NO: 16), and bacteriophage AP205 (SEQ ID NO: 28). Furthermore, the A1 protein of bacteriophage Qβ (SEQ ID NO: 5) or C-terminal truncated forms missing as much as 100, 150 or 180 amino acids from its C-terminus may be incorporated in a capsid assembly of Qβ coat proteins. Generally, the percentage of Qβ A1 protein relative to Qβ CP in the capsid assembly will be limited, in order to ensure capsid formation.

Qβ coat protein has also been found to self-assemble into capsids when expressed in *E. coli* (Kozlovska TM. et al., GENE 137: 133-137 (1993)). The obtained capsids or virus-like particles showed an icosahedral phage-like capsid structure with a diameter of 25 nm and T=3 quasi symmetry. Further, the crystal structure of phage Qβ has been solved. The capsid contains 180 copies of the coat protein, which are linked in covalent pentamers and hexamers by disulfide bridges (Golmohammadi, R. et al., Structure 4: 543-5554 (1996)) leading to a remarkable stability of the capsid of Qβ coat protein. Capsids or VLPs made from recombinant Qβ coat protein may contain, however, subunits not linked via disulfide links to other subunits within the capsid, or incompletely linked. However, typically more than about 80% of the subunits are linked via disulfide bridges to each other within the VLP. Thus, upon loading recombinant Qβ capsid on non-reducing SDS-PAGE, bands corresponding to monomeric Qβ coat protein as well as bands corresponding to the hexamer or pentamer of Qβ coat protein are visible. Incompletely disulfide-linked subunits could appear as dimer, trimer or even tetramer band in non-reducing SDS-PAGE. Qβ capsid protein also shows unusual resistance to organic solvents and denaturing agents. Surprisingly, we have observed that DMSO and acetonitrile concentrations as high as 30%, and Guanidinium concentrations as high as 1 M do not affect the stability of the capsid. The high stability of the capsid of Qβ coat protein is an advantageous feature, in particular, for its use in immunization and vaccination of mammals and humans in accordance of the present invention.

Upon expression in *E. coli,* the N-terminal methionine of Qβ coat protein is usually removed, as we observed by N-terminal Edman sequencing as described in Stoll, E. et al. J. Biol. Chem. 252:990-993 (1977). VLP composed from Qβ coat proteins where the N-terminal methionine has not been removed, or VLPs comprising a mixture of Qβ coat proteins where the N-terminal methionine is either cleaved or present are also within the scope of the present invention.

Further preferred virus-like particles of RNA-phages, in particular of Qβ, in accordance of this invention are disclosed in WO 02/056905.

Further RNA phage coat proteins have also been shown to self-assemble upon expression in a bacterial host (Kastelein, RA. et al., Gene 23: 245-254 (1983), Kozlovskaya, TM. et al., Dokl. Akad. Nauk SSSR 287: 452-455 (1986), Adhin, MR. et al., Virology 170: 238-242 (1989), Ni, CZ., et al., Protein Sci. 5: 2485-2493 (1996), Priano, C. et al., J. Mol. Biol. 249: 283-297 (1995)). The Qβ phage capsid contains, in addition to the coat protein, the so called read-through protein A1 and the maturation protein A2. A1 is generated by suppression at the UGA stop codon and has a length of 329 aa. The capsid of phage Qβ recombinant coat protein used in the invention is devoid of the A2 lysis protein, and contains RNA from the host. The coat protein of RNA phages is an RNA binding protein, and interacts with the stem loop of the ribosomal binding site of the replicase gene acting as a translational repressor during the life cycle of the virus. The sequence and structural elements of the interaction are known (Witherell, GW. & Uhlenbeck, OC. Biochemistry 28: 71-76 (1989); Lim F. et al., J. Biol. Chem. 271: 31839-31845 (1996)). The stem loop and RNA in general are known to be involved in the virus assembly (Golmohammadi, R. et al., Structure 4: 543-5554 (1996)).

In a further preferred embodiment of the present invention, the virus-like particle comprises, or alternatively consists essentially of, or alternatively consists of recombinant proteins, or fragments thereof, of a RNA-phage, wherein the recombinant proteins comprise, alternatively consist essentially of or alternatively consist of mutant coat proteins of a RNA phage, preferably of mutant coat proteins of the RNA phages mentioned above. In another preferred embodiment, the mutant coat proteins of the RNA phage have been modified by removal of at least one, or alternatively at least two, lysine residue by way of substitution, or by addition of at least one lysine residue by way of substitution; alternatively, the mutant coat proteins of the RNA phage have been modified by deletion of at least one, or alternatively at least two, lysine residue, or by addition of at least one lysine residue by way of insertion. The deletion, substitution or addition of at least one lysine residue allows varying the degree of coupling, i.e. the amount of Aβ1-6 peptides per subunits of theVLP of the RNA-phages, in particular, to match and tailor the requirements of the vaccine.

In another preferred embodiment, the virus-like particle comprises, or alternatively consists essentially of, or alternatively consists of recombinant proteins, or fragments thereof, of the RNA-bacteriophage Qβ, wherein the recombinant proteins comprise, or alternatively consist essentially of, or alternatively consist of coat proteins having an amino acid sequence of SEQ ID NO:4, or a mixture of coat proteins having amino acid sequences of SEQ ID NO:4 and of SEQ ID NO: 5 or mutants of SEQ ID NO: 5 and wherein the N-terminal methionine is preferably cleaved.

In a further preferred embodiment of the present invention, the virus-like particle comprises, consists essentially of or alternatively consists of recombinant proteins of Qβ, or fragments thereof, wherein the recombinant proteins comprise, or alternatively consist essentially of, or alternatively consist of mutant Qβ coat proteins. In another preferred embodiment, these mutant coat proteins have been modified by removal of at least one lysine residue by way of substitution, or by addition of at least one lysine residue by way of substitution. Alternatively, these mutant coat proteins have been modified by deletion of at least one lysine residue, or by addition of at least one lysine residue by way of insertion.

Four lysine residues are exposed on the surface of the capsid of Qβ coat protein. Qβ mutants, for which exposed lysine residues are replaced by arginines can also be used for the present invention. The following Qβ coat protein mutants and mutant Qβ VLPs can, thus, be used in the practice of the invention: "Qβ-240" (Lys13-Arg; SEQ ID NO:17), "Qβ-243" (Asn 10-Lys; SEQ ID NO:18), "Qβ-250" (Lys 2-Arg, Lys13-Arg; SEQ ID NO:19), "Qβ-251" (SEQ ID NO:20) and "Qβ-259" (Lys 2-Arg, Lys16-Arg; SEQ ID NO:21). Thus, in further preferred embodiment of the present invention, the virus-like particle comprises, consists essentially of or alternatively consists of recombinant proteins of mutant Qβ coat proteins, which comprise proteins having an amino acid sequence selected from the group of a) the amino acid sequence of SEQ ID NO:17; b) the amino acid sequence of SEQ ID NO: 18; c) the amino acid sequence of SEQ ID NO:19; d) the amino acid sequence of SEQ ID NO:20; and e) the amino acid sequence of SEQ ID NO:21. The construction, expression and purification of the above indicated Qβ coat proteins, mutant Qβ coat protein VLPs and capsids, respectively, are described in WO 02/056905. In particular is hereby referred to Example 18 of above mentioned application.

In a further preferred embodiment of the present invention, the virus-like particle comprises, or alternatively consists essentially of, or alternatively consists of recombinant proteins of Qβ, or fragments thereof, wherein the recombinant proteins comprise, consist essentially of or alternatively consist of a mixture of either one of the foregoing Qβ mutants and the corresponding A1 protein.

In a further preferred embodiment of the present invention, the virus-like particle comprises, or alternatively essentially consists of, or alternatively consists of recombinant proteins, or fragments thereof, of RNA-phage AP205.

The AP205 genome consists of a maturation protein, a coat protein, a replicase and two open reading frames not present in related phages; a lysis gene and an open reading frame playing a role in the translation of the maturation gene (Klovins,J., et al., J. Gen. Virol. 83: 1523-33 (2002)). AP205 coat protein can be expressed from plasmid pAP283-58 (SEQ ID NO: 27), which is a derivative of pQb10 (Kozlovska, T. M.. et al., Gene 137:133-37 (1993)), and which contains an AP205 ribosomal binding site. Alternatively, AP205 coat protein may be cloned into pQb185, downstream of the ribosomal binding site present in the vector. Both approaches lead to expression of the protein and formation of capsids as described in the co-pending US provisional patent application with the title "Molecular Antigen Arrays" and having filed by the present assignee on July 17, 2002, which is incorporated by reference in its entirety. Vectors pQb10 and pQb185 are vectors derived from pGEM vector, and expression of the cloned genes in these vectors is controlled by the *trp* promoter (Kozlovska, T. M. et al., Gene 137:133-37 (1993)). Plasmid pAP283-58 (SEQ ID NO:27) comprises a putative AP205 ribosomal binding site in the following sequence, which is downstream of the XbaI site, and immediately upstream of the ATG start codon of the AP205 coat protein: *tctaga*ATTTTCTGCGCACCCAT CCCGGGTGGCGCCCAAA-GTGAGGAAAATCAC*atg* (SEQ ID NO:57). The vector pQb185 comprises a Shine-Dalgarno sequence downstream from the Xbal site and upstream of the start codon (*tctaga*TTAACCCAACGCGTAGGAGTCAGGCC*atg* (SEQ ID NO:58), Shine-Dalgarno sequence underlined).

In a further preferred embodiment of the present invention, the virus-like particle comprises, or alternatively essentially consists of, or alternatively consists of recombinant coat proteins, or fragments thereof, of the RNA-phage AP205.

This preferred embodiment of the present invention, thus, comprises AP205 coat proteins that form capsids. Such proteins are recombinantly expressed or prepared from natural sources. AP205 coat proteins produced in bacteria spontaneously form capsids, as evidenced by Electron Microscopy (EM) and immunodiffusion. The structural properties of the capsid formed by the AP205 coat protein (SEQ ID NO: 28) and those formed by the coat protein of the AP205 RNA phage are nearly indistinguishable when seen in EM. AP205 VLPs are highly immunogenic, and can be linked with antigens and/or antigenic determinants to generate vaccine constructs displaying the antigens and/or antigenic determinants oriented in a repetitive manner. High titers are elicited against the so displayed antigens showing that bound antigens and/or antigenic determinants are accessible for interacting with antibody molecules and are immunogenic.

In a further preferred embodiment of the present invention, the virus-like particle comprises, or alternatively essentially consists of, or alternatively consists of recombinant mutant coat proteins, or fragments thereof, of the RNA-phage AP205.

Assembly-competent mutant forms of AP205 VLPs, including AP205 coat protein with the substitution of proline at amino acid 5 to threonine (SEQ ID NO: 29), may also be used in the practice of the invention and leads to a further preferred embodiment of the invention. These VLPs, AP205 VLPs derived from natural sources, or AP205 viral particles, may be bound to antigens to produce ordered repetitive arrays of the antigens in accordance with the present invention.

AP205 P5-T mutant coat protein can be expressed from plasmid pAP281-32 (SEQ ID No. 30), which is derived directly from pQb185, and which contains the mutant AP205 coat protein gene instead of the Qβ coat protein gene. Vectors for expression of the AP205 coat protein are transfected into *E. coli* for expression of the AP205 coat protein.

Methods for expression of the coat protein and the mutant coat protein, respectively, leading to the self-assembly into VLPs are described in Examples 20 and 21. Suitable *E. coli* strains include, but are not limited to, *E. coli* K802, JM 109, RR1. Suitable vectors and strains and combinations thereof can be identified by testing expression of the coat protein and mutant coat protein, respectively, by SDS-PAGE and capsid formation and assembly by optionally first purifying the capsids by gel filtration and subsequently testing them in an immunodiffusion assay (Ouchterlony test) or Electron Microscopy (Kozlovska, T. M. et al., Gene 137:133-37 (1993)).

AP205 coat proteins expressed from the vectors pAP283-58 and pAP281-32 may be devoid of the initial Methionine amino-acid, due to processing in the cytoplasm of *E. coli.* Cleaved, uncleaved forms of AP205 VLP or mixtures thereof are further preferred embodiments of the invention.

In a further preferred embodiment of the present invention, the virus-like particle comprises, or alternatively essentially consists of, or alternatively consists of a mixture of recombinant coat proteins, or fragments thereof, of the RNA-phage AP205 and of recombinant mutant coat proteins, or fragments thereof, of the RNA-phage AP205.

In a further preferred embodiment of the present invention, the virus-like particle comprises, or alternatively essentially consists of, or alternatively consists of fragments of recombinant coat proteins or recombinant mutant coat proteins of the RNA-phage AP205.

Recombinant AP205 coat protein fragments capable of assembling into a VLP and a capsid, respectively are also useful in the practice of the invention. These fragments may be generated by deletion, either internally or at the termini of the coat protein and mutant coat protein, respectively. Insertions in the coat protein and mutant coat protein sequence or fusions of antigen sequences to the coat protein and mutant coat protein sequence, and compatible with assembly into a VLP, are further embodiments of the invention and lead to chimeric AP205 coat proteins, and particles, respectively. The outcome of insertions, deletions and fusions to the coat protein sequence and whether it is compatible with assembly into a VLP can be determined by electron microscopy.

The particles formed by the AP205 coat protein, coat protein fragments and chimeric coat proteins described above, can be isolated in pure form by a combination of fractionation steps by precipitation and of purification steps by gel filtration using *e*.*g*. Sepharose CL-4B, Sepharose CL-2B, Sepharose CL-6B columns and combinations thereof. Other methods of isolating virus-like particles are known in the art, and may be used to isolate the virus-like particles (VLPs) of bacteriophage AP205. For example, the use of ultracentrifugation to isolate VLPs of the yeast retrotransposon Ty is described in U.S. Patent No. 4,918,166.

The crystal structure of several RNA bacteriophages has been determined (Golmohammadi, R. et al., Structure 4:543-554 (1996)). Using such information, surface exposed residues can be identified and, thus, RNA-phage coat proteins can be modified such that one or more reactive amino acid residues can be inserted by way of insertion or substitution. As a consequence, those modified forms of bacteriophage coat proteins can also be used for the present invention. Thus, variants of proteins which form capsids or capsid-like structures (*e*.*g*., coat proteins of bacteriophage Qβ, bacteriophage R17, bacteriophage fr, bacteriophage GA, bacteriophage SP, bacteriophage AP205, and bacteriophage MS2) can also be used to prepare compositions of the present invention.

Although the sequence of the variants proteins discussed above will differ from their wild-type counterparts, these variant proteins will generally retain the ability to form capsids or capsid-like structures. Thus, the invention further includes compositions and vaccine compositions, respectively, which further includes variants of proteins which form capsids or capsid-like structures, as well as methods for preparing such compositions and vaccine compositions, respectively, individual protein subunits used to prepare such compositions, and nucleic acid molecules which encode these protein subunits. Thus, included within the scope of the invention are variant forms of wild-type proteins which form capsids or capsid-like structures and retain the ability to associate and form capsids or capsid-like structures.

As a result, the invention further includes compositions and vaccine compositions, respectively, comprising proteins, which comprise, or alternatively consist essentially of, or alternatively consist of amino acid sequences which are at least 80%, 85%, 90%, 95%, 97%, or 99% identical to wild-type proteins which form ordered arrays and having an inherent repetitive structure, respectively.

Further included within the scope of the invention are nucleic acid molecules which encode proteins used to prepare compositions of the present invention.

In other embodiments, the invention further includes compositions comprising proteins, which comprise, or alternatively consist essentially of, or alternatively consist of amino acid sequences which are at least 80%, 85%, 90%, 95%, 97%, or 99% identical to any of the amino acid sequences shown in SEQ ID NOs:4-21.

Proteins suitable for use in the present invention also include C-terminal truncation mutants of proteins which form capsids or capsid-like structures, or VLPs. Specific examples of such truncation mutants include proteins having an amino acid sequence shown in any of SEQ ID NOs:4-21 where 1, 2, 5, 7, 9, 10, 12, 14, 15, or 17 amino acids have been removed from the C-terminus. Typically, theses C-terminal truncation mutants will retain the ability to form capsids or capsid-like structures.

Further proteins suitable for use in the present invention also include N-terminal truncation mutants of proteins which form capsids or capsid-like structures. Specific examples of such truncation mutants include proteins having an amino acid sequence shown in any of SEQ ID NOs:4-21 where 1, 2, 5, 7, 9, 10, 12, 14, 15, or 17 amino acids have been removed from the N-terminus. Typically, these N-terminal truncation mutants will retain the ability to form capsids or capsid-like structures.

Additional proteins suitable for use in the present invention include N- and C-terminal truncation mutants which form capsids or capsid-like structures. Suitable truncation mutants include proteins having an amino acid sequence shown in any of SEQ ID NOs:4-21 where 1, 2, 5, 7, 9, 10, 12, 14, 15, or 17 amino acids have been removed from the N-terminus and 1, 2, 5, 7, 9, 10, 12, 14, 15, or 17 amino acids have been removed from the C-terminus. Typically, these N-terminal and C-terminal truncation mutants will retain the ability to form capsids or capsid-like structures.

The invention further includes compositions comprising proteins which comprise, or alternatively consist essentially of, or alternatively consist of, amino acid sequences which are at least 80%, 85%, 90%, 95%, 97%, or 99% identical to the above described truncation mutants.

The invention thus includes compositions and vaccine compositions prepared from proteins which form capsids or VLPs, methods for preparing these compositions from individual protein subunits and VLPs or capsids, methods for preparing these individual protein subunits, nucleic acid molecules which encode these subunits, and methods for vaccinating and/or eliciting immunological responses in individuals using these compositions of the present invention.

In one embodiment, the invention provides a vaccine composition of the invention further comprising an adjuvant In another embodiment, the vaccine composition of is devoid of an adjuvant In a further embodiment of the invention, the vaccine composition comprises a core particle of the invention, wherein the core particle comprises, preferably is, a virus-like particle, wherein preferably said virus-like particle is a recombinant virus-like particle. Preferably, the virus-like particle comprises, or alternatively consist essentially of, or alternatively consists of, recombinant proteins, or fragments thereof, of a RNA-phage, preferably of coat proteins of RNA phages,. In a preferred embodiment, the coat protein of the RNA phages has an amino acid are selected from the group consisting of: (a) SEQ ID NO:4; (b) a mixture of SEQ ID NO:4 and SEQ ID NO:5; (c) SEQ ID NO:6; (d) SEQ ID NO:7; (e) SEQ ID NO:8; (f) SEQ ID NO:9; (g) a mixture of SEQ ID NO:9 and SEQ ID NO: 10; (h) SEQ ID NO: 11; (i) SEQ ID NO:12; (k) SEQ ID NO:13; (1) SEQ ID NO:14; (m) SEQ ID NO:15; (n) SEQ ID NO:16; and (o) SEQ ID NO:28. Alternatively, the recombinant proteins of the virus-like particle of the vaccine composition of the invention comprise, or alternatively consist essentially of, or alternatively consist of mutant coat proteins of RNA phages, wherein the RNA-phage is selected from the group consisting of: (a) bacteriophage Qβ; (b) bacteriophage R17; (c) bacteriophage fr; (d) bacteriophage GA; (e) bacteriophage SP; (f) bacteriophage MS2; (g) bacteriophage M11; (h) bacteriophage MX1; (i) bacteriophage NL95; (k) bacteriophage f2; (l) bacteriophage PP7; and (m) bacteriophage AP205.

In a preferred embodiment, the mutant coat proteins of said RNA phage have been modified by removal, or by addition of at least one lysine residue by way of substitution. In another preferred embodiment, the mutant coat proteins of said RNA phage have been modified by deletion of at least one lysine residue or by addition of at least one lysine residue by way of insertion. In a preferred embodiment, the virus-like particle comprises recombinant proteins or fragments thereof, of RNA-phage Qβ, or alternatively of RNA-phage fr, or of RNA-phage AP205.

As previously stated, the invention includes virus-like particles or recombinant forms thereof. In one further preferred embodiment, the particles used in compositions of the invention are composed of a Hepatitis B core protein (HBcAg) or a fragment of a HBcAg. In a further embodiment, the particles used in compositions of the invention are composed of a Hepatitis B core protein (HBcAg) or a fragment of a HBcAg protein, which has been modified to either eliminate or reduce the number of free cysteine residues. Zhou et al. (J. Virol. 66:5393-5398 (1992)) demonstrated that HBcAgs which have been modified to remove the naturally resident cysteine residues retain the ability to associate and form capsids. Thus, VLPs suitable for use in compositions of the invention include those comprising modified HBcAgs, or fragments thereof, in which one or more of the naturally resident cysteine residues have been either deleted or substituted with another amino acid residue (*e*.*g*., a serine residue).

The HBcAg is a protein generated by the processing of a Hepatitis B core antigen precursor protein. A number of isotypes of the HBcAg have been identified and their amino acids sequences are readily available to those skilled in the art. In most instances, compositions and vaccine compositions, respectively, of the invention will be prepared using the processed form of a HBcAg (*i.e*., an HBcAg from which the N-terminal leader sequence of the Hepatitis B core antigen precursor protein has been removed).

Further, when HBcAgs are produced under conditions where processing will not occur, the HBcAgs will generally be expressed in "processed" form. For example, when an *E*. *coli* expression system directing expression of the protein to the cytoplasm is used to produce HBcAgs of the invention, these proteins will generally be expressed such that the N-terminal leader sequence of the Hepatitis B core antigen precursor protein is not present.

The preparation of Hepatitis B virus-like particles, which can be used for the present invention, is disclosed, for example, in WO 00/32227, and hereby in particular in Examples 17 to 19 and 21 to 24, as well as in WO 01/85208, and hereby in particular in Examples 17 to 19, 21 to 24, 31 and 41, and in WO 02/056905. For the latter application, it is in particular referred to Example 23, 24, 31 and 51.

The present invention also includes HBcAg variants which have been modified to delete or substitute one or more additional cysteine residues. It is known in the art that free cysteine residues can be involved in a number of chemical side reactions. These side reactions include disulfide exchanges, reaction with chemical substances or metabolites that are, for example, injected or formed in a combination therapy with other substances, or direct oxidation and reaction with nucleotides upon exposure to UV light. Toxic adducts could thus be generated, especially considering the fact that HBcAgs have a strong tendency to bind nucleic acids. The toxic adducts would thus be distributed between a multiplicity of species, which individually may each be present at low concentration, but reach toxic levels when together.

In view of the above, one advantage to the use of HBcAgs in vaccine compositions which have been modified to remove naturally resident cysteine residues is that sites to which toxic species can bind when antigens or antigenic determinants are attached would be reduced in number or eliminated altogether.

A number of naturally occurring HBcAg variants suitable for use in the practice of the present invention have been identified. Yuan et al., (J. Virol. 73:10122-10128 (1999)), for example, describe variants in which the isoleucine residue at position corresponding to position 97 in SEQ ID NO:22 is replaced with either a leucine residue or a phenylalanine residue. The amino acid sequences of a number of HBcAg variants, as well as several Hepatitis B core antigen precursor variants, are disclosed in GenBank reports AAF121240, AF121239, X85297, X02496, X85305, X85303, AF151735, X85259, X85286, X85260, X85317, X85298, AF043593, M20706, X85295, X80925, X85284, X85275, X72702, X85291, X65258, X85302, M32138, X85293, X85315, U95551, X85256, X85316, X85296, AB033559, X59795, X85299, X85307, X65257, X85311, X85301 (SEQ ID NO:23), X85314, X85287, X85272, X85319, AB010289, X85285, AB010289, AF121242, M90520 (SEQ ID NO:24), P03153, AF110999, and M95589. The sequences of the hereinabove mentioned Hepatitis B core antigen precursor variants are further disclosed in WO 01/85208 in SEQ ID NOs: 89 - 138. These HBcAg variants differ in amino acid sequence at a number of positions, including amino acid residues which corresponds to the amino acid residues located at positions 12, 13, 21, 22, 24, 29, 32, 33, 35, 38, 40, 42, 44, 45, 49, 51, 57, 58, 59, 64, 66, 67, 69, 74, 77, 80, 81, 87, 92, 93, 97, 98, 100, 103, 105, 106, 109, 113, 116, 121, 126, 130, 133, 135, 141, 147, 149, 157, 176, 178, 182 and 183 in SEQ OD NO:25. Further HBcAg variants suitable for use in the compositions of the invention, and which may be further modified according to the disclosure of this specification are described in WO 00/198333, WO 00/177158 and WO 00/214478.

As noted above, generally processed HBcAgs (*i*.*e*., those which lack leader sequences) will be used in the compositions and vaccine compositions, respectively, of the invention. The present invention includes vaccine compositions, as well as methods for using these compositions, which employ the above described variant HBcAgs.

Whether the amino acid sequence of a polypeptide has an amino acid sequence that is at least 80%, 85%, 90%, 95%, 97% or 99% identical to one of the above wild-type amino acid sequences, or a subportion thereof, can be determined conventionally using known computer programs such the Bestfit program. When using Bestfit or any other sequence alignment program to determine whether a particular sequence is, for instance, 95% identical to a reference amino acid sequence, the parameters are set such that the percentage of identity is calculated over the full length of the reference amino acid sequence and that gaps in homology of up to 5% of the total number of amino acid residues in the reference sequence are allowed.

The amino acid sequences of the hereinabove mentioned HBcAg variants and precursors are relatively similar to each other. Thus, reference to an amino acid residue of a HBcAg variant located at a position which corresponds to a particular position in SEQ ID NO:25, refers to the amino acid residue which is present at that position in the amino acid sequence shown in SEQ ID NO:25. The homology between these HBcAg variants is for the most part high enough among Hepatitis B viruses that infect mammals so that one skilled in the art would have little difficulty reviewing both the amino acid sequence shown in SEQ ID NO:25 and that of a particular HBcAg variant and identifying "corresponding" amino acid residues. Furthermore, the HBcAg amino acid sequence shown in SEQ ID NO:24, which shows the amino acid sequence of a HBcAg derived from a virus which infect woodchucks, has enough homology to the HBcAg having the amino acid sequence shown in SEQ ID NO:25 that it is readily apparent that a three amino acid residue insert is present in SEQ ID NO:23 between amino acid residues 155 and 156 of SEQ ID NO:25.

The invention also includes vaccine compositions which comprise HBcAg variants of Hepatitis B viruses which infect birds, as wells as vaccine compositions which comprise fragments of these HBcAg variants. For these HBcAg variants one, two, three or more of the cysteine residues naturally present in these polypeptides could be either substituted with another amino acid residue or deleted prior to their inclusion in vaccine compositions of the invention.

As discussed above, the elimination of free cysteine residues reduces the number of sites where toxic components can bind to the HBcAg, and also eliminates sites where cross-linking of lysine and cysteine residues of the same or of neighboring HBcAg molecules can occur. Therefore, in another embodiment of the present invention, one or more cysteine residues of the Hepatitis B virus capsid protein have been either deleted or substituted with another amino acid residue.

In other embodiments, compositions and vaccine compositions, respectively, of the invention will contain HBcAgs from which the C-terminal region (*e*.*g*., amino acid residues 145-185 or 150-185 of SEQ ID NO:25) has been removed. Thus, additional modified HBcAgs suitable for use in the practice of the present invention include C-terminal truncation mutants. Suitable truncation mutants include HBcAgs where 1, 5, 10, 15, 20, 25, 30, 34, 35, amino acids have been removed from the C-terminus.

HBcAgs suitable for use in the practice of the present invention also include N-terminal truncation mutants. Suitable truncation mutants include modified HBcAgs where 1, 2, 5, 7, 9, 10, 12, 14, 15, or 17 amino acids have been removed from the N-terminus.

Further HBcAgs suitable for use in the practice of the present invention include N- and C-terminal truncation mutants. Suitable truncation mutants include HBcAgs where 1, 2, 5, 7, 9, 10, 12, 14, 15, and 17 amino acids have been removed from the N-terminus and 1, 5, 10, 15, 20, 25, 30, 34, 35 amino acids have been removed from the C-terminus.

The invention further includes compositions and vaccine compositions, respectively, comprising HBcAg polypeptides comprising, or alternatively essentially consisting of, or alternatively consisting of, amino acid sequences which are at least 80%, 85%, 90%, 95%, 97%, or 99% identical to the above described truncation mutants.

In certain embodiments of the invention, a lysine residue is introduced into a HBcAg polypeptide, to mediate the binding of ghrelin or ghrelin peptide to the VLP of HBcAg. In preferred embodiments, compositions of the invention are prepared using a HBcAg comprising, or alternatively consisting of, amino acids 1-144, or 1-149, 1-185 of SEQ ID NO:25, which is modified so that the amino acids corresponding to positions 79 and 80 are replaced with a peptide having the amino acid sequence of Gly-Gly-Lys-Gly-Gly (SEQ ID NO:33) resulting in the HBcAg polypeptide having the sequence shown in SEQ ID NO: 26). In further preferred embodiments, the cysteine residues at positions 48 and 107 of SEQ ID NO:25 are mutated to serine. The invention further includes compositions comprising the corresponding polypeptides having amino acid sequences shown in any of the hereinabove mentioned Hepatitis B core antigen precursor variants, which also have above noted amino acid alterations. Further included within the scope of the invention are additional HBcAg variants which are capable of associating to form a capsid or VLP and have the above noted amino acid alterations. Thus, the invention further includes compositions and vaccine compositions, respectively, comprising HBcAg polypeptides which comprise, or alternatively consist of, amino acid sequences which are at least 80%, 85%, 90%, 95%, 97% or 99% identical to any of the wild-type amino acid sequences, and forms of these proteins which have been processed, where appropriate, to remove the N-terminal leader sequence and modified with above noted alterations.

Compositions or vaccine compositions of the invention may comprise mixtures of different HBcAgs. Thus, these vaccine compositions may be composed of HBcAgs which differ in amino acid sequence. For example, vaccine compositions could be prepared comprising a "wild-type" HBcAg and a modified HBcAg in which one or more amino acid residues have been altered (*e*.*g*., deleted, inserted or substituted). Further, preferred vaccine compositions of the invention are those which present highly ordered and repetitive antigen array, wherein the antigen is ghrelin or a ghrelin peptide.

In a further preferred embodiment of the present invention, the at least one ghrelin or ghrelin peptide is bound to said core particle and virus-like particle, respectively, by at least one covalent bond. Preferably, the least one ghrelin or ghrelin peptide is bound to the core particle and virus-like particle, respectively, by at least one covalent bond, said covalent bond being a non-peptide bond leading to a core particle-ghrelin ordered and repetitive array and a ghrelin-VLP-array or - conjugate, respectively. This ghrelin-VLP array and conjugate, respectively, has typically and preferably a repetitive and ordered structure since the at least one, but usually more than one, ghrelin or a ghrelin peptide is bound to the VLP and core particle, respectively, in an oriented manner. Preferably, more than, 120, more than 180, preferably more than 270 , and preferably more than 360 ghrelin or ghrelin peptides are bound to the VLP. The formation of a repetitive and ordered ghrelin-VLP and core particle, respectively, -array and conjugate, respectively, is ensured by an oriented and directed as well as defined binding and attachment, respectively, of the at least one ghrelin or ghrelin peptide to the VLP and core particle, respectively, as will become apparent in the following. Furthermore, the typical inherent highly repetitive and organized structure of the VLPs and core particles, respectively, advantageously contributes to the display of the ghrelin or ghrelin peptide in a highly ordered and repetitive fashion leading to a highly organized and repetitive ghrelin-VLP/core particle array and conjugate, respectively.

Therefore, the preferred inventive conjugates and arrays, respectively, differ from prior art conjugates in their highly organized structure, dimensions, and in the repetitiveness of the antigen on the surface of the array. The preferred embodiment of this invention, furthermore, allows expression of both the particle and the antigen in an expression host guaranteeing proper folding of the antigen, i.e. the at least one ghrelin or ghrelin peptide, and proper folding and assembly of the VLP.

The present invention discloses methods of binding of ghrelin or ghrelin peptide to core particles and VLPs, respectively. As indicated, in one aspect of the invention, the ghrelin or ghrelin peptide is bound to the core particle and VLP, respectively, by way of chemical cross-linking, typically and preferably by using a heterobifunctional cross-linker. Several hetero-bifunctional cross-linkers are known to the art. In preferred embodiments, the hetero-bifunctional cross-linker contains a functional group which can react with preferred first attachment sites, i.e. with the side-chain amino group of lysine residues of the core particle and the VLP or at least one VLP subunit, respectively, and a further functional group which can react with a preferred second attachment site, i.e. a cysteine residue naturally present, made available for reaction by reduction, or engineered on the ghrelin or ghrelin peptide, and optionally also made available for reaction by reduction. The first step of the procedure, typically called the derivatization, is the reaction of the core particle or the VLP with the cross-linker. The product of this reaction is an activated core particle or activated VLP, also called activated carrier. In the second step, unreacted cross-linker is removed using usual methods such as gel filtration or dialysis. In the third step, the ghrelin or ghrelin peptide is reacted with the activated carrier, and this step is typically called the coupling step. Unreacted ghrelin or ghrelin peptide may be optionally removed in a fourth step, for example by dialysis. Several hetero-bifunctional cross-linkers are known to the art. These include the preferred cross-linkers SMPH (Pierce), Sulfo-MBS, Sulfo-EMCS, Sulfo-GMBS, Sulfo-SIAB, Sulfo-SMPB, Sulfo-SMCC, SVSB, SIA and other cross-linkers available for example from the Pierce Chemical Company (Rockford, IL, USA), and having one functional group reactive towards amino groups and one functional group reactive towards cysteine residues. The above mentioned cross-linkers all lead to formation of an amide bond after reaction with the amino group and a thioether linkage with the cysteine. Another class of cross-linkers suitable in the practice of the invention is characterized by the introduction of a disulfide linkage between the ghrelin or ghrelin peptide and the core particle or VLP upon coupling. Preferred cross-linkers belonging to this class include for example SPDP and Sulfo-LC-SPDP (Pierce). The extent of derivatization of the core particle and VLP, respectively, with cross-linker can be influenced by varying experimental conditions such as the concentration of each of the reaction partners, the excess of one reagent over the other, the pH, the temperature and the ionic strength. The degree of coupling, i.e. the amount of ghrelin or ghrelin peptides per subunits of the core particle and VLP, respectively, can be adjusted by varying the experimental conditions described above to match the requirements of the vaccine. Solubility of the ghrelin or ghrelin peptide may impose a limitation on the amount of ghrelin or ghrelin peptide that can be coupled on each subunit and in those cases where the obtained vaccine would be insoluble reducing the amount of ghrelin or ghrelin peptide per subunit is beneficial.

A particularly favored method of binding of ghrelin or ghrelin peptide to the core particle and the VLP, respectively, is the linking of a lysine residue on the surface of the core particle and the VLP, respectively, with a cysteine residue on the ghrelin or ghrelin peptide. Thus, in a preferred embodiment of the present invention, the first attachment site is a lysine residue and the second attachment site is a cysteine residue. In some embodiments, engineering of an amino acid linker containing a cysteine residue, as a second attachment site or as a part thereof, to the ghrelin or ghrelin peptide for coupling to the core particle and VLP, respectively, may be required. Alternatively, a cysteine may be introduced either by insertion or mutation within the ghrelin or ghrelin peptide. Alternatively, the cysteine residue may be introduced by chemical coupling.

The selection of the amino acid linker will be dependent on the nature of the antigen and self-antigen, respectively, i.e. on the nature of the ghrelin or ghrelin peptide, on its biochemical properties, such as pI, charge distribution and glycosylation. In general, flexible amino acid linkers are favored. Preferred embodiments of the amino acid linker are selected from the group consisting of: (a) CGG; (b) N-terminal gamma 1-liner; (c) N-terminal gamma 3-linker; (d) Ig hinge regions; (e) N-terminal glycine linkers; (f) (G)ₖC(G)ₙ with n=0-12 and k=0-5 (SEQ ID NO: 34); (g) N-terminal glycine-serine linkers; (h) (G)ₖC(G)ₘ(S)ₗ(GGGGS)ₙ with n=0-3, k=0-5, m=0-10, 1=0-2 (SEQ ID NO: 35); (i) GGC; (k) GGC-NH2; (l) C-terminal gamma 1-linker; (m) C-terminal gamma 3-liner; (n) C-terminal glycine linkers; (o) (G)ₙC(G)ₖ with n=0-12 and k=0-5 (SEQ ID NO: 36); (p) C-terminal glycine-serine linkers; (q) (G)ₘ(S)ₗ(GGGGS)ₙ(G)ₒC(G)ₖ with n=0-3, k=0-5, m=0-10, l=0-2, and o=0-8 (SEQ ID NO: 37).

Further preferred examples of amino acid linkers are the hinge region of Immunoglobulins, glycine serine linkers (GGGGS)ₙ (SEQ ID NO: 38), and glycine linkers (G)ₙ all further containing a cysteine residue as second attachment site and optionally further glycine residues. Typically preferred examples of said amino acid linkers are N-terminal gamma1: CGDKTHTSPP (SEQ ID NO: 39); C-terminal gamma 1: DKTHTSPPCG (SEQ ID NO: 40); N-terminal gamma 3: CGGPKPSTPPGSSGGAP (SEQ ID NO: 41); C-terminal gamma 3: PKPSTPPGSSGGAPGGCG (SEQ ID NO: 42); N-terminal glycine linker: GCGGGG (SEQ ID NO: 43); C-terminal glycine linker: GGGGCG (SEQ ID NO: 44); C-terminal glycine-lysine linker: GGKKGC (SEQ ID NO: 45); N-terminal glycine-lysine linker: CGKKGG (SEQ ID NO: 46).

In a further preferred embodiment of the present invention, and in particular if the antigen is a ghrelin peptide, GGCG (SEQ ID NO: 47), GGC or GGC-NH2 ("NH2" stands for amidation) linkers at the C-terminus of the peptide or CGG at its N-terminus are preferred as amino acid linkers. In general, glycine residues will be inserted between bulky amino acids and the cysteine to be used as second attachment site, to avoid potential steric hindrance of the bulkier amino acid in the coupling reaction.

The cysteine residue present on the ghrelin or ghrelin peptide has to be in its reduced state to react with the hetero-bifunctional cross-linker on the activated VLP, that is a free cysteine or a cysteine residue with a free sulfhydryl group has to be available. In the instance where the cysteine residue to function as binding site is in an oxidized form, for example if it is forming a disulfide bridge, reduction of this disulfide bridge with *e*.*g*. DTT, TCEP or β-mercaptoethanol is required.

Binding of the ghrelin or ghrelin peptide to the core particle and VLP, respectively, by using a hetero-bifunctional cross-linker according to the preferred methods described above, allows coupling of the ghrelin or ghrelin peptide to the core particle and the VLP, respectively, in an oriented fashion. Other methods of binding the ghrelin or ghrelin peptide to the core particle and the VLP, respectively, include methods wherein the ghrelin or ghrelin peptide is cross-linked to the core particle and the VLP, respectively, using the carbodiimide EDC, and NHS. The ghrelin or ghrelin peptide may also be first thiolated through reaction, for example with SATA, SATP or iminothiolane. The ghrelin or ghrelin peptide, after deprotection if required, may then be coupled to the core particle and the VLP, respectively, as follows. After separation of the excess thiolation reagent, the ghrelin or ghrelin peptide is reacted with the core particle and the VLP, respectively, previously activated with a hetero-bifunctional cross-linker comprising a cysteine reactive moiety, and therefore displaying at least one or several functional groups reactive towards cysteine residues, to which the thiolated ghrelin or ghrelin peptide can react, such as described above. Optionally, low amounts of a reducing agent are included in the reaction mixture. In further methods, the ghrelin or ghrelin peptide is attached to the core particle and the VLP, respectively, using a homo-bifunctional cross-linker such as glutaraldehyde, DSG, BM[PEO]₄, BS³, (Pierce Chemical Company, Rockford, IL, USA) or other known homo-bifunctional cross-linkers with functional groups reactive towards amine groups or carboxyl groups of the core particle and the VLP, respectively,.

Other methods of binding the VLP to a ghrelin or ghrelin peptide include methods where the core particle and the VLP, respectively, is biotinylated, and the ghrelin or ghrelin peptide expressed as a streptavidin-fusion protein, or methods wherein both the ghrelin or ghrelin peptides and the core particle and the VLP, respectively, are biotinylated, for example as described in WO 00/23955. In this case, the ghrelin or ghrelin peptide may be first bound to streptavidin or avidin by adjusting the ratio of ghrelin or ghrelin peptide to streptavidin such that free binding sites are still available for binding of the core particle and the VLP, respectively, which is added in the next step. Alternatively, all components may be mixed in a "one pot" reaction. Other ligand-receptor pairs, where a soluble form of the receptor and of the ligand is available, and are capable of being cross-linked to the core particle and the VLP, respectively, or the ghrelin or ghrelin peptide, may be used as binding agents for binding the ghrelin or ghrelin peptide to the core particle and the VLP, respectively. Alternatively, either the ligand or the receptor may be fused to the ghrelin or ghrelin peptide, and so mediate binding to the core particle and the VLP, respectively, chemically bound or fused either to the receptor, or the ligand respectively. Fusion may also be effected by insertion or substitution.

As already indicated, in a favored embodiment of the present invention, the VLP is the VLP of a RNA phage, and in a more preferred embodiment, the VLP is the VLP of RNA phage Qβ coat protein.

One or several antigen molecules, i.e. a ghrelin or a ghrelin peptide, can be attached to one subunit of the capsid or VLP of RNA phages coat proteins, preferably through the exposed lysine residues of the VLP of RNA phages, if sterically allowable. A specific feature of the VLP of the coat protein of RNA phages and in particular of the Qβ coat protein VLP is thus the possibility to couple several antigens per subunit. This allows for the generation of a dense antigen array.

In a preferred embodiment of the invention, the binding and attachment, respectively, of the at least one ghrelin or ghrelin peptide to the core particle and the virus-like particle, respectively, is by way of interaction and association, respectively, between at least one first attachment site of the virus-like particle and at least one second attachment of the antigen or antigenic determinant. VLPs or capsids of Qβ coat protein display a defined number of lysine residues on their surface, with a defined topology with three lysine residues pointing towards the interior of the capsid and interacting with the RNA, and four other lysine residues exposed to the exterior of the capsid. These defined properties favor the attachment of antigens to the exterior of the particle, rather than to the interior of the particle where the lysine residues interact with RNA. VLPs of other RNA phage coat proteins also have a defined number of lysine residues on their surface and a defined topology of these lysine residues.

In further preferred embodiments of the present invention, the first attachment site is a lysine residue and/or the second attachment comprises sulfhydryl group or a cysteine residue. In a very preferred embodiment of the present invention, the first attachment site is a lysine residue and the second attachment is a cysteine residue.

In very preferred embodiments of the invention, the ghrelin or ghrelin peptide is bound via a cysteine residue, either naturally present on the ghrelin or ghrelin peptide or engineered, to lysine residues of the VLP of RNA phage coat protein, and in particular to the VLP of Qβ coat protein.

Another advantage of the VLPs derived from RNA phages is their high expression yield in bacteria that allows production of large quantities of material at affordable cost.

As indicated, the inventive conjugates and arrays, respectively, differ from prior art conjugates in their highly organized structure, dimensions, and in the repetitiveness of the antigen on the surface of the array. Moreover, the use of the VLPs as carriers allow the formation of robust antigen arrays and conjugates, respectively, with variable antigen density. In particular, the use of VLPs of RNA phages, and hereby in particular the use of the VLP of RNA phage Qβ coat protein allows achievement of a very high epitope density. The preparation of compositions of VLPs of RNA phage coat proteins with a high epitope density can be effected by using the teaching of this application. In prefered embodiment of the invention, when a ghrelin peptide is coupled to, preferably the VLP of Qβ coat protein, an average number of ghrelin peptide per subunit of 0.5, 0.6, 0.7, 0.8, 0.9, 1.0, 1.1, 1.2, 1.3, 1.4, 1.5, 1.6 , 1.7, 1.8, 1.9, 2.0, 2.1, 2.2, 2.3, 2.4 2.5, 2.6, 2.7, 2.8, 2.9, or higher is preferred.

The second attachment site, as defined herein, may be either naturally or non-naturally present with the antigen or the antigenic determinant. In the case of the absence of a suitable natural occurring second attachment site on the antigen or antigenic determinant, such a then non-natural second attachment has to be engineered to the antigen.

As described above, four lysine residues are exposed on the surface of the VLP of Qβ coat protein. Typically these residues are derivatized upon reaction with a cross-linker molecule. In the instance where not all of the exposed lysine residues can be coupled to an antigen, the lysine residues which have reacted with the cross-linker are left with a cross-linker molecule attached to the ε-amino group after the derivatization step. This leads to disappearance of one or several positive charges, which may be detrimental to the solubility and stability of the VLP. By replacing some of the lysine residues with arginines, as in the disclosed Qβ coat protein mutants described below, we prevent the excessive disappearance of positive charges since the arginine residues do not react with the cross-linker. Moreover, replacement of lysine residues by arginines may lead to more defined antigen arrays, as fewer sites are available for reaction to the antigen.

Accordingly, exposed lysine residues were replaced by arginines in the following Qβ coat protein mutants and mutant Qβ VLPs disclosed in this application: Qβ-240 (Lys13-Arg; SEQ ID NO:17), Qβ-250 (Lys 2-Arg, Lys13-Arg; SEQ ID NO:19) and Qβ**-**259 (Lys 2-Arg, Lys16-Arg; SEQ ID NO:21). The constructs were cloned, the proteins expressed, the VLPs purified and used for coupling to peptide and protein antigens. Qβ-251; (SEQ ID NO:20) was also constructed, and guidance on how to express, purify and couple the VLP of Qβ-251 coat protein can be found throughout the application.

In a further embodiment, we disclose a Qβ mutant coat protein with one additional lysine residue, suitable for obtaining even higher density arrays of antigens. This mutant Qβ coat protein, Qβ-243 (Asn 10-Lys; SEQ ID NO:18), was cloned, the protein expressed, and the capsid or VLP isolated and purified, showing that introduction of the additional lysine residue is compatible with self-assembly of the subunits to a capsid or VLP. Thus, ghrelin or ghrelin peptide arrays and conjugates, respectively, may be prepared using VLP of Qβ coat protein mutants. A particularly favored method of attachment of antigens to VLPs, and in particular to VLPs of RNA phage coat proteins is the linking of a lysine residue present on the surface of the VLP of RNA phage coat proteins with a cysteine residue naturally present or engineered on the antigen, i.e. the ghrelin or ghrelin peptide. In order for a cysteine residue to be effective as second attachment site, a sulfhydryl group must be available for coupling. Thus, a cysteine residue has to be in its reduced state, that is, a free cysteine or a cysteine residue with a free sulfhydryl group has to be available. In the instant where the cysteine residue to function as second attachment site is in an oxidized form, for example if it is forming a disulfide bridge, reduction of this disulfide bridge with *e*.*g*. DTT, TCEP or β-mercaptoethanol is required. The concentration of reductand, and the molar excess of reductant over antigen have to be adjusted for each antigen. A titration range, starting from concentrations as low as 10 µM or lower, up to 10 to 20 mM or higher reductant if required is tested, and coupling of the antigen to the carrier assessed. Although low concentrations of reductant are compatible with the coupling reaction as described in WO 02/056905, higher concentrations inhibit the coupling reaction, as a skilled artisan would know, in which case the reductant has to be removed by dialysis or gel filtration. Advantageously, the pH of the dialysis or equilibration buffer is lower than 7, preferably 6. The compatibility of the low pH buffer with antigen activity or stability has to be tested.

Epitope density on the VLP of RNA phage coat proteins can be modulated by the choice of cross-linker and other reaction conditions. For example, the cross-linkers Sulfo-GMBS and SMPH typically allow reaching high epitope density. Derivatization is positively influenced by high concentration of reactands, and manipulation of the reaction conditions can be used to control the number of antigens coupled to VLPs of RNA phage coat proteins, and in particular to VLPs of Qβ coat protein.

Prior to the design of a non-natural second attachment site the position at which it should be fused, inserted or generally engineered has to be chosen. Thus, the location of the second attachment site will be selected such that steric hindrance from the second attachment site or any amino acid linker containing the same is avoided. In further embodiments, an antibody response directed at a site distinct from the interaction site of the self-antigen with its natural ligand is desired. In such embodiments, the second attachment site may be selected such that it prevents generation of antibodies against the interaction site of the self-antigen with its natural ligands.

In the most preferred embodiments, the ghrelin or ghrelin peptide comprises a single second attachment site or a single reactive attachment site capable of association with the first attachment sites on the core particle and the VLPs or VLP subunits, respectively. This ensures a defined and uniform binding and association, respectively, of the at least one, but typically more than one, preferably more than 10, 20, 40, 80, 120, 150, 180, 210, 240, 270, 300, 360, 400, 450 antigens to the core particle and VLP, respectively. The provision of a single second attachment site or a single reactive attachment site on the antigen, thus, ensures a single and uniform type of binding and association, respectively leading to a very highly ordered and repetitive array. For example, if the binding and association, respectively, is effected by way of a lysine- (as the first attachment site) and cysteine- (as a second attachment site) interaction, it is ensured, in accordance with this preferred embodiment of the invention, that only one cysteine residue per antigen, independent whether this cysteine residue is naturally or non-naturally present on the antigen, is capable of binding and associating, respectively, with the VLP and the first attachment site of the core particle, respectively.

In some embodiments, engineering of a second attachment site onto the antigen require the fusion of an amino acid linker containing an amino acid suitable as second attachment site according to the disclosures of this invention. Therefore, in a preferred embodiment of the present invention, an amino acid linker is bound to the antigen or the antigenic determinant by way of at least one covalent bond. Preferably, the amino acid linker comprises, or alternatively consists of, the second attachment site. In a further preferred embodiment, the amino acid linker comprises a sulfhydryl group or a cysteine residue. In another preferred embodiment, the amino acid linker is cysteine. Some criteria of selection of the amino acid linker as well as further preferred embodiments of the amino acid linker according to the invention have already mentioned above.

In a further preferred embodiment of the invention, the at least one antigen or antigenic determinant, i.e. the ghrelin or ghrelin peptide is fused to the core particle and the virus-like particle, respectively. As outlined above, a VLP is typically composed of at least one subunit assembling into a VLP. Thus, in again a further preferred embodiment of the invention, the antigen or antigenic determinant, preferably the at least one ghrelin or ghrelin peptide, is fused to at least one subunit of the virus-like particle or of a protein capable of being incorporated into a VLP generating a chimeric VLP-subunit-ghrelin or ghrelin peptide protein fusion.

Fusion of ghrelin or ghrelin peptides can be effected by insertion into the VLP subunit sequence, or by fusion to either the N- or C-terminus of the VLP-subunit or protein capable of being incorporated into a VLP. Hereinafter, when referring to fusion proteins of a peptide to a VLP subunit, the fusion to either ends of the subunit sequence or internal insertion of the peptide within the subunit sequence are encompassed.

Fusion may also be effected by inserting the ghrelin or ghrelin peptide sequences into a variant of a VLP subunit where part of the subunit sequence has been deleted, that are further referred to as truncation mutants. Truncation mutants may have N- or C-terminal, or internal deletions of part of the sequence of the VLP subunit. For example, the specific VLP HBcAg with, for example, deletion of amino acid residues 79 to 81 is a truncation mutant with an internal deletion. Fusion of ghrelin or ghrelin peptide to either the N- or C-terminus of the truncation mutants VLP-subunits also lead to embodiments of the invention. Likewise, fusion of an epitope into the sequence of the VLP subunit may also be effected by substitution, where for example for the specific VLP HBcAg, amino acids 79-81 are replaced with a foreign epitope. Thus, fusion, as referred to hereinafter, may be effected by insertion of the ghrelin or ghrelin peptide sequence in the sequence of a VLP subunit, by substitution of part of the sequence of the VLP subunit with the ghrelin or ghrelin peptide sequence, or by a combination of deletion, substitution or insertions.

The chimeric ghrelin or ghrelin peptide VLP subunit will be in general capable of self-assembly into a VLP. VLP displaying epitopes fused to their subunits are also herein referred to as chimeric VLPs. As indicated, the virus-like particle comprises or alternatively is composed of at least one VLP subunit. In a further embodiment of the invention, the virus-like particle comprises or alternatively is composed of a mixture of chimeric VLP subunits and non-chimeric VLP subunits, i.e. VLP subunits not having an antigen fused thereto, leading to so called mosaic particles. This may be advantageous to ensure formation of and assembly to a VLP. In those embodiments, the proportion of chimeric VLP-subunits may be 1, 2, 5, 10, 20, 30, 40, 50, 60, 70, 80, 90, 95% or higher.

Flanking amino acid residues may be added to either end of the sequence of the peptide or epitope to be fused to either end of the sequence of the subunit of a VLP, or for internal insertion of such peptidic sequence into the sequence of the subunit of a VLP. Glycine and serine residues are particularly favored amino acids to be used in the flanking sequences added to the ghrelin or ghrelin peptide to be fused. Glycine residues confer additional flexibility, which may diminish the potentially destabilizing effect of fusing a foreign sequence into the sequence of a VLP subunit.

In a specific embodiment of the invention, the VLP is a Hepatitis B core antigen VLP. Fusion proteins to either the N-terminus of HBcAg (Neyrinck, S. et al., Nature Med. 5:1157-1163 (1999)) or insertions in the so called major immunodominant region (MIR) have been described (Pumpens, P. and Grens, E., Intervirology 44:98-114 (2001)), WO 01/98333), and are preferred embodiments of the invention. Naturally occurring variants of HBcAg with deletions in the MIR have also been described (Pumpens, P. and Grens, E., Intervirology 44:98-114 (2001), which is expressly incorporated by reference in their entirety), and fusions to the Nor C-terminus, as well as insertions at the position of the MIR corresponding to the site of deletion as compared to a wt HBcAg are further embodiments of the invention. Fusions to the C-terminus have also been described (Pumpens, P. and Grens, E., Intervirology 44:98-114 (2001)). One skilled in the art will easily find guidance on how to construct fusion proteins using classical molecular biology techniques (Sambrook, J.et al., eds., Molecular Cloning, A Laboratory Manual, 2nd. edition, Cold Spring Habor Laboratory Press, Cold Spring Harbor, N.Y. (1989), Ho et al., Gene 77:51 (1989)). Vectors and plasmids encoding HBcAg and HBcAg fusion proteins and useful for the expression of a HBcAgs and HBcAg fusion proteins have been described (Pumpens, P. & Grens, E. Intervirology 44: 98-114 (2001), Neyrinck, S. et al., Nature Med. 5:1157-1163 (1999)) and can be used in the practice of the invention. We also describe by way of example (Example 6) the insertion of an epitope into the MIR of HBcAg, resulting in a chimeric self-assembling HBcAg. An important factor for the optimization of the efficiency of self-assembly and of the display of the epitope to be inserted in the MIR of HBcAg is the choice of the insertion site, as well as the number of amino acids to be deleted from the HBcAg sequence within the MIR (Pumpens, P. and Grens, E., Intervirology 44:98-114 (2001); EP 421'635; US 6'231'864) upon insertion, or in other words, which amino acids form HBcAg are to be substituted with the new epitope. For example, substitution of HBcAg amino acids 76-80, 79-81, 79-80, 75-85 or 80-81 with foreign epitopes has been described (Pumpens, P. and Grens, E., Intervirology 44:98-114 (2001); EP0421635; US 6'231'864). HBcAg contains a long arginine tail (Pumpens, P. and Grens, E., Intervirology 44:98-114 (2001)) which is dispensable for capsid assembly and capable of binding nucleic acids (Pumpens, P. and Grens, E., Intervirology 44:98-114 (2001)). HBcAg either comprising or lacking this arginine tail are both embodiments of the invention.

In a further preferred embodiment of the invention, the VLP is a VLP of a RNA phage. The major coat proteins of RNA phages spontaneously assemble into VLPs upon expression in bacteria, and in particular in *E. coli.* Specific examples of bacteriophage coat proteins which can be used to prepare compositions of the invention include the coat proteins of RNA bacteriophages such as bacteriophage Qβ (SEQ ID NO:4; PIR Database, Accession No. VCBPQβ referring to Qβ CP and SEQ ID NO: 5; Accession No. AAA16663 referring to Qβ A1 protein) and bacteriophage fr (SEQ ID NO:7; PIR Accession No. VCBPFR).

In a more preferred embodiment, the at least one ghrelin or ghrelin peptide is fused to a Qβ coat protein. Fusion protein constructs wherein epitopes have been fused to the C-terminus of a truncated form of the A1 protein of Qβ, or inserted within the A1 protein have been described (Kozlovska, T. M., et al., Intervirology, 39:9-15 (1996)). The A1 protein is generated by suppression at the UGA stop codon and has a length of 329 aa, or 328 aa, if the cleavage of the N-terminal methionine is taken into account. Cleavage of the N-terminal methionine before an alanine (the second amino acid encoded by the Qβ CP gene) usually takes place in *E. coli,* and such is the case for N-termini of the Qβ coat proteins CP. The part of the A1 gene, 3' of the UGA amber codon encodes the CP extension, which has a length of 195 amino acids. Insertion of the at least one ghrelin or ghrelin peptide between position 72 and 73 of the CP extension leads to further embodiments of the invention (Kozlovska, T. M., et al., Intervirology 39:9-15 (1996)). Fusion of a ghrelin or ghrelin peptide at the C-terminus of a C-terminally truncated Qβ A1 protein leads to further preferred embodiments of the invention. For example, Kozlovska et al, (Intervirology, 39: 9-15 (1996)) describe Qβ A1 protein fusions where the epitope is fused at the C-terminus of the Qβ CP extension truncated at position 19.

As described by Kozlovska et al. (Intervirology, 39: 9-15 (1996)), assembly of the particles displaying the fused epitopes typically requires the presence of both the A1 protein-ghrelin or ghrelin peptide fusion and the wt CP to form a mosaic particle. However, embodiments comprising virus-like particles, and hereby in particular the VLPs of the RNA phage Qβ coat protein, which are exclusively composed of VLP subunits having at least one ghrelin or ghrelin peptide fused thereto, are also within the scope of the present invention.

The production of mosaic particles may be effected in a number of ways. Kozlovska et al., Intervirolog, 39:9-15 (1996), describe two methods, which both can be used in the practice of the invention. In the first approach, efficient display of the fused epitope on the VLPs is mediated by the expression of the plasmid encoding the Qβ A protein fusion having a UGA stop codong between CP and CP extension in a *E. coli* strain harboring a plasmid encoding a cloned UGA suppressor tRNA which leads to translation of the UGA codon into Trp (pISM3001 plasmid (Smiley B.K., et al., Gene 134:33-40 (1993))). In another approach, the CP gene stop codon is modified into UAA, and a second plasmid expressing the A1 protein-ghrelin or ghrelin peptide fusion is cotransformed. The second plasmid encodes a different antibiotic resistance and the origin of replication is compatible with the first plasmid (Kozlovska, T. M., et al., Intervirology 39:9-15 (1996)). In a third approach, CP and the A1 protein-ghrelin or ghrelin peptide fusion are encoded in a bicistronic manner, operatively linked to a promoter such as the Trp promoter, as described in FIG. 1 of Kozlovska et al., Intervirology, 39:9-15 (1996).

In a further embodiment, the ghrelin or ghrelin peptide is inserted between amino acid 2 and 3 (numbering of the cleaved CP, that is wherein the N-terminal methionine is cleaved) of the fr CP, thus leading to a ghrelin or ghrelin peptide-fr CP fusion protein. Vectors and expression systems for construction and expression of fr CP fusion proteins self-assembling to VLP and useful in the practice of the invention have been described (Pushko P. et al., Prot. Eng. 6:883-891 (1993)). In a specific embodiment, the ghrelin or ghrelin peptide sequence is inserted into a deletion variant of the fr CP after amino acid 2, wherein residues 3 and 4 of the fr CP have been deleted (Pushko P. et al., Prot. Eng. 6:883-891 (1993)).

Fusion of epitopes in the N-terminal protuberant β-hairpin of the coat protein of RNA phage MS-2 and subsequent presentation of the fused epitope on the self-assembled VLP of RNA phage MS-2 has also been described (WO 92/13081), and fusion of ghrelin or ghrelin peptide by insertion or substitution into the coat protein of MS-2 RNA phage is also falling under the scope of the invention.

In another embodiment of the invention, the ghrelin or ghrelin peptides are fused to a capsid protein of papillomavirus. In a more specific embodiment, the ghrelin or ghrelin peptides are fused to the major capsid protein L1 of bovine papillomavirus type 1 (BPV-1). Vectors and expression systems for construction and expression of BPV-1 fusion proteins in a baculovirus/insect cells systems have been described (Chackerian, B. et al., Proc. Natl. Acad. Sci. USA 96:2373-2378 (1999), WO 00/23955). Substitution of amino acids 130-136 of BPV-1 L1 with a ghrelin or ghrelin peptide leads to a BPV-1 L1- ghrelin or ghrelin peptide fusion protein, which is a preferred embodiment of the invention. Cloning in a baculovirus vector and expression in baculovirus infected Sf9 cells has been described, and can be used in the practice of the invention (Chackerian, B. et al., Proc. Natl. Acad. Sci. USA 96:2373-2378 (1999), WO 00/23955). Purification of the assembled particles displaying the fused ghrelin or ghrelin peptides can be performed in a number of ways, such as for example gel filtration or sucrose gradient ultracentrifugation (Chackerian, B. et al., Proc. Natl. Acad Sci. USA 96:2373-2378 (1999), WO 00/23955).

In a further embodiment of the invention, the ghrelin or ghrelin peptides are fused to a Ty protein capable of being incorporated into a Ty VLP. In a more specific embodiment, the ghrelin or ghrelin peptides are fused to the p1 or capsid protein encoded by the TYA gene (Roth, J.F., Yeast 16:785-795 (2000)). The yeast retrotransposons Ty1, 2, 3 and 4 have been isolated from *Saccharomyces Cerevisiae,* while the retrotransposon Tf1 has been isolated from Schizosaccharomyces Pombae (Boeke, J.D. and Sandmeyer, S.B., "Yeast Transposable elements," in The molecular and Cellular Biology of the Yeast Saccharomyces: Genome dynamics, Protein Synthesis, and Energetics., p. 193, Cold Spring Harbor Laboratory Press (1991)). The retrotransposons Ty1 and 2 are related to the *copia* class of plant and animal elements, while Ty3 belongs to the *gypsy* family of retrotransposons, which is related to plants and animal retroviruses. In the Ty1 retrotransposon, the p1 protein, also referred to as Gag or capsid protein has a length of 440 amino acids. P1 is cleaved during maturation of the VLP at position 408, leading to the p2 protein, the essential component of the VLP.

Fusion proteins to p1 and vectors for the expression of said fusion proteins in Yeast have been described (Adams, S.E., et al., Nature 329:68-70 (1987)). So, for example, a ghrelin or ghrelin peptide may be fused to p1 by inserting a sequence coding for the ghrelin or ghrelin peptide into the BamH1 site of the pMA5620 plasmid (Adams, S.E., et al., Nature 329:68-70 (1987)). The cloning of sequences coding for foreign epitopes into the pMA5620 vector leads to expression of fusion proteins comprising amino acids 1-381 of p1 of Ty1-15, fused C-terminally to the N-terminus of the foreign epitope. Likewise, N-terminal fusion of ghrelin or ghrelin peptides, or internal insertion into the p1 sequence, or substitution of part of the p1 sequence is also meant to fall within the scope of the invention. In particular, insertion of ghrelin or ghrelin peptides into the Ty sequence between amino acids 30-31, 67-68, 113-114 and 132-133 of the Ty protein p1 (EP0677111) leads to preferred embodiments of the invention.

Further VLPs suitable for fusion of ghrelin or ghrelin peptides are, for example, Retrovirus-like-particles (WO9630523), HIV2 Gag (Kang, Y.C., et al, Biol. Chem. 380:353-364 (1999)), Cowpea Mosaic Virus (Taylor, K.M.et al., Biol. Chem. 380:387-392 (1999)), parvovirus VP2 VLP (Rueda, P. et al., Virology 263:89-99 (1999)), HBsAg (US 4,722,840, EP0020416B1).

Examples of chimeric VLPs suitable for the practice of the invention are also those described in Intervirology 39:1 (1996). Further examples of VLPs contemplated for use in the invention are: BPV-1, HPV-6, HPV-11, HPV-16, HPV-18, HPV-33, HPV-45, CRPV, COPV, HIV GAG, Tobacco Mosaic Virus. Virus-like particles of SV-40, Polyomavirus, Adenovirus, Herpes Simplex Virus, Rotavirus and Norwalk virus have also been made, and chimeric VLPs of those VLPs are also within the scope of the present invention.

In a further preferred embodiment of the present invention, the antigen or antigenic determinant is ghrelin or a ghrelin peptide.

In a further very preferred embodiment of the invention, the antigen or antigenic determinant is selected form the group consisting of a) human ghrelin; b) cat ghrelin; c) dog ghrelin; d) bovine ghrelin; e) sheep ghrelin; f) horse ghrelin g) pig ghrelin; h)a peptide or a fragment thereof of any ghrelin of a)-g).

In a further very preferred embodiment of the invention, the antigen or antigenic determinant comprises, alternatively essentially consists of, or alternatively consists of an amino acid sequence selected from the group consisting of:
(a) GSSFLSPEHQRVQRKESKKPPAKLQPR (SEQ ID NO: 48)
(b) GSSFLSPEHQRVQQRKESKKPPAKLQPR (SEQ ID NO: 31)
(c) GSSFLSPEHQKLQQRKESKKPPAKLQPR (SEQ ID NO: 49)
(d) GSSFLSPEHQKLQRKESKKPPAKLQPR (SEQ ID NO: 50)
(e) GSSFLSPEHQKAQQRKESKKPPAKLQPR (SEQ ID NO: 32)
GSSFLSPEHQKAQRKESKKPPAKLQPR (SEQ ID NO: 51)
KKPPAKLQPR (SEQ ID NO: 52)
PPAKLQPR (SEQ ID NO: 53)
AKLQPR (SEQ ID NO: 54)
GSSFLSPEHQ (SEQ ID NO: 55)
EHQRVQQRKE (SEQ ID NO: 56)
EHQRVQQRKES (SEQ ID NO: 111)
EHQKAQQRKE (SEQ ID NO: 112)
EHQKAQQRKES (SEQ ID NO: 113)
EHQKLQQRKE (SEQ ID NO: 114)
EHQKLQQRKES (SEQ ID NO: 115)
LSPEHQRVQQ (SEQ ID NO: 116)
LSPEHQKAQQ (SEQ ID NO: 117)
(s) LSPEHQKLQQ (SEQ ID NO: 118).

In a further very preferred embodiment of the invention, the antigen or antigenic determinant is the human, cat, pig, horse, sheep, bovine, guinea pig, dog or mouse ghrelin and ghrelin peptide, respectively. Ghrelins and ghrelin peptides, respectively can be produced by expression of DNA encoding ghrelin and ghrelin peptide, respectively, under the control of a strong promotor. Preferably, the DNA does not encode the preproghrelin but only the peptidic backbone of the active n-octanoyl-modified peptide. Various examples hereto have been described in the literature and can be used, possibly after modifications, to express ghrelin of any desired species.

In a further preferred embodiment of the invention, the antigen or antigenic determinant is a ghrelin-derived peptide or a fragment thereof. Preferably the ghrelin peptide is selected from the group consisting: a) human ghrelin peptide; b) bovine ghrelin peptide; c) cat ghrelin peptide; d) dog ghrelin peptide e) pig ghrelin peptide; f) chicken ghrelin peptide; g) mouse ghrelin peptide; h) horse ghrelin peptide; and i) sheep ghrelin and j) a fragment of any ghrelin peptide of a)-i).

Such ghrelin peptides or fragments thereof can be produced using standard molecular biological technologies where the nucleotide sequence coding for the fragment of interest is amplified by PCR and is cloned as a fusion to a polypeptide tag, such as the histdine tag, the Flag tag, myc tag or the constant region of an antibody (Fc region). By introducing an enterokinase cleavage site between the ghrelin fragment and the tag, the ghrelin fragment can be separated from the tag after purification by digestion with enterokinase. In another approach the ghrelin fragment can be synthesized in vitro with or without n-octanoyl-modification using standard peptide synthesis reactions known to a person skilled in the art. In a further approach ghrelin fragments can be produced by protease digestion or chemical cleavage of the full length ghrelin, both methods which are well known to people trained in the art.

In a further preferred embodiment of the present invention, the ghrelin peptide comprises at least one antigenic site of ghrelin. The skilled person in the art knows how to identify the corresponding peptides and amino acid sequences, respectively. Preferable fragments may encompass the N or C terminus of ghrelin. The C-terminus may be particularly useful since the n-octanoyl-modification, which could perhaps interfere with the binding of antibodies specific for the non-modified form, is close to the N-terminus.

Preferred human ghrelin peptides would encompass:
KKPPAKLQPR (SEQ ID NO: 52)
PPAKLQPR (SEQ ID NO: 53)
AKLQPR (SEQ ID NO: 54)
KLQPR (SEQ ID NO: 59)
GSSFLSPEHQ (SEQ ID NO: 55)
EHQRVQQRKES (SEQ ID NO: 111)
LSPEHQRVQQ (SEQ ID NO: 116)
GSSFLSP (SEQ ID NO: 119)

However, peptides from ghrelin internal sites may also be attractive candidates for vaccines. Since ghrelins of various species are highly homologous, it is likely that cross-reactive antibody responses can be induced. Thus, antibody responses against dog or mouse ghrelin may also recognize human ghrelin and vice versa. It is therefore within the scope of this invention that all ghrelin molecules and peptides derived thereof with amino acid identities > than 80 %, preferably higher than 85%, more preferably higher than 90%, or even more preferably higher than 95%, 97% or even 99% to human ghrelin may be used for vaccination.

Guidance on how to modify human ghrelin or ghrelin peptide, in particular, for binding to the virus-like particle is given throughout the application. Immunization against ghrelin using the inventive compositions comprising, preferably human ghrelin or a ghrelin peptide bound to a core particle and VLP, respectively, may provide a way of treating obesity.

In a further very preferred embodiment of the present invention, the antigen or antigenic determinant is a ghrelin or a ghrelin peptide comprising, or alternatively essentially consisting of, or alternatively consisting of an amino acid sequence selected from the group consisting of:
(a) GSSFLSPEHQRVQRKESKKPPAKLQPR (human) (SEQ ID NO: 48)
(b) GSSFLSPEHQRVQQRKESKKPPAKLQPR (human) (SEQ ID NO: 31)
(c) GSSFLSPEHQRVQ (human) (SEQ ID NO: 60)
(d) QRKESKKPPAKLQPR (human) (SEQ ID NO: 61)
(e) PPAKLQPR (human) (SEQ ID NO: 53)
(f) GSSFLSPEHQKLQQRKESKKPPAKLQPR (dog) (SEQ ID NO: 49)
(g) GSSFLSPEHQKLQRKESKKPPAKLQPR (dog) (SEQ ID NO: 50)
(h) GSSFLSPEHQKLQ (dog) (SEQ ID NO: 62)
(i) QRKESKKPPAKLQPR (dog) (SEQ ID NO: 63)
(k) EHQRVQQRKE (human) (SEQ ID NO: 56)
(l) GSSFLSPEHQKAQQRKESKKPPAKLQPR (mouse) (SEQ ID NO: 32)
(n) the amino acid sequence of any fragment of any of (a)-(l).

In a still further preferred embodiment of the present invention, the antigen or antigenic determinant further comprise at least one second attachment site being selected from the group consisting of (i) an attachment site not naturally occurring with said antigen or antigenic determinant; and (ii) an attachment site naturally occurring with said antigen or antigenic determinant. In a preferred embodiment, said attachment site comprises an amino acid linker of the invention, preferably a linker sequence of C, CG, or CGG. Preferably, the antigen or antigenic determinant with said at least second attachment site comprises, or alternatively consists of, or alternatively consists of an amino acid sequence selected from the group consisting of:
CGSSFLSPEHQRVQRKESKKPPAKLQPR (human) (SEQ ID NO: 64)
CGSSFLSPEHQRVQQRKESKKPPAKLQPR (human) (SEQ ID NO: 65)
GSSFLSPEHQRVQRKESKKPPAKLQPRC (human) (SEQ ID NO: 66)
GSSFLSPEHQRVQRKESKKPPAKLQPRGC (human) (SEQ ID NO: 120)
GSSFLSPEHQRVQQRKESKKPPAKLQPRC (human) (SEQ ID NO: 67)
GSSFLSPEHQRVQQRKESKKPPAKLQPRGC (human) (SEQ ID NO: 121)
GSSFLSPEHQRVQC (human) (SEQ ID NO: 68)
GSSFLSPEHQRVQGC (human) (SEQ ID NO: 122)
CQRKESKKPPAKLQPR (human) (SEQ ID NO: 69)
CPPAKLQPR (human) (SEQ ID NO: 70)
CGSSFLSPEHQKLQQRKESKKPPAKLQPR (dog) (SEQ ID NO: 71)
CGSSFLSPEHQKLQRKESKKPPAKLQPR (dog) (SEQ ID NO: 72)
GSSFLSPEHQKLQQRKESKKPPAKLQPRC (dog) (SEQ ID NO: 73)
GSSFLSPEHQKLQQRKESKKPPAKLQPRGC (dog) (SEQ ID NO: 123)
GSSFLSPEHQKLQRKESKKPPAKLQPRC (dog) (SEQ ID NO: 74)
GSSFLSPEHQKLQRKESKKPPAKLQPRGC (dog) (SEQ ID NO: 124)
GSSFLSPEHQKLQC (SEQ ID NO: 75)
GSSFLSPEHQKLQGC (SEQ ID NO: 125)
CEHQRVQQRKE (SEQ ID NO: 76)
CGSSFLSPEHQKAQQRKESKKPPAKLQPR (mouse) (SEQ ID NO: 77)
GSSFLSPEHQKAQRKESKKPPAKLQPRC (mouse) (SEQ ID NO: 126)
GSSFLSPEHQKAQRKESKKPPAKLQPRGC (mouse) (SEQ ID NO: 127)
GSSFLSPEHQKAQQRKESKKPPAKLQPRC (mouse) (SEQ ID NO: 128)
GSSFLSPEHQKAQQRKESKKPPAKLQPRGC (mouse) (SEQ ID NO: 129)
GSSFLSPEHQKAQC (mouse) (SEQ ID NO: 130)
GSSFLSPEHQKAQGC (mouse) (SEQ ID NO: 131)
GGSSFLSPEHQGC (SEQ ID NO: 132)
CKKPPAKLQPR (SEQ ID NO: 133)
CEHQKAQQRKE (SEQ ID NO: 134)
CEHQKAQQRKES (SEQ ID NO: 135)
CLSPEHQKAQQ (SEQ ID NO: 136)
CEHQRVQQRKES (SEQ ID NO: 137)
CLSPEHQRVQQ (SEQ ID NO: 138)
the amino acid sequence of any fragment of any of (a)-(ee).

Some of the very preferred ghrelin peptides, are described in Example 13, wherein there, in particular, the corresponding murine peptides are provided. These peptides comprise an N- or C- terminal cysteine residue as a second attachment added for coupling to VLPs and Pili. These very preferred short ghrelin peptide fragments are capable of having a very enhanced immunogenicity when coupled to VLP and to a core particle, respectively. The preferred ghrelin fragments are, furthermore, capable of also overcoming safety issues that arise when targeting self-proteins as shorter fragment are much more less likely to contain T cell epitopes. Typically, the shorter the peptides, the safer with respect to T cell activation. However, too short peptides may fail to induce high-affinity antibodies that are able to strongly bind ghrelin in solution.

The very preferred ghrelin peptide fragments corresponding to the murine ghrelin fragment of residue 1-10 (GSSFLSPEHQ) (SEQ ID NO: 55) was chosen primarily because the N-terminal segment of ghrelin is identical among all known species. Additionally, it is likely to be identical in species where ghrelin has yet to be identified. Furthermore, the C-terminal residue, a glutamine, would enhance solubility, facilitating the production of a soluble vaccine product when coupled to VLP and to a core particle, respectively. In fact, the solubility of peptides is often a limiting factor for coupling efficiency and vaccine stability. Further reasoning includes avoiding a potential T cell epitope. The choice of a smaller peptide fragment reduces the probability of a T cell epitope being present. Coupling murine ghrelin residues 1-10 via the C-terminus to VLP will induce N-terminal specific antibodies when immunized into mice that are capable of binding active ghrelin and hence, prevent it's passing of the blood brain barrier, leading to reduced food intake.

The further very preferred ghrelin peptide fragments corresponding to the murine ghrelin peptide fragment of residues 42-51 (KKPPAKLQPR) (SEQ ID NO: 52) has been chosen for similar reasons to that above. The only difference being that this peptide fragment, coupled via the N-terminus, is capable of inducing antibodies specific for the C-terminal part of ghrelin when immunized into mice. Moreover, antibody recognition could be enhanced as the *n*-octanoyl group on serine 3 would be avoided, hence reducing its potential for interference. Coupling murine ghrelin residues 42-51 via the N-terminus to VLP will induce antibodies capable of neutralizing active ghrelin and, hence, prevent it's passing of the blood brain barrier, leading to reduced food intake.

Furthermore, the again furher very preferred ghrelin peptide fragments corresponding to the murine ghrelin peptide fragments of residues 31-41 (EHQKAQQRKES) (SEQ ID NO: 113) and residues 28-37 (LSPEHQKAQQ) (SEQ ID NO: 117) were chosen and encompass the central segment of ghrelin. Similarly, these ghrelin fragments would avoid potential T cell epitopes and would also avoid the n-octanoyl group on serine 3. Coupling of these peptides via the N- or C-terminus to VLP will induce antibodies capable of neutralizing active ghrelin and, hence, prevent it's passing of the blood brain barrier, leading to reduced food intake.

Further preferred ghrelin peptides suitable for use for the present invention can be identified by using existing or future monoclonal or polyclonal antibodies.

Further preferred ghrelin molecules and peptides derived from the molecules may be discovered in the future in species where no sequence information is available yet.

It will be understood by one of ordinary skill in the relevant arts that other suitable modifications and adaptations to the methods and applications described herein are readily apparent and may be made without departing from the scope of the invention or any embodiment thereof. Having now described the present invention in detail, the same will be more clearly understood by reference to the following examples.

### EXAMPLES

### EXAMPLE 1

### Construction and expression of mutant Qβ coat proteins, and purification of mutant Qβ coat protein VLPs or Capsids.

### Plasmid construction and cloning of mutant coat proteins

### Construction of pQβ-240:

The plasmid pQβ10 (Kozlovska, TM, et al., Gene 137:133-137) was used as an initial plasmid for the construction of pQβ-240. The mutation Lys13→Arg was created by inverse PCR. The inverse primers were designed in inverted tail-to-tail directions:
5'-GGTAACATCGGTCGAGATGGAAAACAAACTCTGGTCC-3' (SEQ ID NO: 78)
and
5'-GGACCAGAGTTTGTTTTCCATCTCGACCGATGTTACC-3' (SEQ ID NO: 79).

The products of the first PCR were used as templates for the second PCR reaction, in which an upstream primer
5'-AGCTCGCCCGGGGATCCTCTAG-3' (SEQ ID NO: 80)
and a downstream primer
5'-CGATGCATTTCATCCTTAGTTATCAATACGC-TGGGTTCAG3' (SEQ ID NO: 81)
were used. The product of the second PCR was digested with *XbaI* and *Mph1103I* and cloned into the pQβ10 expression vector, which was cleaved by the same restriction enzymes. The PCR reactions were performed with PCR kit reagents and according to producer protocol (MBI Fermentas, Vilnius, Lithuania).

Sequencing using the direct label incorporation method verified the desired mutations. *E.coli* cells harbouring pQβ-240 supported efficient synthesis of 14-kD protein co migrating upon SDS-PAGE with control Qβ coat protein isolated from Qβ phage particles.

Resulting amino acid sequence: (SEQ ID NO: 17)

### Construction ofpQβ-243:

The plasmid pQβ10 was used as an initial plasmid for the construction of pQβ-243.The mutation Asn10→Lys was created by inverse PCR. The inverse primers were designed in inverted tail-to-tail directions:
5'-GGCAAAATTAGAGACTGTTACTTTAGGTAAGATCGG -3' (SEQ ID NO: 82)
and
5'-CCGATCTTACCTAAAGTAACAGTCTCTAATTTTGCC -3' (SEQ ID NO: 83).

The products of the first PCR were used as templates for the second PCR reaction, in which an upstream primer
5'-AGCTCGCCCGGGGATCCTCTAG-3' (SEQ ID NO: 80)
and a downstream primer
5'-CGATGCATTTCATCCTTAGTTATCAATACGCTGGGTTCAG-3' (SEQ ID NO: 81)
were used. The product of the second PCR was digested with *XbaI* and *Mph1103I* and cloned into the pQβ10 expression vector, which was cleaved by the same restriction enzymes. The PCR reactions were performed with PCR kit reagents and according to producer protocol (MBI Fermentas, Vilnius, Lithuania).

Sequencing using the direct label incorporation method verified the desired mutations. *E.coli* cells harbouring pQβ-243 supported efficient synthesis of 14-kD protein co migrating upon SDSD-PAGE with control Qβ coat protein isolated from Qβ phage particles.

Resulting amino acid sequence: (SEQ ID NO: 18)

### Construction of pQβ-250:

The plasmid pQβ-240 was used as an initial plasmid for the construction of pQβ-250. The mutation Lys2→Arg was created by site-directed mutagenesis. An upstream primer
5'-GGCCATGGCACGACTCGAGACTGTTACTTTAGG-3' (SEQ ID NO: 84)
and a downstream primer
5'-GATTTAGGTGACACTATAG-3' (SEQ ID NO: 85)
were used for the synthesis of the mutant PCR-fragment, which was introduced into the pQβ-185 expression vector at the unique restriction sites ***NcoI*** and ***HindIII.*** The PCR reactions were performed with PCR kit reagents and according to producer protocol (MBI Fermentas, Vilnius, Lithuania).

Sequencing using the direct label incorporation method verified the desired mutations. *E.coli* cells harbouring pQβ-250 supported efficient synthesis of 14-kD protein co migrating upon PAGE with control Qβ coat protein isolated from Qβ phage particles.

Resulting amino acid sequence: (SEQ ID NO: 19)

### Construction of pQβ-251:

The plasmid pQβ10 was used as an initial plasmid for the construction of pQβ-251. The mutation Lys16→Arg was created by inverse PCR. The inverse primers were designed in inverted tail-to-tail directions:
5'-GATGGACGTCAAACTCTGGTCCTCAATCCGCGTGGGG -3' (SEQ ID NO: 86)
and
5'-CCCCACGCGGATTGAGGACCAGAGTTTGACGTCCATC -3' (SEQ ID NO: 87).

The products of the first PCR were used as templates for the second PCR reaction, in which an upstream primer
5'-AGCTCGCCCGGGGATCCTCTAG-3' (SEQ ID NO: 80)
and a downstream primer
5'-CGATGCATTTCATCCTTAGTTATCAATACGCTGGGTTCAG-3' (SEQ ID NO: 81)
were used. The product of the second PCR was digested with *XbaI* and *Mph1103I* and cloned into the pQβ10 expression vector, which was cleaved by the same restriction enzymes. The PCR reactions were performed with PCR kit reagents and according to producer protocol (MBI Fermentas, Vilnius, Lithuania).

Sequencing using the direct label incorporation method verified the desired mutations. *E.coli* cells harbouring pQβ-251 supported efficient synthesis of 14-kD protein co migrating upon SDS-PAGE with control Qβ coat protein isolated from Qβ phage particles. The resulting amino acid sequence encoded by this construct is shown in SEQ. ID NO: 20.

### Construction of pQβ-259:

The plasmid pQβ-251 was used as an initial plasmid for the construction of pQβ-259. The mutation Lys2→Arg was created by site-directed mutagenesis. An upstream primer
5'-GGCCATGGCAGGACTCGAGACTGTTACTTTAGG-3' (SEQ ID NO: 84)
and a downstream primer
5'-GATTTAGGTGACACTATAG-3' (SEQ ID NO: 85)
were used for the synthesis of the mutant PCR-fragment, which was introduced into the pQβ-185 expression vector at the unique restriction sites *NcoI* and *HindIII.* The PCR reactions were performed with PCR kit reagents and according to producer protocol (MBI Fermentas, Vilnius, Lithuania).

Sequencing using the direct label incorporation method verified the desired mutations. *E.coli* cells harbouring pQβ-259 supported efficient synthesis of 14-kD protein co migrating upon SDS-PAGE with control Qβ coat protein isolated from Qβ phage particles.

Resulting amino acid sequence: (SEQ ID NO: 21)

### General procedures for Expression and purification of Qβ and Qβ mutants

### Expression

E.coli JM109 was transformed with Qβ coat protein expression plasmids. 5 ml of LB liquid medium containing 20 µg/ml ampicillin were inoculated with clones transformed with with Qβ coat protein expression plasmids. The inoculated culture was incubated at 37 °C for 16-24 h without shaking. The prepared inoculum was subsequently diluted 1:100 in 100-300 ml of fresh LB medium, containing 20 µg/ml ampicillin and incubated at 37°C overnight without shaking. The resulting second inoculum was diluted 1:50 in M9 medium containing 1 % Casamino acids and 0.2 % glucose in flasks, and incubated at 37 °C overnight under shaking.

### Purification

Solutions and buffers for the purification procedure:
1. Lysis buffer LB
   50mM Tris-HCl pH8,0 with 5mM EDTA , 0,1%
   tritonX100 and freshly prepared PMSF at a concentration of 5micrograms per ml. Without lysozyme and DNAse.
2. SAS
   Saturated ammonium sulphate in water
3. Buffer NET.
   20 mM Tris-HCl, pH 7.8 with 5mM EDTA and
   150 mM NaCl.
4. PEG
   40% (w/v) polyethylenglycol 6000 in NET

### Disruption and lysis

Frozen cells were resuspended in LB at 2 ml/g cells. The mixture was sonicated with 22 kH five times for15 seconds, with intervals of 1min to cool the solution on ice. The lysate was then centrifuged at 14 000 rpm, for 1h using a Janecki K 60 rotor. The centrifugation steps described below were all performed using the same rotor, except otherwise stated. The supernatant was stored at 4° C, while cell debris was washed twice with LB. After centrifugation, the supernatants of the lysate and wash fractions were pooled.

### Fractionation

A saturated ammonium sulphate solution was added dropwise under stirring to the above pooled lysate. The volume of the SAS was adjusted to be one fifth of total volume, to obtain 20% of saturation. The solution was left standing overnight, and was centrifuged the next day at 14 000 rpm, for 20 min. The pellet was washed with a small amount of 20% ammonium sulphate, and centrifuged again. The obtained supernatants were pooled, and SAS was added dropwise to obtain 40% of saturation. The solution was left standing overnight, and was centrifuged the next day at 14 000 rpm, for 20 min. The obtained pellet was solubilised in NET buffer.

### Chromatography

The capsid or VLP protein resolubilized in NET buffer was loaded on a Sepharose CL- 4B column. Three peaks eluted during chromatography. The first one mainly contained membranes and membrane fragments, and was not collected. Capsids were contained in the second peak, while the third one contained other E.coli proteins.

The peak fractions were pooled, and the NaCl concentration was adjusted to a final concentration of 0.65 M. A volume of PEG solution corresponding to one half of the pooled peak fraction was added dropwise under stirring. The solution was left to stand overnight without stirring. The capsid protein was sedimented by centrifugation at 14 000 rpm for 20 min. It was then solubilized in a minimal volume of NET and loaded again on the Sepharose CL- 4B column. The peak fractions were pooled, and precipitated with ammonium sulphate at 60% of saturation (w/v). After centrifugation and resolubilization in NET buffer, capsid protein was loaded on a Sepharose CL-6B column for rechromatography.

### Dialysis and drying

The peak fractions obtained above were pooled and extensively dialysed against sterile water, and lyophilized for storage.

### Expression and purification Qβ-240

Cells (*E*. *coli* JM 109, transformed with the plasmid pQβ-240) were resuspended in LB, sonicated five times for 15 seconds (water ice jacket) and centrifuged at 13000 rpm for one hour. The supernatant was stored at 4°C until further processing, while the debris were washed 2 times with 9 ml of LB, and finally with 9 ml of 0,7 M urea in LB. All supernatants were pooled, and loaded on the Sepharose CL-4B column. The pooled peak fractions were precipitated with ammonium sulphate and centrifuged. The resolubilized protein was then purified further on a Sepharose 2B column and finally on a Sepharose 6B column. The capsid peak was finally extensively dialyzed against water and lyophilized as described above. The assembly of the coat protein into a capsid was confirmed by electron microscopy.

### Expression and purification Qβ-243

Cells (*E*. *coli* RR1) were resuspended in LB and processed as described in the general procedure. The protein was purified by two successive gel filtration steps on the sepharose CL-4B column and finally on a sepharose CL-2B column. Peak fractions were pooled and lyophilized as described above. The assembly of the coat protein into a capsid was confirmed by electron microscopy.

### Expression and purification of Qβ-250

Cells (*E*. *coli* JM 109, transformed with pQβ-250) were resuspended in LB and processed as described above. The protein was purified by gel filtration on a Sepharose CL-4B and finally on a Sepharose CL-2B column, and lyophilized as described above. The assembly of the coat protein into a capsid was confirmed by electron microscopy.

### Expression and purification of Qβ-259

Cells (*E*. *coli* JM 109, transformed with pQβ-259) were resuspended in LB and sonicated. The debris were washed once with 10 ml of LB and a second time with 10 ml of 0,7 M urea in LB. The protein was purified by two gel-filtration chromatogaphy steps, on a Sepharose CL-4 B column. The protein was dialyzed and lyophilized, as described above. The assembly of the coat protein into a capsid was confirmed by electron microscopy.

### EXAMPLE 2

### Insertion of a peptide containing a Lysine residue into the c/e1 epitope of HBcAg (1-149).

The c/e1 epitope (residues 72 to 88) of HBcAg is located in the tip region on the surface of the Hepatitis B virus capsid (HBcAg). A part of this region (Proline 79 and Alanine 80) was genetically replaced by the peptide Gly-Gly-Lys-Gly-Gly (SEQ ID NO: 33), resulting in the HBcAg-Lys construct (SEQ ID NO: 26). The introduced Lysine residue contains a reactive amino group in its side chain that can be used for intermolecular chemical crosslinking of HBcAg particles with any antigen containing a free cysteine group.

HBcAg-Lys DNA, having the amino acid sequence shown in SEQ ID NO:78, was generated by PCR: The two fragments encoding HBcAg fragments (amino acid residues 1 to 78 and 81 to 149) were amplified separately by PCR. The primers used for these PCRs also introduced a DNA sequence encoding the Gly-Gly-Lys-Gly-Gly peptide (SEQ ID NO: 33). The HBcAg (1 to 78) fragment was amplified from pEco63 using primers EcoRIHBcAg(s) and Lys-HBcAg(as). The HBcAg (81 to 149) fragment was amplified from pEco63 using primers Lys-HBcAg(s) and HBcAg(1-149)Hind(as). Primers Lys-HBcAg(as) and Lys-HBcAg(s) introduced complementary DNA sequences at the ends of the two PCR products allowing fusion of the two PCR products in a subsequent assembly PCR. The assembled fragments were amplified by PCR using primers EcoRIHBcAg(s) and HbcAg(1-149) Hind(as).

For the PCRs, 100 pmol of each oligo and 50 ng of the template DNAs were used in the 50 ml reaction mixtures with 2 units of Pwo polymerase, 0.1 mM dNTPs and 2 mM MgS04. For both reactions, temperature cycling was carried out as follows: 94°C for 2 minutes; 30 cycles of 94°C (1 minute), 50°C (1 minute), 72°C (2 minutes).

### Primer sequences:

EcoRIHBcAg(s):
(5'-CCGGAATTCATGGACATTGACCCTTATAAAG-3') (SEQ ID NO: 88);

HBcAg(1-149)Hind(as):
(5'-CGCGTCCCAAGCTTCTAAACAACAGTAGTCTCCGGAAG-3') (SEQ ID NO: 91).

For fusion of the two PCR fragments by PCR 100 pmol of primers EcoRIHBcAg(s) and HBcAg(1-149)Hind(as) were used with 100 ng of the two purified PCR fragments in a 50 ml reaction mixture containing 2 units of Pwo polymerase, 0.1 mM dNTPs and 2 mM MgSO₄. PCR cycling conditions were: 94°C for 2 minutes; 30 cycles of 94°C (1 minute), 50°C (1 minute), 72°C (2 minutes). The assembled PCR product was analyzed by agarose gel electrophoresis, purified and digested for 19 hours in an appropriate buffer with EcoRI and HindIII restriction enzymes. The digested DNA fragment was ligated into EcoRI/HindIII-digested pKK vector to generate pKK-HBcAg-Lys expression vector. Insertion of the PCR product into the vector was analyzed by EcoRI/HindIII restriction analysis and DNA sequencing of the insert.

### EXAMPLE 3

### Expression and purification of HBcAg-Lys.

*E. coli* strains K802 or JM109 were transformed with pKK-HBcAg-Lys. 1 ml of an overnight culture of bacteria was used to innoculate 100 ml of LB medium containing 100 µg/ml ampicillin. This culture was grown for 4 hours at 37°C until an OD at 600 nm of approximately 0.8 was reached. Induction of the synthesis of HBcAg-Lys was performed by addition of IPTG to a final concentration of 1 mM. After induction, bacteria were further shaken at 37°C for 4 hours. Bacteria were harvested by centrifugation at 5000 x g for 15 minutes. The pellet was frozen at - 80°C. The pellet was thawed and resuspended in bacteria lysis buffer (10 mM Na₂HPO₄, pH 7.0, 30 mM NaCl, 0.25% Tween-20, 10 mM EDTA) supplemented with 200 µg/ml lysozyme and 10 µl of Benzonase (Merck). Cells were incubated for 30 minutes at room temperature and disrupted by sonication. *E. coli* cells harboring pKK-HBcAg-Lys expression plasmid or a control plasmid were used for induction of HBcAg-Lys expression with IPTG. Prior to the addition of IPTG, a sample was removed from the bacteria culture carrying the pKK-HBcAg-Lys plasmid and from a culture carrying the control plasmid. Four hours after addition of IPTG, samples were again removed from the culture containing pKK-HBcAg-Lys and from the control culture. Protein expression was monitored by SDS-PAGE followed by Coomassie staining.

The lysate was then centrifuged for 30 minutes at 12,000 x g in order to remove insoluble cell debris. The supernatant and the pellet were analyzed by Western blotting using a monoclonal antibody against HBcAg (YVS1841, purchased from Accurate Chemical and Scientific Corp., Westbury, NY, USA), indicating that a significant amount of HBcAg-Lys protein was soluble. Briefly, lysates from *E. coli* cells expressing HBcAg-Lys and from control cells were centrifuged at 14,000 x g for 30 minutes. Supernatant (= soluble fraction) and pellet (= insoluble fraction) were separated and diluted with SDS sample buffer to equal volumes. Samples were analyzed by SDS-PAGE followed by Western blotting with anti-HBcAg monoclonal antibody YVS 1841.

The cleared cell lysate was used for step-gradient centrifugation using a sucrose step gradient consisting of a 4 ml 65% sucrose solution overlaid with 3 ml 15% sucrose solution followed by 4 ml of bacterial lysate. The sample was centrifuged for 3 hrs with 100,000 x g at 4°C. After centrifugation, 1 ml fractions from the top of the gradient were collected and analyzed by SDS-PAGE followed by Coomassie staining. The HBcAg-Lys protein was detected by Coomassie staining.

The HBcAg-Lys protein was enriched at the interface between 15 and 65% sucrose indicating that it had formed a capsid particle. Most of the bacterial proteins remained in the sucrose-free upper layer of the gradient, therefore step-gradient centrifugation of the HBcAg-Lys particles led both to enrichment and to a partial purification of the particles.

Expression and purification of HBcAg-Lys in large scale was performed as follows. An overnight culture was prepared by inoculating a single colony in 100 ml LB, 100 µg/ml Ampicillin and growing the culture overnight at 37°C. 25 ml of the preculture were diluted in 800 ml LB Ampicillin medium the next day, and the culture grown to an optical density OD⁶⁰⁰ of 0.6-0.8. The culture was then induced with 1 mM IPTG, and left to grow for another 4 hours. The cells were harvested and lysed essentially as described above.

HBcAg-Lys was then purified by first precipitating the protein with ammonium sulphate (30% saturation) from the cleared cell lysate, then loading the resolubilized pellet on a gel filtration column (Sephacryl S-400, Pharmacia). The pooled fractions were precipitated again with ammonium sulphate, the pellet resolubilized and loaded a second time on the same gel filtration column. The fractions were finally pooled and concentrated, and the concentration assessed using a Bradford test (BioRad).

### EXAMPLE 4

### Construction of an HBcAg devoid of free cysteine residues and containing an inserted lysine residue.

A Hepatitis core Antigen (HBcAg), referred to herein as HBcAg-lys-2cys-Mut, devoid of cysteine residues at positions corresponding to 48 and 107 in SEQ ID NO:25 and containing an inserted lysine residue was constructed using the following methods.

The two mutations were introduced by first separately amplifying three fragments of the HBcAg-Lys gene prepared as described above in Example 2 with the following PCR primer combinations. PCR methods and conventional cloning techniques were used to prepare the HBcAg-lys-2cys-Mut gene.

In brief, the following primers were used to prepare fragment 1:
Primer 1: EcoRIHBcAg(s)
   CCGGAATTCATGGACATTGACCCTTATAAAG (SEQ ID NO: 88)
Primer 2: 48as
   GTGCAGTATGGTGAGGTGAGGAATGCTCAGGAGACTC (SEQ ID NO: 92)

The following primers were used to prepare fragment 2:
Primer 3: 48s
   GAGTCTCCTGAGCATTCCTCACCTCACCATACTGCAC (SEQ ID NO: 93)
Primer 4: 107as
   CTTCCAAAAGTGAGGGAAGAAATGTGAAACCAC (SEQ ID NO: 94)

The following primers were used to prepare fragment 3:
Primer 5: HBcAg149hind-as
Primer 6: 107s
   GTGGTTTCACATTTCTTCCCTCACTTTTGGAAG (SEQ ID NO: 96)

Fragments 1 and 2 were then combined with PCR primers EcoRIHBcAg(s) and 107as to give fragment 4. Fragment 4 and fragment 3 were then combined with primers EcoRIHBcAg(s) and HBcAg149hind-as to produce the full length gene. The full length gene was then digested with the EcoRI (GAATTC) and HindIII (AAGCTT) enzymes and cloned into the pKK vector (Pharmacia) cut at the same restriction sites. Expression and purification of HBcAg-lys-2cys-Mut were performed as set out in Example 3.

### EXAMPLE 5

### Construction of HBcAg1-185-Lys.

Hepatitis core Antigen (HBcAg) 1-185 was modified as described in Example 2. A part of the c/e1 epitope (residues 72 to 88) region (Proline 79 and Alanine 80) was genetically replaced by the peptide Gly-Gly-Lys-Gly-Gly (SEQ ID NO: 33), resulting in the HBcAg-Lys construct (SEQ ID NO: 26). The introduced Lysine residue contains a reactive amino group in its side chain that can be used for intermolecular chemical crosslinking of HBcAg particles with any antigen containing a free cysteine group. PCR methods and conventional cloning techniques were used to prepare the HBcAg1-185-Lys gene.

The Gly-Gly-Lys-Gly-Gly sequence (SEQ ID NO: 33) was inserted by amplifying two separate fragments of the HBcAg gene from pEco63, as described above in Example 2 and subsequently fusing the two fragments by PCR to assemble the full length gene. The following PCR primer combinations were used:
fragment 1:
   Primer 1: EcoRIHBcAg(s) ((SEQ ID NO: 88), see Example 2)
   Primer 2: Lys-HBcAg(as) ((SEQ ID NO: 89), see Example 2)
fragment 2:
   Primer 3: Lys-HBcAg(s) ((SEQ ID NO: 90), see Example 2)
   Primer 4: HBcAgwtHindIIII
      CGCGTCCCAAGCTTCTAACATTGAGATTCCCGAGATTG (SEQ ID NO: 97)
Assembly:
   Primer 1: EcoRIHBcAg(s) ((SEQ ID NO: 88), see example 2)
   Primer 2: HBcAgwtHindIIII (SEQ ID NO: 97)

The assembled full length gene was then digested with the EcoRI (GAATTC) and HindIII (AAGCTT) enzymes and cloned into the pKK vector (Pharmacia) cut at the same restriction sites.

### EXAMPLE 6

### Fusion of a peptide epitope in the MIR region of HbcAg.

The residues 79 and 80 of HBcAg1-185 were substituted with the epitope CεH3 of sequence VNLTWSRASG (SEQ ID NO: 98). The CεH3 sequence stems from the sequence of the third constant domain of the heavy chain of human IgE. The epitope was inserted in the HBcAg1-185 sequence using an assembly PCR method. In the first PCR step, the HBcAg1-185 gene originating from ATCC clone pEco63 and amplified with primers HBcAg-wt EcoRI fwd and HBcAg-wt Hind III rev was used as template in two separate reactions to amplify two fragments containing sequence elements coding for the CεH3 sequence. These two fragments were then assembled in a second PCR step, in an assembly PCR reaction.

Primer combinations in the first PCR step: CεH3fwd with HBcAg-wt Hind III rev, and HBcAg-wt EcoRI fwd with CεH3rev. In the assembly PCR reaction, the two fragments isolated in the first PCR step were first assembled during 3 PCR cycles without outer primers, which were added afterwards to the reaction mixture for the next 25 cycles. Outer primers: HBcAg-wt EcoRI fwd and HBcAg-wt Hind III rev.

The PCR product was cloned in the pKK223.3 using the EcoRI and HindIII sites, for expression in E. coli (see Example 2). The chimeric VLP was expressed in *E. coli* and purified as described in Example 2. The elution volume at which the HBcAg1-185- CεH3 eluted from the gel filtration showed assembly of the fusion proteins to a chimeric VLP.

Primer sequences: HBcAg-wt EcoRI fwd:
5' *CCG*gaattcATGGACATTGACCCTTATAAAG (SEQ ID NO: 103)
HBcAg-wt Hind III rev:
5' *CGCGTCC*CaagcttCTAACATTGAGATTCCCGAGATTG (SEQ ID NO: 104)

### EXAMPLE 7

### Fusion of a ghrelin peptide epitope in the MIR region of HbcAg.

The residues 79 and 80 of HBcAg1-185 are substituted with the ghrelin peptide epitope of sequence: EHQRVQQRKE (SEQ ID NO: 56). Two overlapping primers are designed using the same strategy described in Example 6, and the fusion protein constructed by assembly PCR. The PCR product is cloned in the pKK223.3 vector, and expressed in E. coli K802. The chimeric VLPs are expressed and purified as described in Example 3.

### EXAMPLE 8

### Fusion of a ghrelin peptide epitope to the C-terminus of the Qβ A1 protein truncated at position 19 of the CP extension.

A primer annealing to the 5' end of the Qβ A1 gene and a primer annealing to the 3' end of the A 1 gene and comprising additionally a sequence element coding a ghrelin fragment, of sequence KKPPAKLQPR (SEQ ID NO: 52), are used in a PCR reaction with pQβ10 as template. The PCR product is cloned in pQβ10 (Kozlovska T.M. et al., Gene 137: 133-37 (1993)), and the chimeric VLP expressed and purified as described in Example 1.

### EXAMPLE 9

### Insertion of a ghrelin peptide epitope between positions 2 and 3 of fr coat protein.

Complementary primers coding for the sequence of the ghrelin peptide of sequence EHQRVQQRKE (SEQ ID NO: 56), and containing *Bsp119*I compatible ends and additional nucleotides enabling in frame insertion, are inserted in the Bop119I site of the pFrd8 vector (Pushko, P. et al., Prot. Eng. 6: 883-91 (1993)) by standard molecular biology techniques. Alternatively, the overhangs of the pFrd8 vector are filled in with Klenow after digestion with *Bsp119*I, and oligonucleotides coding for the sequence of the ghrelin peptide murine ghrelin peptide and additional nucleotides for in frame cloning are ligated in pFrd8 after the Klenow treatment. Clones with the insert in the right orientation are analyzed by sequencing. Expression and purification of the chimeric fusion protein in E. coli JM109 or E. coli K802 is performed as described in Pushko, P. et al, Prot. Eng. 6:883-91 (1993), but for the chromatography steps which are performed using a Sepharose CL-4B or Sephacryl S-400 (Pharmacia). The cell lysate is precipitated with ammonium sulphate, and purified by two successive gel filtration purification steps, similarly to the procedure described for Qβ in Example 1.

### EXAMPLE 10

### Insertion of a ghrelin peptide epitope between positions 67 and 68 ofTyl protein p1 in the vector pOGS8111.

Two complementary oligonucleotides coding for the human ghrelin peptide sequence EHQRVQQRKE (SEQ ID NO: 56), with ends compatible with the NheI site of pOGS8111 are synthesized. Additional nucleotides are added to allow for in frame insertion of a sequence coding for the murine ghrelin epitope according to the description of EP06777111. The amino acids AS and SS flanking the inserted epitope are encoded by the altered NheI sites resulting from the insertion of the oligonucleotide in the TyA(d) gene of pOGS8111.

POGS8111 is transformed into *S. cervisiae* strain MC2, for expression of the chimeric Ty VLP as described in EP0677111 and references therein. The chimeric Ty VLP is purified by sucrose gradient ultracentrifugation as described in EP0677111.

### EXAMPLE 11

### Insertion of a ghrelin peptide epitope in to the major capsid protein L1 of papillomavirus type 1 (BPV-1).

A sequence coding for the ghrelin epitope having the sequence EHQRVQQRKE (SEQ ID NO: 56) is substituted to the sequence coding for amino acids 130-136 of the BPV-1 L1 gene cloned in the pFastBac1 (GIBCOBRL) vector as described (Chackerian, B. et al., Proc. Natl. Acad. USA 96: 2373-2378 (1999)). The sequence of the construct is verified by nucleotide sequence analysis. Recombinant baculovirus is generated using the GIBCOBRL baculovirus system as described by the manufacturer. The chimeric VLPs are purified from baculovirus infected Sf9 cells as described by Kimbauer, R. et al., Proc. Natl. Acad. Sci. 89:12180-84 (1992) and Greenstone, H.L., et al., Proc. Natl. Acad. Sci. 95:1800-05 (1998).

### EXAMPLE 12

### Immunization of mice with ghrelin-peptides fused to VLPs.

Chimeric VLPs displaying the murine ghrelin epitope of sequence EHQRVQQRKE (SEQ ID NO: 56) generated in Examples 7, 9, 10 and 11 are used for immunization of mice as described in Example 15. The sera obtained from the immunized mice are analyzed in a ghrelin -specific ELISA as described in Example 14.

The effect of the vaccine is examined by following the weight increase of the mice and by measuring food uptake.

### EXAMPLE 13

### Coupling of murine ghrelin and murine ghrelin peptides to Qβ capsid protein: ghrelin peptide vaccines.

The following ghrelin peptides are chemically synthesized:
cGhrel: CGSSFLSPEHQKAQQRKESKKPPAKLQPR (SEQ ID NO: 77)
GhrelC: GSSFLSPEHQKAQQRKESKKPPAKLQPRGC (SEQ ID NO: 105)
cGhrQ14: CGSSFLSPEHQKAQRKESKKPPAKLQPR (SEQ ID NO: 106)
GhrQ14C: GSSFLSPEHQKAQRKESKKPPAKLQPRGC (SEQ ID NO: 107)
Ghre124-33C GGSSFLSPEHQGC (SEQ ID NO: 132)
cGhre142-51 CKKPPAKLQPR (SEQ ID NO: 133)
cGhre131-40 CEHQKAQQRKE (SEQ ID NO: 134)
cGhre131-41 CEHQKAQQRKES (SEQ ID NO: 135)
cGhre128-37 CLSPEHQKAQQ (SEQ ID NO: 136)
which comprise an added N- or C- terminal cysteine residue for coupling to VLPs and Pili, and used for chemical coupling to Qβ as described in the following.

A solution of 5 ml of 140 µM Qβ capsid protein in 20 mM Hepes, 150 mM NaCl pH 7.4 is reacted for 30 minutes with 108 µl of a 65 mM solution of SMPH (Pierce) in H₂O at 25°C on a rocking shaker. The reaction solution is subsequently dialyzed twice for 2 hours against 5 L of 20 mM Hepes, 150 mM NaCl, pH 7.2 at 4°C. 100 µl of the dialyzed reaction mixture is then reacted either with 28.6 µl of a 10 mM stock solution (in DMSO) of the ghrelin peptide (1:10 peptide/ Qβ capsid protein ratio). The coupling reaction is performed for 2h at 15 °C in a water bath. The reaction mixture is subsequently dialyzed for 24 h against 2x 5 L of 20 mM Hepes; 150 mM NaCl, pH 7.2 at 4°C.

The coupled products are centrifuged and supernatants and pellets are analysed on 16% SDS-PAGE gels under reducing conditions. Gels are stained with Coomassie Brilliant Blue.

### Coupling of ghrelin peptides to fr capsid protein

A solution of 120 µM fr capsid protein in 20 mM Hepes, 150 mM NaCl pH 7.2 is reacted for 30 minutes with a 10 fold molar excess of SMPH (Pierce) ), diluted from a stock solution in DMSO, at 25 °C on a rocking shaker. The reaction solution is subsequently dialyzed twice for 2 hours against 1 L of 20 mM Hepes, 150 mM NaCl, pH 7.2 at 4 °C. The dialyzed fr reaction mixture is then reacted with equimolar concentration of ghrelin peptide or a ration of 1:2 ghrelin peptide / fr over night at 16 °C on a rocking shaker. Coupling products are analysed by SDS-PAGE.

### Coupling of ghrelin peptides to HBcAg-Lys-2cys-Mut

A solution of 120 µM HBcAg-Lys-2cys-Mut in 20 mM Hepes, 150 mM NaCl pH 7.2 is reacted for 30 minutes with a 10 fold molar excess of SMPH (Pierce), diluted from a stock solution in DMSO, at 25 °C on a rocking shaker. The reaction solution is subsequently dialyzed twice for 2 hours against 1 L of 20 mM Hepes, 150 mM NaCl, pH 7.2 at 4 °C. The dialyzed HBcAg-Lys-2cys-Mut reaction mixture is then reacted with equimolar concentration of ghrelin peptide or a ration of 1:2 ghrelin peptide / HBcAg-Lys-2cys-Mut over night at 16 °C on a rocking shaker. Coupling products are analysed by SDS-PAGE.

### Coupling of ghrelin peptides to Pili

A solution of 125 µM Type-1 pili of *E.coli* in 20 mM Hepes, pH 7.2, is reacted for 60 minutes with a 50-fold molar excess of cross-linker SMPH (Pierce), diluted from a stock solution in DMSO, at RT on a rocking shaker. The reaction mixture is desalted on a PD-10 column (Amersham-Pharmacia Biotech). The protein-containing fractions eluating from the column are pooled, and the desalted derivatized pili protein is reacted with the ghrelin peptides in equimolar or in a ratio of 1:2 peptide pili over night at 16 °C on a rocking shaker. Coupling products are analysed by SDS-PAGE.

### EXAMPLE 14

### Immunization of mice with VLP-ghrelin peptide conjugates.

### Immunization of mice with Ghrelin-peptides coupled to Qβ

Female C57BL/6 mice are immunized with the murine ghrelin peptides listed in example 13 coupled to Qβ mixed with alum, emulsified in IFA or with no adjuvant 50 µg are injected subcutaneously on day 0 and on day 14 and day 28. Mice are bled retroorbitally on day 14, 21, 28, 42 and subsequently at monthly intervals thereafter, after immunization. Serum is analyzed using a ghrelin protein-specific ELISA.

Having now fully described the present invention in some detail by way of illustration and example for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof and that such modifications or changes are intended to be encompassed within the scope of the appended claims.

All publications, patents and patent applications mentioned in this specification are indicative of the level of skill of those skilled in the art to which this invention pertains, and are herein incorporated by reference to the same extent as if each individual publication, patent or patent application was specifically and individually indicated to be incorporated by reference.

### EXAMPLE 15

### Coupling of C-Ghrelin and Ghrelin-GC to Qβ capsid protein

A solution of 2 ml of 2.0 mg/ml Qβ capsid protein in 20 mM Hepes, 150 mM NaCl pH 7.2 was reacted for 30 minutes with 114.4 µl of a SMPH (Pierce) solution (from a 50 mM stock solution dissolved in DMSO) at 25°C. The reaction solution was subsequently dialyzed twice for 2 hours against 2 L of 20 mM Hepes, 150 mM NaCl, pH 7.2 at 4°C.

The dialysed, derivatized Qβ VLP was subsequently reacted with the murine C-Ghrelin (SEQ ID: No. 77) or murine Ghrelin-GC peptide (SEQ ID: No. 105) for 2 hours at 15°C in 20 mM Hepes, 150 mM NaCl, pH 7.2. The coupling reaction was performed in multiple aliquots, where 1 ml of derivatized Qβ VLP (at a concentration of 2mg/ml) was reacted with 286µl of a 10mM peptide solution. 10% acetonitrile was added to the coupling reaction. The reaction solution was dialyzed once for 2 hours and then overnight against 2 L of 20 mM Hepes, 150 mM NaCl, pH 7.2 at 4°C.

Coupled products were analyzed on 16% SDS-PAGE gels. Gels were stained with Coomassie Brilliant Blue. Results are shown in Fig. 1. Coupled products could be detected in the Coomassie-stained gels and clearly demonstrate the covalent coupling of C-Ghrelin or Ghrelin-GC to Qβ capsid protein. Coupling bands corresponding to one, two, three or four peptides coupled per subunit are indicated by arrows.

Figure 1 shows the coupling products from the reaction of murine C-Ghrelin or murine Ghrelin-GC to Qβ capsid protein. Lane 2 and 4 show the coupling products in the soluble fraction of Qβ-C-Ghrelin and Ghrelin-GC-Qβ, respectively. Very little product is in the insoluble fraction.

### EXAMPLE 16

### Immunization of mice with C-Ghrelin or Ghrelin-GC coupled to Qβ capsid protein

Female, C57BL/6 mice (5 per group) were vaccinated with either murine C-Ghrelin (SEQ ID: No. 77) or murine Ghrelin-GC (SEQ ID: No. 105) coupled to Qβ capsid protein. 50 µg of dialyzed vaccine from each sample was diluted in PBS to a volume of 200 µl and injected subcutaneously (100 µl on two ventral sides) on days 0, 14 and 42. The vaccine was administered without any additional adjuvant. As a control, a group of mice were immunized with PBS. Mice were bled retro-orbitally on day 0, 14, 21, 42 and their serum was analyzed using a Ghrelin-specific ELISA (outlined in example 17). Furthermore, body weight and food consumption of mice was monitored at regular intervals over the course of the experiment (outlined in example 18).

### EXAMPLE 17

### Detection of ghrelin-specific antibodies in an ELISA

ELISA plates were coated with serine-octanylated, murine ghrelin (Bachem, Product No. H-4862) at a concentration of 20µg/ml. The plates were blocked and then incubated with serially diluted mouse sera from day 14, 21 and 42. Bound antibodies were detected with enzymatically labeled anti-mouse IgG antibody. As a control, pre-immune serum of the same mice was also tested (Figure 2).

In mice immunized with Qβ-C-Ghrelin or Ghrelin-CG-Qβ, an average titer on day 21 of 13000 and 8000 respectively, was reached (Figure 2). Pre-immune sera or sera from mice immunized with PBS did not show any reactivity with ghrelin. This clearly demonstrates that a ghrelin-VLP conjugate is able to induce a high antibody titer against ghrelin even when it is a self protein.

### EXAMPLE 18

### Efficacy experiments with C-Ghrelin or Ghrelin-GC coupled to Qβ capsid protein

Female, C57BL/6 mice (5 per group) were vaccinated with either murine C-Ghrelin or Ghrelin-GC coupled to Qβ capsid protein as described in example 16. As a control, mice were immunized with PBS. All mice were placed on a normal diet between days 0 and 14. Subsequently, all mice were given a high fat (45%) diet to facilitate the development of diet-induced obesity. Food and water was administered *ad libitum.* Individual mice were monitored for body weight changes and food and water consumption per cage (i.e. group) was also monitored at regular intervals after immunization (at day 5, 11, 14, 21, 28, 35, 40, 49, and 55 after immunization) (see Fig. 3).

In mice immunized with murine Qβ-C-Ghrelin or murine Ghrelin-CG-Qβ, an average decrease of approximately 30-40% in food intake was achieved, compared to control mice immunized with PBS (Fig. 3). This clearly demonstrates that a ghrelin-VLP conjugate is able to suppress appetite and reduce food consumption.

### EXAMPLE 19

### Efficacy experiments with C-Ghrelin or Ghrelin-GC coupled to Qβ capsid protein in an animal model of obesity

Female, C57BL/6 *ob*/*ob* mice (5 per group) are vaccinated with either murine C-Ghrelin or Ghrelin-GC coupled to Qβ capsid protein. Control mice are immunized with PBS or Qβ VLP alone. 50 µg of dialyzed vaccine or control protein is diluted in a 200 µl volume of PBS and injected subcutaneously (100 µl on two ventral sides), with or without alum, on day 0. Mice are boosted with the corresponding formulation on day 14, 28 and 42.

Mice are bled retro-orbitally on day 0, 14, 21, 42, 56, 70 and then at monthly intervals. The sera are analyzed for ghrelin-specific antibodies in a Ghrelin-specific ELISA, as described in example 17. Mice will be boosted accordingly if ghrelin-specific antibody titers significantly decline over the period of the experiment. The effect of the vaccine is examined by measuring food consumption under *ad libitum* conditions and by following the weight increase of the mice as described in example 18.

### EXAMPLE 20:

### Cloning of the AP205 Coat Protein gene

The cDNA of AP205 coat protein (CP) (SEQ ID NO: 28) was assembled from two cDNA fragments generated from phage AP205 RNA by using a reverse transcription-PCR technique and cloning in the commercial plasmid pCR 4-TOPO for sequencing. Reverse transcription techniques are well known to those of ordinary skill in the relevant art. The first fragment, contained in plasmid p205-246, contained 269 nucleotides upstream of the CP sequence and 74 nucleotides coding for the first 24 N-terminal amino acids of the CP. The second fragment, contained in plasmid p205-262, contained 364 nucleotides coding for amino acidsl2-131of CP and an additional 162 nucleotides downstream of the CP sequence. Both p205-246 and p205-262 were a generous gift from J. Klovins.

The plasmid 283.-58 was designed by two-step PCR, in order to fuse both CP fragments from plasmids p205-246 and p205-262 in one full-length CP sequence.

An upstream primer p1.44 containing the *NcoI* site for cloning into plasmid pQb185, or p1.45 containing the *XbaI* site for cloning into plasmid pQb10, and a downstream primer p1.46 containing the *HindIII* restriction site were used (recognition sequence of the restriction enzyme underlined):
p1.44 5'-AACC ATG GCA AAT AAG CCA ATG CAA CCG-3' (SEQ ID NO: 139)
p1.45 5'-AATCTAGAATTTTCTGCGCACCCATCCCGG-3' (SEQ ID NO: 140)
p1.46 5'-AAAAGC TTA AGC AGT AGT ATC AGA CGA TAC G-3' (SEQ ID NO: 141)

Two additional primers, p1.47, annealing at the 5' end of the fragment contained in p205-262, and p1.48, annealing at the 3' end of the fragment contained in plasmid p205-246 were used to amplify the fragments in the first PCR. Primers p1.47 and p 1.48 are complementary to each other.

In the first two PCR reactions, two fragments were generated. The first fragment was generated with primers p1.45 and p1.48 and template p205-246. The second fragment was generated with primers p1.47 and p1.46, and template p205-262. Both fragments were used as templates for the second PCR reaction, a splice-overlap extension, with the primer combination p1.45 and p1.46 or p1.44 and p1.46 . The product of the two second-step PCR reactions were digested with *XbaI* or *NcoI* respectively, and *HindIII,* and cloned with the same restriction sites into pQb10 or pQb185 respectively, two pGEM-derived expression vectors under the control of *E.coli* tryptophan operon promoter.

Two plasmids were obtained, pAP283-58 (SEQ ID NO: 27), containing the gene coding for wt AP205 CP (SEQ ID NO: 28) in pQb10, and pAP281-32 (SEQ ID NO: 30) with mutation Pro5→Thr (SEQ ID NO: 29), in pQb185 . The coat protein sequences were verified by DNA sequencing. PAP283-58 contains 49 nucleotides upstream of the ATG codon of the CP, downstream of the XbaI site, and contains the putative original ribosomal binding site of the coat protein mRNA.

### EXAMPLE 21:

### Expression and Purification of Recombinant AP205 VLP

### A. Expression of recombinant AP205 VLP

*E.coli* JM109 was transformed with plasmid pAP283-58. 5 ml of LB liquid medium with 20 µg/ml ampicillin were inoculated with a single colony, and incubated at 37°C for 16-24 h without shaking.

The prepared inoculum was diluted 1:100 in 100-300 ml of LB medium, containing 20 µg/ml ampicillin and incubated at 37 °C overnight without shaking. The resulting second inoculum was diluted 1:50 in 2TY medium, containing 0.2 % glucose and phosphate for buffering, and incubated at 37 °C overnight on a shaker. Cells were harvested by centrifugation and frozen at -80°C.

### B. Purification of recombinant AP205 VLP

Solutions and buffers:
Lysis buffer
   50mM Tris-HCl pH 8.0 with 5mM EDTA , 0.1%
   tritonX100 and PMSF at 5 micrograms per ml.
SAS
   Saturated ammonium sulphate in water
Buffer NET.
   20 mM Tris-HCl, pH 7.8 with 5mM EDTA and
   150 mM NaCl.
PEG
   40% (w/v) polyethylenglycol 6000 in NET

### Lysis:

Frozen cells were resuspended in lysis buffer at 2 ml/g cells. The mixture was sonicated with 22 kH five times for15 seconds, with intervals of 1min to cool the solution on ice. The lysate was then centrifuged for 20 minutes at 12 000 rpm, using a F34-6-38 rotor (Ependorf). The centrifugation steps described below were all performed using the same rotor, except otherwise stated. The supernatant was stored at 4° C, while cell debris were washed twice with lysis buffer. After centrifugation, the supernatants of the lysate and wash fractions were pooled.

Ammonium-sulphate precipitation can be further used to purify AP205 VLP. In a first step, a concentration of ammonium-sulphate at which AP205 VLP does not precipitate is chosen. The resulting pellet is discarded. In the next step, an ammonium sulphate concentration at which AP205 VLP quantitatively precipitates is selected, and AP205 VLP is isolated from the pellet of this precipitation step by centrifugation (14 000 rpm, for 20 min). The obtained pellet is solubilised in NET buffer.

### Chromatography:

The capsid protein from the pooled supernatants was loaded on a Sepharose 4B column (2.8 X 70 cm), and eluted with NET buffer, at 4 ml/hour/fraction. Fractions 28-40 were collected; and precipitated with ammonium sulphate at 60% saturation. The fractions were analyzed by SDS-PAGE and Western Blot with an antiserum specific for AP205 prior to precipitation. The pellet isolated by centrifugation was resolubilized in NET buffer, and loaded on a Sepharose 2B column (2.3 X 65 cm), eluted at 3 ml/h/fraction. Fractions were analysed by SDS-PAGE, and fractions 44-50 were collected, pooled and precipitated with ammonium sulphate at 60% saturation. The pellet isolated by centrifugation was resolubilized in NET buffer, and purified on a Sepharose 6B column (2.5 X 47 cm), eluted at 3 ml/hour/fraction. The fractions were analysed by SDS-PAGE. Fractions 23-27 were collected, the salt concentration adjusted to 0.5 M, and precipitated with PEG 6000, added from a 40% stock in water and to a final concentration of 13.3%. The pellet isolated by centrifugation was resolubilized in NET buffer, and loaded on the same Sepharose 2B column as above, eluted in the same manner. Fractions 43-53 were collected, and precipitated with ammonium sulphate at a saturation of 60%. The pellet isolated by centrifugation was resolubilized in water, and the obtained protein solution was extensively dialyzed against water. About 10 mg of purified protein per gram of cells could be isolated.

Examination of the virus-like particles in Electron microscopy showed that they were identical to the phage particles.

## Claims

1. A composition comprising:
(a) a core particle with at least one first attachment site wherein said core particle is a virus-like particle of an RNA-phage; and
(b) at least one antigen or antigenic determinant with at least one second attachment site,
wherein said antigen or antigenic determinant is ghrelin or a ghrelin peptide, wherein the ghrelin peptide contains at least five consecutive amino acids of ghrelin, and wherein said second attachment site being selected from :
(i) an attachment site not naturally occurring with said antigen or antigenic determinant; and
(ii) an attachment site naturally occurring with said antigen or antigenic determinant,
wherein said second attachment site is capable of association to said first attachment site; and wherein said ghrelin or a ghrelin peptide and said core particle interact through said association to form an ordered and repetitive antigen array.

2. The composition of claim 1, wherein said virus-like particle is a recombinant virus-like particle.

3. The composition of claim 1, wherein said virus-like particle comprises, or alternatively consists of, recombinant proteins, or fragments thereof, of a RNA-phage, wherein preferably said RNA-phage is selected from :
(a) bacteriophage Qβ;
(b) bacteriophage R17;
(c) bacteriophage fr;
(d) bacteriophage GA;
(e) bacteriophage SP;
(f) bacteriophage MS2;
(g) bacteriophage M11;
(h) bacteriophage MX1;
(i) bacteriophage NL95;
(k) bacteriophage f2;
(l) bacteriophage PP7; and
(m) bacteriophage AP205.

4. The composition of claim 1, wherein said virus-like particle comprises, or alternatively consists of, recombinant proteins, or fragments thereof, of RNA-phage Qβ, of RNA-phage fr or of RNA-phage AP205.

5. The composition of any one of claims 3 or 4, wherein the recombinant proteins comprise, or alternatively consist essentially of, or alternatively consist of coat proteins of RNA phages, and wherein preferably said coat proteins of RNA phages have an amino acid sequence selected from :
(a) SEQ ID NO:4;
(b) a mixture of SEQ ID NO:4 and SEQ ID NO:5;
(c) SEQ ID NO:6;
(d) SEQ ID NO:7;
(e) SEQ ID NO:8;
(f) SEQ ID NO:9;
(g) a mixture of SEQ ID NO:9 and SEQ ID NO: 10;
(h) SEQ ID NO:11;
(i) SEQ ID NO: 12;
(k) SEQ ID NO: 13;
(l) SEQ ID NO: 14;
(m) SEQ ID NO:15;
(n) SEQ ID NO:16; and
(o) SEQ ID NO:28.

6. The composition of any one of claims 3 or 4, wherein the recombinant proteins comprise, or alternatively consist essentially of, or alternatively consist of mutant coat proteins of RNA phages, and wherein preferably said RNA-phage is selected from :
(a) bacteriophage Qβ;
(b) bacteriophage R17;
(c) bacteriophage fr;
(d) bacteriophage GA;
(e) bacteriophage SP;
(f) bacteriophage MS2;
(g) bacteriophage M11;
(h) bacteriophage MX1;
(i) bacteriophage NL95;
(k) bacteriophage f2;
(l) bacteriophage PP7; and
(m) bacteriophage AP205.

7. The composition of claim 6, wherein said mutant coat proteins of said RNA phage have been modified by (i) removal of at least one lysine residue by way of substitution; or (ii) addition of at least one lysine residue by way of substitution; or (iii) deletion of at least one lysine residue; or (iv) addition of at least one lysine residue by way of insertion.

8. The composition of any one of the preceding claims, wherein said second attachment site is capable of association to said first attachment site through at least one covalent bond, and wherein preferably said covalent bond is a non-peptide bond.

9. The composition of any one of the preceding claims, wherein said ghrelin or said ghrelin peptide is fused to said core particle.

10. The composition of any one of the preceding claims, wherein said antigen or antigenic determinant is a ghrelin or a ghrelin peptide being selected from :
(a) human ghrelin or human ghrelin peptide;
(b) bovine ghrelin or bovine ghrelin peptide;
(c) sheep ghrelin or sheep ghrelin peptide;
(d) dog ghrelin or dog ghrelin peptide;
(e) cat ghrelin or cat ghrelin peptide;
(f) mouse ghrelin or mouse ghrelin peptide;
(g) pig ghrelin or pig ghrelin peptide;
(h) horse ghrelin or horse ghrelin peptide; and
(k) a peptide or fragment thereof of any ghrelin of (a)-(h);
wherein preferably said antigen or antigenic determinant is
a) a ghrelin peptide, preferably a human ghrelin peptide or a dog ghrelin peptide, or
b) a ghrelin, preferably a human ghrelin or dog ghrelin.

11. The composition of any one of the preceding claims, wherein said ghrelin or said ghrelin peptide comprises, or preferably has, an amino acid sequence selected from :
(a) GSSFLSPEHQRVQRKESKKPPAKLQPR (SEQ ID NO: 48)
(b) GSSFLSPEHQRVQQRKESKKPPAKLQPR(SEQ ID NO: 31)
(c) GSSFLSPEHQKLQQRKESKKPPAKLQPR(SEQ ID NO: 49)
(d) GSSFLSPEHQKLQRKESKKPPAKLQPR(SEQ ID NO: 50)
(e) GSSFLSPEHQKAQQRKESKKPPAKLQPR(SEQ ID NO: 32)
(f) GSSFLSPEHQKAQRKESKKPPAKLQPR(SEQ ID NO: 51)
(g) KKPPAKLQPR(SEQ ID NO: 52)
(h) PPAKLQPR(SEQ ID NO: 53)
(i) AKLQPR(SEQ ID NO: 54)
(j) GSSFLSPEHQ(SEQ ID NO: 55)
(k) EHQRVQQRKE(SEQ ID NO: 56)
(l) KLQPR (SEQ ID NO: 59)
(m) GSSFLSPEHQRVQ (SEQ ID NO: 60)
(n) QRKESKKPPAKLQPR (SEQ ID NO: 61)
(o) GSSFLSPEHQKLQ (SEQ ID NO: 62)
(p) QRKESKKPPAKLQPR (SEQ ID NO: 63)
(q) EHQRVQQRKES (SEQ ID NO: 111)
(r) EHQKAQQRKE (SEQ ID NO: 112)
(s) EHQKAQQRKES (SEQ ID NO: 113)
(t) EHQKLQQRKE (SEQ ID NO: 114)
(u) EHQKLQQRKES (SEQ ID NO: 115)
(v) LSPEHQRVQQ (SEQ ID NO: 116)
(w) LSPEHQKAQQ (SEQ ID NO: 117)
(x) LSPEHQKLQQ (SEQ ID NO: 118), and
(y) GSSFLSP (SEQ ID NO: 119);
wherein preferably said ghrelin or said ghrelin peptide with said at least second attachment site comprises, preferably consists of, an amino acid sequence selected from :
(a) CGSSFLSPEHQRVQRKESKKPPAKLQPR(SEQ ID NO: 64)
(b) CGSSFLSPEHQRVQQRKESKKPPAKLQPR(SEQ ID NO: 65)
(c) CGSSFLSPEHQKLQQRKESKKPPAKLQPR(SEQ ID NO: 71)
(d) CGSSFLSPEHQKLQRKESKKPPAKLQPR(SEQ ID NO: 72)
(e) CGSSFLSPEHQKAQQRKESKKPPAKLQPR(SEQ ID NO: 77)
(f) CGSSFLSPEHQKAQRKESKKPPAKLQPR(SEQ ID NO: 106)
(g) GSSFLSPEHQRVQRKESKKPPAKLQPRC(SEQ ID NO: 66)
(h) GSSFLSPEHQRVQRKESKKPPAKLQPRGC (SEQ ID NO: 120)
(i) GSSFLSPEHQRVQQRKESKKPPAKLQPRC(SEQ ID NO: 67)
(j) GSSFLSPEHQRVQQRKESKKPPAKLQPRGC (SEQ ID NO: 121)
(k) GSSFLSPEHQKLQQRKESKKPPAKLQPRC(SEQ ID NO: 73)
(l) GSSFLSPEHQKLQQRKESKKPPAKLQPRGC (SEQ ID NO: 123)
(m) GSSFLSPEHQKLQRKESKKPPAKLQPRC(SEQ ID NO: 74)
(n) GSSFLSPEHQKLQRKESKKPPAKLQPRGC (SEQ ID NO: 124)
(o) GSSFLSPEHQKAQQRKESKKPPAKLQPRC(SEQ ID NO: 105)
(p) GSSFLSPEHQKAQRKESKKPPAKLQPRC(SEQ ID NO: 107)
(q) CKKPPAKLQPR(SEQ ID NO: 108)
(r) CPPAKLQPR(SEQ ID NO: 70)
(s) CAKLQPR (SEQ ID NO: 109)
(t) GSSFLSPEHQC (SEQ ID NO: 110)
(u) CEHQRVQQRKE (SEQ ID NO: 76)
(v) GSSFLSPEHQRVQC (SEQ ID NO: 68)
(w) GSSFLSPEHQRVQGC (SEQ ID NO: 122)
(x) CQRKESKKPPAKLQPR (SEQ ID NO: 69)
(y) GSSFLSPEHQKLQC (SEQ ID NO: 75)
(z) GSSFLSPEHQKLQGC (SEQ ID NO: 125)
(aa) GSSFLSPEHQKAQRKESKKPPAKLQPRC (SEQ ID NO: 126)
(bb) GSSFLSPEHQKAQRKESKKPPAKLQPRGC (SEQ ID NO: 127)
(cc) GSSFLSPEHQKAQQRKESKKPPAKLQPRC (SEQ ID NO: 128)
(dd)GSSFLSPEHQKAQQRKESKKPPAKLQPRGC (SEQ ID NO: 129)
(ee) GSSFLSPEHQKAQC (SEQ ID NO: 130)
(ff) GSSFLSPEHQKAQGC (SEQ ID NO: 131)
(gg) GGSSFLSPEHQGC (SEQ ID NO: 132)
(hh) CKKPPAKLQPR (SEQ ID NO: 133)
(ii) CEHQKAQQRKE (SEQ ID NO: 134)
(jj) CEHQKAQQRKES (SEQ ID NO: 135)
(kk) CLSPEHQKAQQ (SEQ ID NO: 136)
(ll) CEHQRVQQRKES (SEQ ID NO: 137) and
(mm) CLSPEHQRVQQ (SEQ ID NO: 138).

12. The composition of any one of the preceding claims further comprising an amino acid linker, wherein said amino acid linker comprises, or alternatively consists of, said second attachment site, and wherein preferably said second attachment or said amino acid linker with said second attachment site is bound to said ghrelin or said ghrelin peptide at its C-terminus, or alternatively at its N-terminus, and wherein further preferably said second attachment site or said amino acid linker with said second attachment site is selected from :
(a) GGC;
(b) GGC-CONH2;
(c) GC;
(d) GC-CONH2;
(e) C; and
(f) C-CONH2.

13. The composition of any one of the preceding claims, wherein said antigen or antigenic determinant is a biologically inactive form of ghrelin, or preferably of a ghrelin peptide, not containing a n-octanoyl-modification.

14. The composition of claim 1, wherein said antigen or antigenic determinant contains an N-octanoyl modification of a serine residue being or corresponding to the serine residue at position of SEQ ID NO: 31.

15. The composition of claim 1, wherein said first attachment is a lysine residue and said second attachment site is a cysteine residue.

16. A pharmaceutical composition comprising:
(a) the composition of claim 1; and
(b) an acceptable pharmaceutical carrier;
and wherein preferably said pharmaceutical composition further comprises an adjuvant or wherein preferably said pharmaceutical composition is devoid of an adjuvant.

17. A vaccine composition comprising the composition of any one of the preceding claims, and wherein preferably said vaccine composition further comprises an adjuvant or wherein preferably said vaccine composition is devoid of an adjuvant.

18. A process for producing a composition of any one of the preceding claims comprising:
(a) providing a core particle as defined in any one of claims 1 to 8 and 15;
(b) providing at least one antigen or antigenic determinant as defined in any one of claims 1 and 8 to 14; and
(c) combining said core particle and said at least one antigen or antigenic determinant, wherein said antigen or antigenic determinant and said core particle interact through said association to form an ordered and repetitive antigen array.

19. Composition of any one of claims 1 to 16 for use as a medicament.

20. Use of a composition of any one of claims 1 to 16 for the manufacture of a medicament for treatment of obesity.

21. Composition of any one of claims 1 to 16 for use for the treatment of obesity.

22. A composition of any one of claims 1 to 16 for use in a method of immunization comprising administering said composition to an animal or human, wherein preferably said antigen or antigenic determinant is a self-antigen, and/or wherein preferably (i) said animal is a human, and wherein said antigen or antigenic determinant is a human ghrelin or a human ghrelin peptide; or (ii) wherein said animal is of feline origin, and wherein said antigen or antigenic determinant is a ghrelin or ghrelin peptide; or (iii) wherein said animal is of canine origin, and wherein said antigen or antigenic determinant is a feline ghreline or ghreline peptide.

## Patentansprüche

1. Zusammensetzung, umfassend:
(a) ein Kernpartikel mit mindestens einer ersten Anlagerungsstelle, wobei das Kernpartikel ein virusartiges Partikel eines RNA-Phagen ist; und
(b) mindestens ein(e) Antigen oder antigene Determinante mit mindestens einer zweiten Anlagerungsstelle,
wobei das Antigen oder die antigene Determinante Ghrelin oder ein Ghrelinpeptid ist, wobei das Ghrelinpeptid mindestens fünf aufeinander folgende Aminosäuren von Ghrelin enthält, und wobei die zweite Anlagerungsstelle ausgewählt ist aus:
(i) einer Anlagerungsstelle, die mit dem Antigen oder der antigenen Determinante nicht natürlich vorkommt; und
(ii) einer Anlagerungsstelle, die mit dem Antigen oder der antigenen Determinante natürlich vorkommt,
wobei die zweite Anlagerungsstelle zur Assoziation mit der ersten Anlagerungsstelle fähig ist; und wobei das Ghrelin oder ein Ghrelinpeptid und das Kernpartikel durch die Assoziation wechselwirken, um einen geordneten und repetitiven Antigen-Array zu bilden.

2. Zusammensetzung nach Anspruch 1, wobei das virusartige Partikel ein rekombinantes virusartiges Partikel ist.

3. Zusammensetzung nach Anspruch 1, wobei das virusartige Partikel rekombinante Proteine oder Fragmente davon eines RNA-Phagen umfasst oder alternativ daraus besteht, vorzugsweise wobei der RNA-Phage ausgewählt ist aus:
(a) Bakteriophage Qβ;
(b) Bakteriophage R17;
(c) Bakteriophage fr;
(d) Bakteriophage GA;
(e) Bakteriophage SP;
(f) Bakteriophage MS2;
(g) Bakteriophage M11;
(h) Bakteriophage MX1;
(i) Bakteriophage NL95;
(k) Bakteriophage f2;
(l) Bakteriophage PP7; und
(m) Bakteriophage AP205.

4. Zusammensetzung nach Anspruch 1, wobei das virusartige Partikel rekombinante Proteine oder Fragmente davon von RNA-Phage Qβ, RNA-Phage fr oder von RNA-Phage AP205 umfasst oder alternativ daraus besteht.

5. Zusammensetzung nach einem beliebigen der Ansprüche 3 oder 4, wobei die rekombinanten Proteine Hüllproteine von RNA-Phagen umfassen oder alternativ im Wesentlichen daraus bestehen oder alternativ daraus bestehen, und vorzugsweise
wobei die Hüllproteine von RNA-Phagen eine Aminosäuresequenz aufweisen, die ausgewählt ist aus:
(a) SEQ ID NO: 4;
(b) einem Gemisch aus SEQ ID NO: 4 und SEQ ID NO: 5;
(c) SEQ ID NO: 6;
(d) SEQ ID NO: 7;
(e) SEQ ID NO: 8;
(f) SEQ ID NO: 9;
(g) einem Gemisch aus SEQ ID NO: 9 und SEQ ID NO: 10;
(h) SEQ ID NO: 11;
(i) SEQ ID NO: 12;
(k) SEQ ID NO: 13;
(l) SEQ ID NO: 14;
(m) SEQ ID NO: 15;
(n) SEQ ID NO: 16; und
(o) SEQ ID NO: 28.

6. Zusammensetzung nach einem beliebigen der Ansprüche 3 oder 4, wobei die rekombinanten Proteine mutierte Hüllproteine von RNA-Phagen umfassen oder alternativ im Wesentlichen daraus bestehen oder alternativ daraus bestehen, und vorzugsweise wobei der RNA-Phage ausgewählt ist aus:
(a) Bakteriophage Qβ;
(b) Bakteriophage R17;
(c) Bakteriophage fr;
(d) Bakteriophage GA;
(e) Bakteriophage SP;
(f) Bakteriophage MS2;
(g) Bakteriophage M11;
(h) Bakteriophage MX1;
(i) Bakteriophage NL95;
(k) Bakteriophage f2;
(l) Bakteriophage PP7; und
(m) Bakteriophage AP205.

7. Zusammensetzung nach Anspruch 6, wobei die mutierten Hüllproteine von dem RNA-Phagen modifiziert worden sind durch (i) Entfernen von mindestens einem Lysinrest mittels Substitution; oder (ii) Addition von mindestens einem Lysinrest mittels Substitution; oder (iii) Deletion von mindestens einem Lysinrest; oder (iv) Addition von mindestens einem Lysinrest mittels Insertion.

8. Zusammensetzung nach einem beliebigen der vorangehenden Ansprüche, wobei die zweite Anlagerungsstelle zur Assoziation mit der ersten Anlagerungsstelle durch mindestens eine kovalente Bindung fähig ist, und vorzugsweise wobei die kovalente Bindung eine Nicht-Peptid-Bindung ist.

9. Zusammensetzung nach einem beliebigen der vorangehenden Ansprüche, wobei das Ghrelin oder das Ghrelinpeptid mit dem Kernpartikel fusioniert ist.

10. Zusammensetzung nach einem beliebigen der vorangehenden Ansprüche, wobei das Antigen oder die antigene Determinante ein Ghrelin oder ein Ghrelinpeptid ist, das ausgewählt ist aus:
(a) Humanghrelin oder Humanghrelinpeptid;
(b) Rinderghrelin oder Rinderghrelinpeptid;
(c) Schafghrelin oder Schafghrelinpeptid;
(d) Hundeghrelin oder Hundeghrelinpeptid;
(e) Katzenghrelin oder Katzenghrelinpeptid;
(f) Mäuseghrelin oder Mäuseghrelinpeptid;
(g) Schweineghrelin oder Schweineghrelinpeptid;
(h) Pferdeghrelin oder Pferdeghrelinpeptid; und
(k) einem Peptid oder Fragment davon von einem beliebigen Ghrelin von (a)-(h);
vorzugsweise wobei das Antigen oder die antigene Determinante ist
a) ein Ghrelinpeptid, vorzugsweise ein Humanghrelinpeptid oder ein Hundeghrelinpeptid, oder
b) ein Ghrelin, vorzugsweise ein Humanghrelin oder Hundeghrelin.

11. Zusammensetzung nach einem beliebigen der vorangehenden Ansprüche, wobei das Ghrelin oder das Ghrelinpeptid eine Aminosäuresequenz umfasst oder vorzugsweise aufweist, die ausgewählt ist aus:
(a) GSSFLSPEHQRVQRKESKKPPAKLQPR (SEQ ID NO: 48)
(b) GSSFLSPEHQRVQQRKESKKPPAKLQPR (SEQ ID NO: 31)
(c) GSSFLSPEHQKLQQRKESKKPPAKLQPR (SEQ ID NO: 49)
(d) GSSFLSPEHQKLQRKESKKPPAKLQPR (SEQ ID NO: 50)
(e) GSSFLSPEHQKAQQRKESKKPPAKLQPR (SEQ ID NO: 32)
(f) GSSFLSPEHQKAQRKESKKPPAKLQPR (SEQ ID NO: 51)
(g) KKPPAKLQPR (SEQ ID NO: 52)
(h) PPAKLQPR (SEQ ID NO: 53)
(i) AKLQPR (SEQ ID NO: 54)
(j) GSSFLSPEHQ (SEQ ID NO: 55)
(k) EHQRVQQRKE (SEQ ID NO: 56)
(l) KLQPR (SEQ ID NO: 59)
(m) GSSFLSPEHQRVQ (SEQ ID NO: 60)
(n) QRKESKKPPAKLQPR (SEQ ID NO: 61)
(o) GSSFLSPEHQKLQ (SEQ ID NO: 62)
(p) QRKESKKPPAKLQPR (SEQ ID NO: 63)
(q) EHQRVQQRKES (SEQ ID NO: 111)
(r) EHQKAQQRKE (SEQ ID NO: 112)
(s) EHQKAQQRKES (SEQ ID NO: 113)
(t) EHQKLQQRKE (SEQ ID NO: 114)
(u) EHQKLQQRKES (SEQ ID NO: 115)
(v) LSPEHQRVQQ (SEQ ID NO: 116)
(w) LSPEHQKAQQ (SEQ ID NO: 117)
(x) LSPEHQKLQQ (SEQ ID NO: 118), und
(y) GSSFLSP (SEQ ID NO: 119);
vorzugsweise wobei das Ghrelin oder das Ghrelinpeptid mit der mindestens zweiten Anlagerungsstelle eine Aminosäuresequenz umfasst, vorzugsweise daraus besteht, die ausgewählt ist aus:
(a) CGSSFLSPEHQRVQRKESKKPPAKLQPR (SEQ ID NO: 64)
(b) CGSSFLSPEHQRVQQRKESKKPPAKLQPR (SEQ ID NO: 65)
(c) CGSSFLSPEHQKLQQRKESKKPPAKLQPR (SEQ ID NO: 71)
(d) CGSSFLSPEHQKLQRKESKKPPAKLQPR (SEQ ID NO: 72)
(e) CGSSFLSPEHQKAQQRKESKKPPAKLQPR (SEQ ID NO: 77)
(f) CGSSFLSPEHQKAQRKESKKPPAKLQPR (SEQ ID NO: 106)
(g) GSSFLSPEHQRVQRKESKKPPAKLQPRC (SEQ ID NO: 66)
(h) GSSFLSPEHQRVQRKESKKPPAKLQPRGC (SEQ ID NO: 120)
(i) GSSFLSPEHQRVQQRKESKKPPAKLQPRC (SEQ ID NO: 67)
(j) GSSFLSPEHQRVQQRKESKKPPAKLQPRGC (SEQ ID NO: 121)
(k) GSSFLSPEHQKLQQRKESKKPPAKLQPRC (SEQ ID NO: 73)
(l) GSSFLSPEHQKLQQRKESKKPPAKLQPRGC (SEQ ID NO: 123)
(m) GSSFLSPEHQKLQRKESKKPPAKLQPRC (SEQ ID NO: 74)
(n) GSSFLSPEHQKLQRKESKKPPAKLQPRGC (SEQ ID NO: 124)
(o) GSSFLSPEHQKAQQRKESKKPPAKLQPRC (SEQ ID NO: 105)
(p) GSSFLSPEHQKAQRKESKKPPAKLQPRC (SEQ ID NO: 107)
(q) CKKPPAKLQPR (SEQ ID NO: 108)
(r) CPPAKLQPR (SEQ ID NO: 70)
(s) CAKLQPR (SEQ ID NO: 109)
(t) GSSFLSPEHQC (SEQ ID NO: 110)
(u) CEHQRVQQRKE (SEQ ID NO: 76)
(v) GSSFLSPEHQRVQC (SEQ ID NO: 68)
(w) GSSFLSPEHQRVQGC (SEQ ID NO: 122)
(x) CQRKESKKPPAKLQPR (SEQ ID NO: 69)
(y) GSSFLSPEHQKLQC (SEQ ID NO: 75)
(z) GSSFLSPEHQKLQGC (SEQ ID NO: 125)
(aa) GSSFLSPEHQKAQRKESKKPPAKLQPRC (SEQ ID NO: 126)
(bb) GSSFLSPEHQKAQRKESKKPPAKLQPRGC (SEQ ID NO: 127)
(cc) GSSFLSPEHQKAQQRKESKKPPAKLQPRC (SEQ ID NO: 128)
(dd) GSSFLSPEHQKAQQRKESKKPPAKLQPRGC (SEQ ID NO: 129)
(ee) GSSFLSPEHQKAQC (SEQ ID NO: 130)
(ff) GSSFLSPEHQKAQGC (SEQ ID NO: 131)
(gg) GGSSFLSPEHQGC (SEQ ID NO: 132)
(hh)CKKPPAKLQPR (SEQ ID NO: 133)
(ii) CEHQKAQQRKE (SEQ ID NO: 134)
(jj) CEHQKAQQRKES (SEQ ID NO: 135)
(kk) CLSPEHQKAQQ (SEQ ID NO: 136)
(ll) CEHQRVQQRKES (SEQ ID NO: 137) und
(mm) CLSPEHQRVQQ (SEQ ID NO: 138).

12. Zusammensetzung nach einem beliebigen der vorangehenden Ansprüche, weiterhin umfassend einen Aminosäure-Linker, wobei der Aminosäure-Linker die zweite Anlagerungsstelle umfasst oder alternativ daraus besteht, und vorzugsweise wobei die zweite Anlagerungsstelle oder der Aminosäure-Linker mit der zweiten Anlagerungsstelle an das Ghrelin oder das Ghrelinpeptid an seinem C-Terminus oder alternativ an seinem N-Terminus gebunden ist, und weiterhin vorzugsweise wobei die zweite Anlagerungsstelle oder der Aminosäure-Linker mit der zweiten Anlagerungsstelle ausgewählt ist aus:
(a) GGC;
(b) GGC-CONH2;
(c) GC;
(d) GC-CONH2;
(e) C; und
(f) C-CONH2.

13. Zusammensetzung nach einem beliebigen der vorangehenden Ansprüche, wobei das Antigen oder die antigene Determinante eine biologisch inaktive Form von Ghrelin oder vorzugsweise von einem Ghrelinpeptid, nicht enthaltend eine N-Oktanoyl-Modifikation, ist.

14. Zusammensetzung nach Anspruch 1, wobei das Antigen oder die antigene Determinante eine N-Oktanoyl-Modifikation eines Serinrestes enthält, der der Serinrest an Position von SEQ ID NO: 31 ist oder diesem entspricht.

15. Zusammensetzung nach Anspruch 1, wobei die erste Anlagerungsstelle ein Lysinrest ist und die zweite Anlagerungsstelle ein Cysteinrest ist.

16. Pharmazeutische Zusammensetzung, umfassend:
(a) die Zusammensetzung nach Anspruch 1; und
(b) einen verträglichen pharmazeutischen Träger;
und vorzugsweise wobei die pharmazeutische Zusammensetzung weiterhin ein Adjuvans umfasst oder vorzugsweise wobei die pharmazeutische Zusammensetzung frei von einem Adjuvans ist.

17. Vakzinzusammensetzung, umfassend die Zusammensetzung nach einem beliebigen der vorangehenden Ansprüche, vorzugsweise wobei die Vakzinzusammensetzung weiterhin ein Adjuvans umfasst oder vorzugsweise wobei die Vakzinzusammensetzung frei von einem Adjuvans ist.

18. Verfahren zum Herstellen einer Zusammensetzung gemäß einem beliebigen der vorangehenden Ansprüche, umfassend:
(a) Bereitstellen eines wie in einem beliebigen der Ansprüche 1 bis 8 und 15 definierten Kernpartikels;
(b) Bereitstellen mindestens eines/r Antigens oder antigenen Determinante wie definiert in einem beliebigen der Ansprüche 1 und 8 bis 14; und
(c) Kombinieren des Kempartikels und des/der mindestens einen Antigens oder antigenen Determinante, wobei das Antigen oder die antigene Determinante und das Kernpartikel durch die Assoziation wechselwirken, um einen geordneten und repetitiven Antigen-Array zu bilden.

19. Zusammensetzung nach einem beliebigen der Ansprüche 1 bis 16 zur Verwendung als ein Medikament.

20. Verwendung einer Zusammensetzung nach einem beliebigen der Ansprüche 1 bis 16 zur Herstellung eines Medikaments zur Behandlung von Fettsucht.

21. Zusammensetzung nach einem beliebigen der Ansprüche 1 bis 16 zur Verwendung für die Behandlung von Fettsucht.

22. Zusammensetzung nach einem beliebigen der Ansprüche 1 bis 16 zur Verwendung in einem Immunisierungsverfahren, umfassend Verabreichen der Zusammensetzung an ein Tier oder einen Menschen, vorzugsweise wobei das Antigen oder die antigene Determinante ein Selbst-Antigen ist, und/oder vorzugsweise wobei (i) das Tier ein Mensch ist, und wobei das Antigen oder die antigene Determinante ein Humanghrelin oder ein Humanghrelinpeptid ist; oder (ii) wobei das Tier felinen Ursprungs ist und wobei das Antigen oder die antigene Determinante ein Ghrelin oder Ghrelinpeptid ist; oder (iii) wobei das Tier caninen Ursprungs ist und wobei das Antigen oder die antigene Determinante ein felines Ghrelin oder Ghrelinpeptid ist.

## Revendications

1. Composition comprenant :
(a) une particule noyau avec au moins un premier site de liaison dans laquelle ladite particule noyau est une particule pseudovirale d'un phage à ARN ; et
(b) au moins un antigène ou un déterminant antigénique avec au moins un deuxième site de liaison,
dans laquelle ledit antigène ou déterminant antigénique est une ghréline ou un peptide de ghréline, dans laquelle le peptide de ghréline contient au moins cinq acides aminés consécutifs de ghréline, et dans laquelle ledit deuxième site de liaison est choisi parmi :
(i) un site de liaison n'existant pas naturellement avec ledit antigène ou déterminant antigénique ; et
(ii) un site de liaison existant naturellement avec ledit antigène ou déterminant antigénique,
dans laquelle ledit deuxième site de liaison est capable d'association audit premier site de liaison ; et dans laquelle ladite ghréline ou un peptide de ghréline et ladite particule noyau interagissent par le biais de ladite association pour former un réseau ordonné et répétitif d'antigènes.

2. Composition selon la revendication 1, dans laquelle ladite particule pseudovirale est une particule pseudovirale recombinante.

3. Composition selon la revendication 1, dans laquelle ladite particule pseudovirale comprend, ou alternativement consiste en, des protéines recombinantes, ou des fragments de celles-ci, d'un phage à ARN, dans laquelle de préférence ledit phage à ARN est choisi parmi :
(a) le bactériophage Qβ ;
(b) le bactériophage R17 ;
(c) le bactériophage fr ;
(d) le bactériophage GA ;
(e) le bactériophage SP ;
(f) le bactériophage MS2 ;
(g) le bactériophage M11 ;
(h) le bactériophage MX1 ;
(i) le bactériophage NL95 ;
(k) le bactériophage f2 ;
(l) le bactériophage PP7 ; et
(m) le bactériophage AP205.

4. Composition selon la revendication 1, dans laquelle ladite particule pseudovirale comprend, ou alternativement consiste en, des protéines recombinantes, ou des fragments de celles-ci, d'un phage à ARN Qβ, d'un phage à ARN fr ou d'un phage à ARN AP205.

5. Composition selon l'une quelconque des revendications 3 ou 4, dans laquelle les protéines recombinantes comprennent, ou alternativement consistent essentiellement en, ou alternativement consistent en, des protéines du manteau de phages à ARN, et dans laquelle de préférence lesdites protéines du manteau de phages à ARN ont une séquence d'acides aminés choisie parmi :
(a) SEQ ID NO: 4 ;
(b) un mélange de SEQ ID NO: 4 et SEQ ID NO: 5 ;
(c) SEQ ID NO: 6 ;
(d) SEQ ID NO: 7 ;
(e) SEQ ID NO: 8 ;
(f) SEQ ID NO: 9 ;
(g) un mélange de SEQ ID NO: 9 et SEQ ID NO: 10 ;
(h) SEQ ID NO: 11 ;
(i) SEQ ID NO: 12 ;
(k) SEQ ID NO: 13 ;
(l) SEQ ID NO: 14 ;
(m) SEQ ID NO: 15 ;
(n) SEQ ID NO: 16 ; et
(o) SEQ ID NO: 28.

6. Composition selon l'une quelconque des revendications 3 ou 4, dans laquelle les protéines recombinantes comprennent, ou alternativement consistent essentiellement en, ou alternativement consistent en, des protéines du manteau mutantes de phages à ARN, et dans laquelle de préférence ledit phage à ARN est choisi parmi :
(a) le bactériophage Qβ ;
(b) le bactériophage R17 ;
(c) le bactériophage fr ;
(d) le bactériophage GA ;
(e) le bactériophage SP ;
(f) le bactériophage MS2 ;
(g) le bactériophage M11 ;
(h) le bactériophage MX1 ;
(i) le bactériophage NL95 ;
(k) le bactériophage f2 ;
(l) le bactériophage PP7 ; et
(m) le bactériophage AP205.

7. Composition selon la revendication 6, dans laquelle lesdites protéines du manteau mutantes dudit phage à ARN ont été modifiées par (i) la suppression d'au moins un résidu lysine via une substitution ; ou (ii) l'addition d'au moins un résidu lysine via une substitution ; ou (iii) la délétion d'au moins un résidu lysine ; ou (iv) l'addition d'au moins un résidu lysine via une insertion.

8. Composition selon l'une quelconque des revendications précédentes, dans laquelle ledit deuxième site de liaison est capable d'association audit premier site de liaison par le biais d'au moins une liaison covalente, et dans laquelle de préférence ladite liaison covalente est une liaison non peptidique.

9. Composition selon l'une quelconque des revendications précédentes, dans laquelle ladite ghréline ou ledit peptide de ghréline est fusionné(e) à ladite particule noyau.

10. Composition selon l'une quelconque des revendications précédentes, dans laquelle ledit antigène ou déterminant antigénique est une ghréline ou un peptide de ghréline qui est choisi parmi :
(a) une ghréline humaine ou un peptide de ghréline humaine ;
(b) une ghréline bovine ou un peptide de ghréline bovine ;
(c) une ghréline de mouton ou un peptide de ghréline de mouton ;
(d) une ghréline de chien ou un peptide de ghréline de chien ;
(e) une ghréline de chat ou un peptide de ghréline de chat ;
(f) une ghréline de souris ou un peptide de ghréline de souris ;
(g) une ghréline de porc ou un peptide de ghréline de porc ;
(h) une ghréline de cheval ou un peptide de ghréline de cheval ; et
(k) un peptide ou un fragment de celui-ci de n'importe quelle ghréline de (a) à (h) ;
dans laquelle de préférence ledit antigène ou déterminant antigénique est
a) un peptide de ghréline, de préférence un peptide de ghréline humaine ou un peptide de ghréline de chien, ou
b) une ghréline, de préférence une ghréline humaine ou une ghréline de chien.

11. Composition selon l'une quelconque des revendications précédentes, dans laquelle ladite ghréline ou ledit peptide de ghréline comprend ou, de préférence possède, une séquence d'acides aminés choisie parmi :
(a) GSSFLSPEHQRVQRKESKKPPAKLQPR (SEQ ID NO: 48)
(b) GSSFLSPEHQRVQQRKESKKPPAKLQPR(SEQ ID NO: 31)
(c) GSSFLSPEHQKLQQRKESKKPPAKLQPR(SEQ ID NO: 49)
(d) GSSFLSPEHQKLQRKESKKPPAKLQPR(SEQ ID NO: 50)
(e) GSSFLSPEHQKAQQRKESKKPPAKLQPR(SEQ ID NO: 32)
(f) GSSFLSPEHQKAQRKESKKPPAKLQPR(SEQ ID NO: 51)
(g) KKPPAKLQPR(SEQ ID NO: 52)
(h) PPAKLQPR(SEQ ID NO: 53)
(i) AKLQPR(SEQ ID NO: 54)
(j) GSSFLSPEHQ(SEQ ID NO: 55)
(k) EHQRVQQRKE(SEQ ID NO: 56)
(l) KLQPR (SEQ ID NO: 59)
(m) GSSFLSPEHQRVQ (SEQ ID NO: 60)
(n) QRKESKKPPAKLQPR (SEQ ID NO: 61)
(o) GSSFLSPEHQKLQ (SEQ ID NO: 62)
(p) QRKESKKPPAKLQPR (SEQ ID NO: 63)
(q) EHQRVQQRKES (SEQ ID NO: 111)
(r) EHQKAQQRKE (SEQ ID NO: 112)
(s) EHQKAQQRKES (SEQ ID NO: 113)
(t) EHQKLQQRKE (SEQ ID NO: 114)
(u) EHQKLQQRKES (SEQ ID NO: 115)
(v) LSPEHQRVQQ (SEQ ID NO: 116)
(w) LSPEHQKAQQ (SEQ ID NO: 117)
(x) LSPEHQKLQQ (SEQ ID NO: 118), et
(y) GSSFLSP (SEQ ID NO: 119) ;
dans laquelle de préférence ladite ghréline ou ledit peptide de ghréline avec ledit au moins deuxième site de liaison comprend, de préférence consiste en, une séquence d'acides aminés choisie parmi :
(a) CGSSFLSPEHQRVQRKESKKPPAKLQPR(SEQ ID NO: 64)
(b) CGSSFLSPEHQRVQQRKESKKPPAKLQPR(SEQ ID NO: 65)
(c) CGSSFLSPEHQKLQQRKESKKPPAKLQPR(SEQ ID NO: 71)
(d) CGSSFLSPEHQKLQRKESKKPPAKLQPR(SEQ ID NO: 72)
(e) CGSSFLSPEHQKAQQRKESKKPPAKLQPR(SEQ ID NO: 77)
(f) CGSSFLSPEHQKAQRKESKKPPAKLQPR(SEQ ID NO: 106)
(g) GSSFLSPEHQRVQRKESKKPPAKLQPRC(SEQ ID NO: 66)
(h) GSSFLSPEHQRVQRKESKKPPAKLQPRGC (SEQ ID NO: 120)
(i) GSSFLSPEHQRVQQRKESKKPPAKLQPRC(SEQ ID NO: 67)
(j) GSSFLSPEHQRVQQRKESKKPPAKLQPRGC (SEQ ID NO: 121)
(k) GSSFLSPEHQKLQQRKESKKPPAKLQPRC(SEQ ID NO: 73)
(l) GSSFLSPEHQKLQQRKESKKPPAKLQPRGC (SEQ ID NO: 123)
(m) GSSFLSPEHQKLQRKESKKPPAKLQPRC(SEQ ID NO: 74)
(n) GSSFLSPEHQKLQRKESKKPPAKLQPRGC (SEQ ID NO: 124)
(o) GSSFLSPEHQKAQQRKESKKPPAKLQPRC(SEQ ID NO: 105)
(p) GSSFLSPEHQKAQRKESKKPPAKLQPRC(SEQ ID NO: 107)
(q) CKKPPAKLQPR(SEQ ID NO: 108)
(r) CPPAKLQPR(SEQ ID NO: 70)
(s) CAKLQPR(SEQ ID NO: 109)
(t) GSSFLSPEHQC(SEQ ID NO: 110)
(u) CEHQRVQQRKE(SEQ ID NO: 76)
(v) GSSFLSPEHQRVQC (SEQ ID NO: 68)
(w) GSSFLSPEHQRVQGC (SEQ ID NO: 122)
(x) CQRKESKKPPAKLQPR (SEQ ID NO: 69)
(y) GSSFLSPEHQKLQC (SEQ ID NO: 75)
(z) GSSFLSPEHQKLQGC (SEQ ID NO: 125)
(aa) GSSFLSPEHQKAQRKESKKPPAKLQPRC (SEQ ID NO: 126)
(bb) GSSFLSPEHQKAQRKESKKPPAKLQPRGC (SEQ ID NO: 127)
(cc) GSSFLSPEHQKAQQRKESKKPPAKLQPRC (SEQ ID NO: 128)
(dd) GSSFLSPEHQKAQQRKESKKPPAKLQPRGC (SEQ ID NO: 129)
(ee) GSSFLSPEHQKAQC (SEQ ID NO: 130)
(ff) GSSFLSPEHQKAQGC (SEQ ID NO: 131)
(gg) GGSSFLSPEHQGC (SEQ ID NO: 132)
(hh) CKKPPAKLQPR (SEQ ID NO: 133)
(ii) CEHQKAQQRKE (SEQ ID NO: 134)
(jj) CEHQKAQQRKES (SEQ ID NO: 135)
(kk) CLSPEHQKAQQ (SEQ ID NO: 136)
(ll) CEHQRVQQRKES (SEQ ID NO: 137) et
(mm) CLSPEHQRVQQ (SEQ ID NO: 138).

12. Composition selon l'une quelconque des revendications précédentes comprenant en outre un lieur d'acides aminés, dans laquelle ledit lieur d'acides aminés comprend, ou alternativement consiste en, ledit deuxième site de liaison, et dans laquelle de préférence ladite deuxième liaison ou ledit lieur d'acides aminés avec ledit deuxième site de liaison est lié à ladite ghréline ou audit peptide de ghréline à son extrémité C, ou alternativement à son extrémité N, et dans laquelle en outre de préférence ledit deuxième site de liaison ou ledit lieur d'acides aminés avec ledit deuxième site de liaison est choisi parmi :
(a) GGC ;
(b) GGC-CONH2 ;
(c) GC ;
(d) GC-CONH2 ;
(e) C ; et
(f) C-CONH2.

13. Composition selon l'une quelconque des revendications précédentes, dans laquelle ledit antigène ou déterminant antigénique est une forme biologiquement inactive de ghréline ou, de préférence d'un peptide de ghréline, ne contenant pas de modification n-octanoyle.

14. Composition selon la revendication 1, dans laquelle ledit antigène ou déterminant antigénique contient une modification N-octanoyle d'un résidu sérine qui est ou correspond au résidu sérine à la position de SEQ ID NO: 31.

15. Composition selon la revendication 1, dans laquelle ladite première liaison est un résidu lysine et ledit deuxième site de liaison est un résidu cystéine.

16. Composition pharmaceutique comprenant .
(a) la composition selon la revendication 1, et
(b) un support pharmaceutique acceptable ;
et dans laquelle de préférence, ladite composition pharmaceutique comprend en outre un adjuvant ou dans laquelle de préférence ladite composition pharmaceutique est dépourvue d'adjuvant.

17. Composition vaccinale comprenant la composition selon l'une quelconque des revendications précédentes, et dans laquelle de préférence ladite composition vaccinale comprend en outre un adjuvant ou dans laquelle de préférence ladite composition vaccinale est dépourvue d'adjuvant.

18. Procédé pour produire une composition selon l'une quelconque des revendications précédentes comprenant :
(a) la fourniture d'une particule noyau tel que défini dans l'une quelconque des revendications 1 à 8 et 15 ;
(b) la fourniture d'au moins un antigène ou déterminant antigénique tel que défini dans l'une quelconque des revendications 1 et 8 à 14 ; et
(c) la combinaison de ladite particule noyau et dudit au moins un antigène ou déterminant antigénique,
dans laquelle ledit antigène ou déterminant antigénique et ladite particule noyau interagissent par le biais de ladite association pour former un réseau ordonné et répétitif d'antigènes.

19. Composition selon l'une quelconque des revendications 1 à 16, pour une utilisation en tant que médicament.

20. Utilisation d'une composition selon l'une quelconque des revendications 1 à 16 pour la fabrication d'un médicament destiné au traitement de l'obésité.

21. Composition selon l'une quelconque des revendications 1 à 16 pour une utilisation pour le traitement de l'obésité.

22. Composition selon l'une quelconque des revendications 1 à 16 pour une utilisation dans un procédé d'immunisation comprenant l'administration de ladite composition à un animal ou un humain, dans laquelle de préférence ledit antigène ou déterminant antigénique est un auto-antigène, et/ou dans laquelle de préférence (i) ledit animal est un humain, et dans laquelle ledit antigène ou déterminant antigénique est une ghréline humaine ou un peptide de ghréline humaine, ou (ii) dans laquelle ledit animal est d'origine féline, et dans laquelle ledit antigène ou déterminant antigénique est une ghréline ou un peptide de ghréline ; ou (iii ) dans laquelle ledit animal est d'origine canine, et dans laquelle ledit antigène ou déterminant antigénique est une ghréline ou un peptide de ghréline de félin.


### SEQUENCE LISTING

<110> Cytos Biotechnology AG
   Bachmann, Martin F
   Fulurija, Alma
<120> Grehlin-Carrier.Conjugates
<130> PA040WO
<150> US 60/396,638
   <151> 2002-07-19
<160> 146
<170> PatentIn version 3.2
<210> 1
   <211> 172
   <212> PRT
   <213> Escherichia coli
<400> 1
   Met Ala Val Val Ser Phe Gly Val Asn Ala Ala Pro Thr Thr Pro Gln
   1 5 10 15
   Gly Gln Gly Arg Val Thr Phe Asn Gly Thr Val Val Asp Ala Pro Cys
   20 25 30
   Ser Ile Ser Gln Lys Ser Ala Asp Gln Ser Ile Asp Phe Gly Gln Leu
   35 40 45
   Ser Lys Ser Phe Leu Ala Asn Asp Gly Gln Ser Lys Pro Met Asn Leu
   50 55 60
   Asp Ile Glu Leu Val Asn Cys Asp Ile Thr Ala Phe Lys Asn Gly Asn
   65 70 75 80
   Ala Lys Thr Gly Ser Val Lys Leu Ala Phe Thr Gly Pro Thr Val Ser
   85 90 95
   Gly His Pro Ser Glu Leu Ala Thr Asn Gly Gly Pro Gly Thr Ala Ile
   100 105 110
   Met Ile Gln Ala Ala Gly Lys Asn Val Pro Phe Asp Gly Thr Glu Gly
   115 120 125
   Asp Pro Asn Leu Leu Lys Asp Gly Asp Asn Val Leu His Tyr Thr Thr
   130 135 140
   Val Gly Lys Lys Ser Ser Asp Gly Asn Ala Gln Ile Thr Glu Gly Ala
   145 150 155 160
   Phe Ser Gly Val Ala Thr Phe Asn Leu Ser Tyr Gln
   165 170
<210> 2
   <211> 182
   <212> PRT
   <213> Escherichia coli
<400> 2
   Met Lys Ile Lys Thr Leu Ala Ile Val Val Leu Ser Ala Leu Ser Leu
   1 5 10 15
   Ser Ser Thr Ala Ala Leu Ala Ala Ala Thr Thr Val Asn Gly Gly Thr
   20 25 30
   Val His Phe Lys Gly Glu Val Val Asn Ala Ala Cys Ala Val Asp Ala
   35 40 45
   Gly Ser Val Asp Gln Thr Val Gln Leu Gly Gln Val Arg Thr Ala Ser
   50 55 60
   Leu Ala Gln Glu Gly Ala Thr Ser Ser Ala Val Gly Phe Asn Ile Gln
   65 70 75 80
   Leu Asn Asp Cys Asp Thr Asn Val Ala Ser Lys Ala Ala Val Ala Phe
   85 90 95
   Leu Gly Thr Ala Ile Asp Ala Gly His Thr Asn Val Leu Ala Leu Gln
   100 105 110
   Ser Ser Ala Ala Gly Ser Ala Thr Asn Val Gly Val Gln Ile Leu Asp
   115 120 125
   Arg Thr Gly Ala Ala Leu Thr Leu Asp Gly Ala Thr Phe Ser Ser Glu
   130 135 140
   Thr Thr Leu Asn Asn Gly Thr Asn Thr Ile Pro Phe Gln Ala Arg Tyr
   145 150 155 160
   Phe Ala Thr Gly Ala Ala Thr Pro Gly Ala Ala Asn Ala Asp Ala Thr
   165 170 175
   Phe Lys Val Gln Tyr Gln
   180
<210> 3
   <211> 853
   <212> DNA
   <213> Escherichia coli
<400> 3
   acgtttctgt ggctcgacgc atcttcctca ttcttctctc caaaaaccac ctcatgcaat 60
   ataaacatct ataaataaag ataacaaata gaatattaag ccaacaaata aactgaaaaa 120
   gtttgtccgc gatgctttac ctctatgagt caaaatggcc ccaatgtttc atcttttggg 180
   ggaaactgtg cagtgttggc agtcaaactc gttgacaaac aaagtgtaca gaacgactgc 240
   ccatgtcgat ttagaaatag ttttttgaaa ggaaagcagc atgaaaatta aaactctggc    300
   aatcgttgtt ctgtcggctc tgtccctcag ttctacgacg gctctggccg ctgccacgac    360
   ggttaatggt gggaccgttc actttaaagg ggaagttgtt aacgccgctt gcgcagttga    420
   tgcaggctct gttgatcaaa ccgttcagtt aggacaggtt cgtaccgcat cgctggcaca    480
   ggaaggagca accagttctg ctgtcggttt taacattcag ctgaatgatt gcgataccaa    540
   tgttgcatct aaagccgctg ttgccttttt aggtacggcg attgatgcgg gtcataccaa    600
   cgttctggct ctgcagagtt cagctgcggg tagcgcaaca aacgttggtg tgcagatcct    660
   ggacagaacg ggtgctgcgc tgacgctgga tggtgcgaca tttagttcag aaacaaccct    720
   gaataacgga accaatacca ttccgttcca ggcgcgttat tttgcaaccg gggccgcaac    780
   cccgggtgct gctaatgcgg atgcgacctt caaggttcag tatcaataac ctacctaggt    840
   tcagggacgt tca                                                       853
<210> 4
   <211> 132
   <212> PRT
   <213> Bacteriophage Q-beta
<400> 4
   Ala Lys Leu Glu Thr Val Thr Leu Gly Asn Ile Gly Lys Asp Gly Lys
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65                  70                  75                  80
   Asp Pro Ser Val Thr Arg Gln Ala Tyr Ala Asp Val Thr Phe Ser Phe
                   85                  90                  95
   Thr Gln Tyr Ser Thr Asp Glu Glu Arg Ala Phe Val Arg Thr Glu Leu
               100                 105                 110
   Ala Ala Leu Leu Ala Ser Pro Leu Leu Ile Asp Ala Ile Asp Gln Leu
           115                 120                 125
   Asn Pro Ala Tyr
       130
<210> 5
   <211> 329
   <212> PRT
   <213> Bacteriophage Q-beta
<400> 5
   Met Ala Lys Leu Glu Thr Val Thr Leu Gly Asn Ile Gly Lys Asp Gly
   1               5                   10                  15
   Lys Gln Thr Leu Val Leu Asn Pro Arg Gly Val Asn Pro Thr Asn Gly
               20                  25                  30
   Val Ala Ser Leu Ser Gln Ala Gly Ala Val Pro Ala Leu Glu Lys Arg
           35                  40                  45
   Val Thr Val Ser Val Ser Gln Pro Ser Arg Asn Arg Lys Asn Tyr Lys
       50                  55                  60
   Val Gln Val Lys Ile Gln Asn Pro Thr Ala Cys Thr Ala Asn Gly Ser
   65                  70                  75                  80
   Cys Asp Pro Ser Val Thr Arg Gln Ala Tyr Ala Asp Val Thr Phe Ser
                   85                  90                  95
   Phe Thr Gln Tyr Ser Thr Asp Glu Glu Arg Ala Phe Val Arg Thr Glu
               100                 105                 110
   Leu Ala Ala Leu Leu Ala Ser Pro Leu Leu Ile Asp Ala Ile Asp Gln
           115                 120                 125
   Leu Asn Pro Ala Tyr Trp Thr Leu Leu Ile Ala Gly Gly Gly Ser Gly
       130                 135                 140
   Ser Lys Pro Asp Pro Val Ile Pro Asp Pro Pro Ile Asp Pro Pro Pro
   145                 150                 155                 160
   Gly Thr Gly Lys Tyr Thr Cys Pro Phe Ala Ile Trp Ser Leu Glu Glu
                   165                 170                 175
   Val Tyr Glu Pro Pro Thr Lys Asn Arg Pro Trp Pro Ile Tyr Asn Ala
               180                 185                 190
   Val Glu Leu Gln Pro Arg Glu Phe Asp Val Ala Leu Lys Asp Leu Leu
           195                 200                 205
   Gly Asn Thr Lys Trp Arg Asp Trp Asp Ser Arg Leu Ser Tyr Thr Thr
       210                 215                 220
   Phe Arg Gly Cys Arg Gly Asn Gly Tyr Ile Asp Leu Asp Ala Thr Tyr
   225                 230                 235                 240
   Leu Ala Thr Asp Gln Ala Met Arg Asp Gln Lys Tyr Asp Ile Arg Glu
                   245                 250                 255
   Gly Lys Lys Pro Gly Ala Phe Gly Asn Ile Glu Arg Phe Ile Tyr Leu
   260 265 270
   Lys Ser Ile Asn Ala Tyr Cys Ser Leu Ser Asp Ile Ala Ala Tyr His
   275 280 285
   Ala Asp Gly Val Ile Val Gly Phe Trp Arg Asp Pro Ser Ser Gly Gly
   290 295 300
   Ala Ile Pro Phe Asp Phe Thr Lys Phe Asp Lys Thr Lys Cys Pro Ile
   305 310 315 320
   Gln Ala Val Ile Val Val Pro Arg Ala
   325
<210> 6
   <211> 129
   <212> PRT
   <213> Bacteriophage R17
<400> 6
   Ala Ser Asn Phe Thr Gln Phe Val Leu Val Asn Asp Gly Gly Thr Gly
   1 5 10 15
   Asn Val Thr Val Ala Pro Ser Asn Phe Ala Asn Gly Val Ala Glu Trp
   20 25 30
   Ile Ser Ser Asn Ser Arg Ser Gln Ala Tyr Lys Val Thr Cys Ser Val
   35 40 45
   Arg Gln Ser Ser Ala Gln Asn Arg Lys Tyr Thr Ile Lys Val Glu Val
   50 55 60
   Pro Lys Val Ala Thr Gln Thr Val Gly Gly Val Glu Leu Pro Val Ala
   65 70 75 80
   Ala Trp Arg Ser Tyr Leu Asn Met Glu Leu Thr Ile Pro Ile Phe Ala
   85 90 95
   Thr Asn Ser Asp Cys Glu Leu Ile Val Lys Ala Met Gln Gly Leu Leu
   100 105 110
   Lys Asp Gly Asn Pro Ile Pro Ser Ala Ile Ala Ala Asn Ser Gly Ile
   115 120 125
   Tyr
<210> 7
   <211> 130
   <212> PRT
   <213> Bacteriophage fr
<400> 7
   Met Ala Ser Asn Phe Glu Glu Phe Val Leu Val Asp Asn Gly Gly Thr
   1 5 10 15
   Gly Asp Val Lys Val Ala Pro Ser Asn Phe Ala Asn Gly Val Ala Glu
   20 25 30
   Trp Ile Ser Ser Asn Ser Arg Ser Gln Ala Tyr Lys Val Thr Cys Ser
   35 40 45
   Val Arg Gln Ser Ser Ala Asn Asn Arg Lys Tyr Thr Val Lys Val Glu
   50 55 60
   Val Pro Lys Val Ala Thr Gln Val Gln Gly Gly Val Glu Leu Pro Val
   65 70 75 80
   Ala Ala Trp Arg Ser Tyr Met Asn Met Glu Leu Thr Ile Pro Val Phe
   85 90 95
   Ala Thr Asn Asp Asp Cys Ala Leu Ile Val Lys Ala Leu Gln Gly Thr
   100 105 110
   Phe Lys Thr Gly Asn Pro Ile Ala Thr Ala Ile Ala Ala Asn Ser Gly
   115 120 125
   Ile Tyr
   130
<210> 8
   <211> 130
   <212> PRT
   <213> Bacteriophage GA
<400> 8
   Met Ala Thr Leu Arg Ser Phe Val Leu Val Asp Asn Gly Gly Thr Gly
   1 5 10 15
   Asn Val Thr Val Val Pro Val Ser Asn Ala Asn Gly Val Ala Glu Trp
   20 25 30
   Leu Ser Asn Asn Ser Arg Ser Gln Ala Tyr Arg Val Thr Ala Ser Tyr
   35 40 45
   Arg Ala Ser Gly Ala Asp Lys Arg Lys Tyr Ala Ile Lys Leu Glu Val
   50 55 60
   Pro Lys Ile Val Thr Gln Val Val Asn Gly Val Glu Leu Pro Gly Ser
   65 70 75 80
   Ala Trp Lys Ala Tyr Ala Ser Ile Asp Leu Thr Ile Pro Ile Phe Ala
   85 90 95
   Ala Thr Asp Asp Val Thr Val Ile Ser Lys Ser Leu Ala Gly Leu Phe
   100 105 110
   Lys Val Gly Asn Pro Ile Ala Glu Ala Ile Ser Ser Gln Ser Gly Phe
   115 120 125
   Tyr Ala
   130
<210> 9
   <211> 132
   <212> PRT
   <213> Bacteriophage SP
<400> 9
   Met Ala Lys Leu Asn Gln Val Thr Leu Ser Lys Ile Gly Lys Asn Gly
   1 5 10 15
   Asp Gln Thr Leu Thr Leu Thr Pro Arg Gly Val Asn Pro Thr Asn Gly
   20 25 30
   Val Ala Ser Leu Ser Glu Ala Gly Ala Val Pro Ala Leu Glu Lys Arg
   35 40 45
   Val Thr Val Ser Val Ala Gln Pro Ser Arg Asn Arg Lys Asn Phe Lys
   50 55 60
   Val Gln Ile Lys Leu Gln Asn Pro Thr Ala Cys Thr Arg Asp Ala Cys
   65 70 75 80
   Asp Pro Ser Val Thr Arg Ser Ala Phe Ala Asp Val Thr Leu Ser Phe
   85 90 95
   Thr Ser Tyr Ser Thr Asp Glu Glu Arg Ala Leu Ile Arg Thr Glu Leu
   100 105 110
   Ala Ala Leu Leu Ala Asp Pro Leu Ile Val Asp Ala Ile Asp Asn Leu
   115 120 125
   Asn Pro Ala Tyr
   130
<210> 10
   <211> 329
   <212> PRT
   <213> Bacteriophage SP
<400> 10
   Ala Lys Leu Asn Gln Val Thr Leu Ser Lys Ile Gly Lys Asn Gly Asp
   1 5 10 15
   Gln Thr Leu Thr Leu Thr Pro Arg Gly Val Asn Pro Thr Asn Gly Val
   20 25 30
   Ala Ser Leu Ser Glu Ala Gly Ala Val Pro Ala Leu Glu Lys Arg Val
   35 40 45
   Thr Val Ser Val Ala Gln Pro Ser Arg Asn Arg Lys Asn Phe Lys Val
   50 55 60
   Gln Ile Lys Leu Gln Asn Pro Thr Ala Cys Thr Arg Asp Ala Cys Asp
   65 70 75 80
   Pro Ser Val Thr Arg Ser Ala Phe Ala Asp Val Thr Leu Ser Phe Thr
   85 90 95
   Ser Tyr Ser Thr Asp Glu Glu Arg Ala Leu Ile Arg Thr Glu Leu Ala
   100 105 110
   Ala Leu Leu Ala Asp Pro Leu Ile Val Asp Ala Ile Asp Asn Leu Asn
   115 120 125
   Pro Ala Tyr Trp Ala Ala Leu Leu Val Ala Ser Ser Gly Gly Gly Asp
   130 135 140
   Asn Pro Ser Asp Pro Asp Val Pro Val Val Pro Asp Val Lys Pro Pro
   145 150 155 160
   Asp Gly Thr Gly Arg Tyr Lys Cys Pro Phe Ala Cys Tyr Arg Leu Gly
   165 170 175
   Ser Ile Tyr Glu Val Gly Lys Glu Gly Ser Pro Asp Ile Tyr Glu Arg
   180 185 190
   Gly Asp Glu Val Ser Val Thr Phe Asp Tyr Ala Leu Glu Asp Phe Leu
   195 200 205
   Gly Asn Thr Asn Trp Arg Asn Trp Asp Gln Arg Leu Ser Asp Tyr Asp
   210 215 220
   Ile Ala Asn Arg Arg Arg Cys Arg Gly Asn Gly Tyr Ile Asp Leu Asp
   225 230 235 240
   Ala Thr Ala Met Gln Ser Asp Asp Phe Val Leu Ser Gly Arg Tyr Gly
   245 250 255
   Val Arg Lys Val Lys Phe Pro Gly Ala Phe Gly Ser Ile Lys Tyr Leu
   260 265 270
   Leu Asn Ile Gln Gly Asp Ala Trp Leu Asp Leu Ser Glu Val Thr Ala
   275 280 285
   Tyr Arg Ser Tyr Gly Met Val Ile Gly Phe Trp Thr Asp Ser Lys Ser
   290 295 300
   Pro Gln Leu Pro Thr Asp Phe Thr Gln Phe Asn Ser Ala Asn Cys Pro
   305 310 315 320
   Val Gln Thr Val Ile Ile Ile Pro Ser
   325
<210> 11
   <211> 130
   <212> PRT
   <213> Bacteriophage MS2
<400> 11
   Met Ala Ser Asn Phe Thr Gln Phe Val Leu Val Asp Asn Gly Gly Thr
   1 5 10 15
   Gly Asp Val Thr Val Ala Pro Ser Asn Phe Ala Asn Gly Val Ala Glu
   20 25 30
   Trp Ile Ser Ser Asn Ser Arg Ser Gln Ala Tyr Lys Val Thr Cys Ser
   35 40 45
   Val Arg Gln Ser Ser Ala Gln Asn Arg Lys Tyr Thr Ile Lys Val Glu
   50 55 60
   Val Pro Lys Val Ala Thr Gln Thr Val Gly Gly Val Glu Leu Pro Val
   65 70 75 80
   Ala Ala Trp Arg Ser Tyr Leu Asn Met Glu Leu Thr Ile Pro Ile Phe
   85 90 95
   Ala Thr Asn Ser Asp Cys Glu Leu Ile Val Lys Ala Met Gln Gly Leu
   100 105 110
   Leu Lys Asp Gly Asn Pro Ile Pro Ser Ala Ile Ala Ala Asn Ser Gly
   115 120 125
   Ile Tyr
   130
<210> 12
   <211> 133
   <212> PRT
   <213> Bacteriophage M11
<400> 12
   Met Ala Lys Leu Gln Ala Ile Thr Leu Ser Gly Ile Gly Lys Lys Gly
   1 5 10 15
   Asp Val Thr Leu Asp Leu Asn Pro Arg Gly Val Asn Pro Thr Asn Gly
   20 25 30
   Val Ala Ala Leu Ser Glu Ala Gly Ala Val Pro Ala Leu Glu Lys Arg
   35 40 45
   Val Thr Ile Ser Val Ser Gln Pro Ser Arg Asn Arg Lys Asn Tyr Lys
   50 55 60
   Val Gln Val Lys Ile Gln Asn Pro Thr Ser Cys Thr Ala Ser Gly Thr
   65 70 75 80
   Cys Asp Pro Ser Val Thr Arg Ser Ala Tyr Ser Asp Val Thr Phe Ser
   85 90 95
   Phe Thr Gln Tyr Ser Thr Val Glu Glu Arg Ala Leu Val Arg Thr Glu
   100 105 110
   Leu Gln Ala Leu Leu Ala Asp Pro Met Leu Val Asn Ala Ile Asp Asn
   115 120 125
   Leu Asn Pro Ala Tyr
   130
<210> 13
   <211> 133
   <212> PRT
   <213> Bacteriophage MX1
<400> 13
   Met Ala Lys Leu Gln Ala Ile Thr Leu Ser Gly Ile Gly Lys Asn Gly
   1               5                   10                  15
   Asp Val Thr Leu Asn Leu Asn Pro Arg Gly Val Asn Pro Thr Asn Gly
               20                  25                  30
   Val Ala Ala Leu Ser Glu Ala Gly Ala Val Pro Ala Leu Glu Lys Arg
           35                  40                  45
   Val Thr Ile Ser Val Ser Gln Pro Ser Arg Asn Arg Lys Asn Tyr Lys
       50                  55                  60
   Val Gln Val Lys Ile Gln Asn Pro Thr Ser Cys Thr Ala Ser Gly Thr
   65                  70                  75                  80
   Cys Asp Pro Ser Val Thr Arg Ser Ala Tyr Ala Asp Val Thr Phe Ser
                   85                  90                  95
   Phe Thr Gln Tyr Ser Thr Asp Glu Glu Arg Ala Leu Val Arg Thr Glu
               100                 105                 110
   Leu Lys Ala Leu Leu Ala Asp Pro Met Leu Ile Asp Ala Ile Asp Asn
           115                 120                 125
   Leu Asn Pro Ala Tyr
       130
<210> 14
   <211> 330
   <212> PRT
   <213> Bacteriophage NL95
<400> 14
   Met Ala Lys Leu Asn Lys Val Thr Leu Thr Gly Ile Gly Lys Ala Gly
   1               5                   10                  15
   Asn Gln Thr Leu Thr Leu Thr Pro Arg Gly Val Asn Pro Thr Asn Gly
               20                  25                  30
   Val Ala Ser Leu Ser Glu Ala Gly Ala Val Pro Ala Leu Glu Lys Arg
           35                  40                  45
   Val Thr Val Ser Val Ala Gln Pro Ser Arg Asn Arg Lys Asn Tyr Lys
       50                  55                  60
   Val Gln Ile Lys Leu Gln Asn Pro Thr Ala Cys Thr Lys Asp Ala Cys
   65                  70                  75                  80
   Asp Pro Ser Val Thr Arg Ser Gly Ser Arg Asp Val Thr Leu Ser Phe
                   85                  90                  95
   Thr Ser Tyr Ser Thr Glu Arg Glu Arg Ala Leu Ile Arg Thr Glu Leu
               100                 105                 110
   Ala Ala Leu Leu Lys Asp Asp Leu Ile Val Asp Ala Ile Asp Asn Leu
           115                 120                 125
   Asn Pro Ala Tyr Trp Ala Ala Leu Leu Ala Ala Ser Pro Gly Gly Gly
       130                 135                 140
   Asn Asn Pro Tyr Pro Gly Val Pro Asp Ser Pro Asn Val Lys Pro Pro
   145                 150                 155                 160
   Gly Gly Thr Gly Thr Tyr Arg Cys Pro Phe Ala Cys Tyr Arg Arg Gly
                   165                 170                 175
   Glu Leu Ile Thr Glu Ala Lys Asp Gly Ala Cys Ala Leu Tyr Ala Cys
               180                 185                 190
   Gly Ser Glu Ala Leu Val Glu Phe Glu Tyr Ala Leu Glu Asp Phe Leu
           195                 200                 205
   Gly Asn Glu Phe Trp Arg Asn Trp Asp Gly Arg Leu Ser Lys Tyr Asp
       210                 215                 220
   Ile Glu Thr His Arg Arg Cys Arg Gly Asn Gly Tyr Val Asp Leu Asp
   225 230 235 240
   Ala Ser Val Met Gln Ser Asp Glu Tyr Val Leu Ser Gly Ala Tyr Asp
   245 250 255
   Val Val Lys Met Gln Pro Pro Gly Thr Phe Asp Ser Pro Arg Tyr Tyr
   260 265 270
   Leu His Leu Met Asp Gly Ile Tyr Val Asp Leu Ala Glu Val Thr Ala
   275 280 285
   Tyr Arg Ser Tyr Gly Met Val Ile Gly Phe Trp Thr Asp Ser Lys Ser
   290 295 300
   Pro Gln Leu Pro Thr Asp Phe Thr Arg Phe Asn Arg His Asn Cys Pro
   305 310 315 320
   Val Gln Thr Val Ile Val Ile Pro Ser Leu
   325 330
<210> 15
   <211> 129
   <212> PRT
   <213> Bacteriophage f2
<400> 15
   Ala Ser Asn Phe Thr Gln Phe Val Leu Val Asn Asp Gly Gly Thr Gly
   1               5                   10                  15
   Asn Val Thr Val Ala Pro Ser Asn Phe Ala Asn Gly Val Ala Glu Trp
               20                  25                  30
   Ile Ser Ser Asn Ser Arg Ser Gln Ala Tyr Lys Val Thr Cys Ser Val
           35                  40                  45
   Arg Gln Ser Ser Ala Gln Asn Arg Lys Tyr Thr Ile Lys Val Glu Val
       50                  55                  60
   Pro Lys Val Ala Thr Gln Thr Val Gly Gly Val Glu Leu Pro Val Ala
   65                  70                  75                  80
   Ala Trp Arg Ser Tyr Leu Asn Leu Glu Leu Thr Ile Pro Ile Phe Ala
                   85                  90                  95
   Thr Asn Ser Asp Cys Glu Leu Ile Val Lys Ala Met Gln Gly Leu Leu
               100                 105                 110
   Lys Asp Gly Asn Pro Ile Pro Ser Ala Ile Ala Ala Asn Ser Gly Ile
           115                 120                 125
   Tyr
<210> 16
   <211> 128
   <212> PRT
   <213> Bacteriophage PP7
<400> 16
   Met Ser Lys Thr Ile Val Leu Ser Val Gly Glu Ala Thr Arg Thr Leu
   1               5                   10                  15
   Thr Glu Ile Gln Ser Thr Ala Asp Arg Gln Ile Phe Glu Glu Lys Val
               20                  25                  30
   Gly Pro Leu Val Gly Arg Leu Arg Leu Thr Ala Ser Leu Arg Gln Asn
           35                  40                  45
   Gly Ala Lys Thr Ala Tyr Arg Val Asn Leu Lys Leu Asp Gln Ala Asp
       50                  55                  60
   Val Val Asp Cys Ser Thr Ser Val Cys Gly Glu Leu Pro Lys Val Arg
   65                  70                  75                  80
   Tyr Thr Gln Val Trp Ser His Asp Val Thr Ile Val Ala Asn Ser Thr
                   85                  90                  95
   Glu Ala Ser Arg Lys Ser Leu Tyr Asp Leu Thr Lys Ser Leu Val Ala
               100                 105                 110
   Thr Ser Gln Val Glu Asp Leu Val Val Asn Leu Val Pro Leu Gly Arg
           115                 120                 125
<210> 17
   <211> 132
   <212> PRT
   <213> Artificial sequence
<220>
   <223> Bacteriophage Qbeta 240 mutant
<400> 17
   Ala Lys Leu Glu Thr Val Thr Leu Gly Asn Ile Gly Arg Asp Gly Lys
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 18
   <211> 132
   <212> PRT
   <213> Artificial sequence
<220>
   <223> Bacteriophage Q-beta 243 mutant
<400> 18
   Ala Lys Leu Glu Thr Val Thr Leu Gly Lys Ile Gly Lys Asp Gly Lys
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 19
   <211> 132
   <212> PRT
   <213> Artificial Sequence
<220>
   <223> Bacteriophage Q-beta 250 mutant
<400> 19
   Ala Arg Leu Glu Thr Val Thr Leu Gly Asn Ile Gly Arg Asp Gly Lys
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65                  70                  75                  80
   Asp Pro Ser Val Thr Arg Gln Lys Tyr Ala Asp Val Thr Phe Ser Phe
                   85                  90                  95
   Thr Gln Tyr Ser Thr Asp Glu Glu Arg Ala Phe Val Arg Thr Glu Leu
               100                 105                 110
   Ala Ala Leu Leu Ala Ser Pro Leu Leu Ile Asp Ala Ile Asp Gln Leu
           115                 120                 125
   Asn Pro Ala Tyr
       130
<210> 20
   <211> 132
   <212> PRT
   <213> Artificial sequence
<220>
   <223> Bacteriophage Q-beta 251 mutant
<400> 20
   Ala Lys Leu Glu Thr Val Thr Leu Gly Asn Ile Gly Lys Asp Gly Arg
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 21
   <211> 132
   <212> PRT
   <213> Artificial sequence
<220>
   <223> Bacteriophage Q-beta 259 mutant
<400> 21
   Ala Arg Leu Glu Thr Val Thr Leu Gly Asn Ile Gly Lys Asp Gly Arg
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65                  70                  75                  80
   Asp Pro Ser Val Thr Arg Gln Lys Tyr Ala Asp Val Thr Phe Ser Phe
                   85                  90                  95
   Thr Gln Tyr Ser Thr Asp Glu Glu Arg Ala Phe Val Arg Thr Glu Leu
               100                 105                 110
   Ala Ala Leu Leu Ala Ser Pro Leu Leu Ile Asp Ala Ile Asp Gln Leu
           115                 120                 125
   Asn Pro Ala Tyr
       130
<210> 22
   <211> 185
   <212> PRT
   <213> Hepatitis B virus
<400> 22
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys
               180                 185
<210> 23
   <211> 212
   <212> PRT
   <213> Hepatitis B virus
<400> 23
   Met Gln Leu Phe His Leu Cys Leu Ile Ile Ser Cys Ser Cys Pro Thr
   1               5                   10                  15
   Val Gln Ala Ser Lys Leu Cys Leu Gly Trp Leu Trp Gly Met Asp Ile
   20 25 30
   Asp Pro Tyr Lys Glu Phe Gly Ala Thr Val Glu Leu Leu Ser Phe Leu
   35 40 45
   Pro Ser Asp Phe Phe Pro Ser Val Arg Asp Leu Leu Asp Thr Ala Ser
   50 55 60
   Ala Leu Tyr Arg Glu Ala Leu Glu Ser Pro Glu His Cys Ser Pro His
   65 70 75 80
   His Thr Ala Leu Arg Gln Ala Ile Leu Cys Trp Gly Asp Leu Met Asn
   85 90 95
   Leu Ala Thr Trp Val Gly Gly Asn Leu Glu Asp Pro Val Ser Arg Asp
   100 105 110
   Leu Val Val Gly Tyr Val Asn Thr Thr Val Gly Leu Lys Phe Arg Gln
   115 120 125
   Leu Leu Trp Phe His Ile Ser Cys Leu Thr Phe Gly Arg Glu Thr Val
   130 135 140
   Ile Glu Tyr Leu Val Ser Phe Gly Val Trp Ile Arg Thr Pro Pro Ala
   145 150 155 160
   Tyr Arg Pro Pro Asn Ala Pro Ile Leu Ser Thr Leu Pro Glu Thr Thr
   165 170 175
   Val Val Arg Arg Arg Gly Arg Ser Pro Arg Arg Arg Thr Pro Ser Pro
   180 185 190
   Arg Arg Arg Arg Ser Gln Ser Pro Arg Arg Arg Arg Ser Gln Ser Arg
   195 200 205
   Glu Ser Gln Cys
   210
<210> 24
   <211> 188
   <212> PRT
   <213> Hepatitis B virus
<400> 24
   Met Asp Ile Asp Pro Tyr Lys Glu Phe Gly Ser Ser Tyr Gln Leu Leu
   1 5 10 15
   Asn Phe Leu Pro Leu Asp Phe Phe Pro Asp Leu Asn Ala Leu Val Asp
   20 25 30
   Thr Ala Thr Ala Leu Tyr Glu Glu Glu Leu Thr Gly Arg Glu His Cys
   35 40 45
   Ser Pro His His Thr Ala Ile Arg Gln Ala Leu Val Cys Trp Asp Glu
   50 55 60
   Leu Thr Lys Leu Ile Ala Trp Met Ser Ser Asn Ile Thr Ser Glu Gln
   65 70 75 80
   Val Arg Thr Ile Ile Val Asn His Val Asn Asp Thr Trp Gly Leu Lys
   85 90 95
   Val Arg Gln Ser Leu Trp Phe His Leu Ser Cys Leu Thr Phe Gly Gln
   100 105 110
   His Thr Val Gln Glu Phe Leu Val Ser Phe Gly Val Trp Ile Arg Thr
   115 120 125
   Pro Ala Pro Tyr Arg Pro Pro Asn Ala Pro Ile Leu Ser Thr Leu Pro
   130 135 140
   Glu His Thr Val Ile Arg Arg Arg Gly Gly Ala Arg Ala Ser Arg Ser
   145 150 155 160
   Pro Arg Arg Arg Thr Pro Ser Pro Arg Arg Arg Arg Ser Gln Ser Pro
   165 170 175
   Arg Arg Arg Arg Ser Gln Ser Pro Ser Thr Asn Cys
   180 185
<210> 25
   <211> 185
   <212> PRT
   <213> Hepatitis B virus
<400> 25
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys
               180                 185
<210> 26
   <211> 152
   <212> PRT
   <213> Hepatitis B virus
<400> 26
   Met Asp Ile Asp Pro Tyr Lys Glu Phe Gly Ala Thr Val Glu Leu Leu
   1 5 10 15
   Ser Phe Leu Pro Ser Asp Phe Phe Pro Ser Val Arg Asp Leu Leu Asp
   20 25 30
   Thr Ala Ala Ala Leu Tyr Arg Asp Ala Leu Glu Ser Pro Glu His Cys
   35 40 45
   Ser Pro His His Thr Ala Leu Arg Gln Ala Ile Leu Cys Trp Gly Asp
   50 55 60
   Leu Met Thr Leu Ala Thr Trp Val Gly Thr Asn Leu Glu Asp Gly Gly
   65 70 75 80
   Lys Gly Gly Ser Arg Asp Leu Val Val Ser Tyr Val Asn Thr Asn Val
   85 90 95
   Gly Leu Lys Phe Arg Gln Leu Leu Trp Phe His Ile Ser Cys Leu Thr
   100 105 110
   Phe Gly Arg Glu Thr Val Leu Glu Tyr Leu Val Ser Phe Gly Val Trp
   115 120 125
   Ile Arg Thr Pro Pro Ala Tyr Arg Pro Pro Asn Ala Pro Ile Leu Ser
   130 135 140
   Thr Leu Pro Glu Thr Thr Val Val
   145 150
<210> 27
   <211> 3635
   <212> DNA
   <213> Artificial sequence
<220>
   <223> plasmid pAP283-58
<400> 27
   cgagctcgcc cctggcttat cgaaattaat acgactcact atagggagac cggaattcga 60
   gctcgcccgg ggatcctcta gaattttctg cgcacccatc ccgggtggcg cccaaagtga 120
   ggaaaatcac atggcaaata agccaatgca accgatcaca tctacagcaa ataaaattgt 180
   gtggtcggat ccaactcgtt tatcaactac attttcagca agtctgttac gccaacgtgt 240
   taaagttggt atagccgaac tgaataatgt ttcaggtcaa tatgtatctg tttataagcg 300
   tcctgcacct aaaccggaag gttgtgcaga tgcctgtgtc attatgccga atgaaaacca 360
   atccattcgc acagtgattt cagggtcagc cgaaaacttg gctaccttaa aagcagaatg 420
   ggaaactcac aaacgtaacg ttgacacact cttcgcgagc ggcaacgccg gtttgggttt 480
   ccttgaccct actgcggcta tcgtatcgtc tgatactact gcttaagctt gtattctata 540
   gtgtcaccta aatcgtatgt gtatgataca taaggttatg tattaattgt agccgcgttc 600
   taacgacaat atgtacaagc ctaattgtgt agcatctggc ttactgaagc agaccctatc 660
   atctctctcg taaactgccg tcagagtcgg tttggttgga cgaaccttct gagtttctgg 720
   taacgccgtt ccgcaccccg gaaatggtca ccgaaccaat cagcagggtc atcgctagcc 780
   agatcctcta cgccggacgc atcgtggccg gcatcaccgg cgcacacagt gcggttgctg 840
   gcgcctatat cgccgacatc accgatgggg aagatcgggc tcgccacttc gggctcatga 900
   gcgcttgttt cggcgtgggt atggtggcag gccccgtggc cgggggactg ttgggcgcca 960
   tctccttgca tgcaccattc cttgcggcgg cggtgcttca acggcctcaa cctactactg 1020
   ggctgcttcc taatgcagga gtcgcataag ggagagcgtc gatatggtgc actctcagta 1080
   caatctgctc tgatgccgca tagttaagcc aactccgcta tcgctacgtg actgggtcat 1140
   ggctgcgccc cgacacccgc caacacccgc tgacgcgccc tgacgggctt gtctgctccc 1200
   ggcatccgct tacagacaag ctgtgaccgt ctccgggagc tgcatgtgtc agaggttttc 1260
   accgtcatca ccgaaacgcg cgaggcagct tgaagacgaa agggcctcgt gatacgccta 1320
   tttttatagg ttaatgtcat gataataatg gtttcttaga cgtcaggtgg cacttttcgg 1380
   ggaaatgtgc gcggaacccc tatttgttta tttttctaaa tacattcaaa tatgtatccg 1440
   ctcatgagac aataaccctg ataaatgctt caataatatt gaaaaaggaa gagtatgagt 1500
   attcaacatt tccgtgtcgc ccttattccc ttttttgcgg cattttgcct tcctgttttt 1560
   gctcacccag aaacgctggt gaaagtaaaa gatgctgaag atcagttggg tgcacgagtg 1620
   ggttacatcg aactggatct caacagcggt aagatccttg agagttttcg ccccgaagaa 1680
   cgttttccaa tgatgagcac ttttaaagtt ctgctatgtg gcgcggtatt atcccgtatt 1740
   gacgccgggc aagagcaact cggtcgccgc atacactatt ctcagaatga cttggttgag 1800
   tactcaccag tcacagaaaa gcatcttacg gatggcatga cagtaagaga attatgcagt 1860
   gctgccataa ccatgagtga taacactgcg gccaacttac ttctgacaac gatcggagga 1920
   ccgaaggagc taaccgcttt tttgcacaac atgggggatc atgtaactcg ccttgatcgt 1980
   tgggaaccgg agctgaatga agccatacca aacgacgagc gtgacaccac gatgcctgta 2040
   gcaatggcaa caacgttgcg caaactatta actggcgaac tacttactct agcttcccgg 2100
   caacaattaa tagactggat ggaggcggat aaagttgcag gaccacttct gcgctcggcc 2160
   cttccggctg gctggtttat tgctgataaa tctggagccg gtgagcgtgg gtctcgcggt 2220
   atcattgcag cactggggcc agatggtaag ccctcccgta tcgtagttat ctacacgacg 2280
   gggagtcagg caactatgga tgaacgaaat agacagatcg ctgagatagg tgcctcactg 2340
   attaagcatt ggtaactgtc agaccaagtt tactcatata tactttagat tgatttaaaa 2400
   cttcattttt aatttaaaag gatctaggtg aagatccttt ttgataatct catgaccaaa 2460
   atcccttaac gtgagttttc gttccactga gcgtcagacc ccgtagaaaa gatcaaagga 2520
   tcttcttgag atcctttttt tctgcgcgta atctgctgct tgcaaacaaa aaaaccaccg 2580
   ctaccagcgg tggtttgttt gccggatcaa gagctaccaa ctctttttcc gaaggtaact 2640
   ggcttcagca gagcgcagat accaaatact gtccttctag tgtagccgta gttaggccac 2700
   cacttcaaga actctgtagc accgcctaca tacctcgctc tgctaatcct gttaccagtg 2760
   gctgctgcca gtggcgataa gtcgtgtctt accgggttgg actcaagacg atagttaccg 2820
   gataaggcgc agcggtcggg ctgaacgggg ggttcgtgca cacagcccag cttggagcga 2880
   acgacctaca ccgaactgag atacctacag cgcgagcatt gagaaagcgc cacgcttccc 2940
   gaagggagaa aggcggacag gtatccggta agcggcaggg tcggaacagg agagcgcacg 3000
   agggagcttc cagggggaaa cgcctggtat ctttatagtc ctgtcgggtt tcgccacctc 3060
   tgacttgagc gtcgattttt gtgatgctcg tcaggggggc ggagcctatg gaaaaacgcc 3120
   agcaacgcgg cctttttacg gttcctggcc ttttgctggc cttttgctca catgttcttt 3180
   cctgcgttat cccctgattc tgtggataac cgtattaccg cctttgagtg agctgatacc 3240
   gctcgccgca gccgaacgac gagcgcagcg agtcagtgag cgaggaagcg gaagagcgcc 3300
   caatacgcaa accgcctctc cccgcgcgtt ggccgattca ttaatgcagc tgtggtgtca 3360
   tggtcggtga tcgccagggt gccgacgcgc atctcgactg catggtgcac caatgcttct 3420
   ggcgtcaggc agccatcgga agctgtggta tggccgtgca ggtcgtaaat cactgcataa 3480
   ttcgtgtcgc tcaaggcgca ctcccgttct ggataatgtt ttttgcgccg acatcataac 3540
   ggttctggca aatattctga aatgagctgt tgacaattaa tcatcgaact agttaactag 3600
   tacgcaagtt cacgtaaaaa gggtatcgcg gaatt 3635
<210> 28
   <211> 131
   <212> PRT
   <213> Bacteriophage AP205
<400> 28
   Met Ala Asn Lys Pro Met Gln Pro Ile Thr Ser Thr Ala Asn Lys Ile
   1 5 10 15
   Val Trp Ser Asp Pro Thr Arg Leu Ser Thr Thr Phe Ser Ala Ser Leu
   20 25 30
   Leu Arg Gln Arg Val Lys Val Gly Ile Ala Glu Leu Asn Asn Val Ser
   35 40 45
   Gly Gln Tyr Val Ser Val Tyr Lys Arg Pro Ala Pro Lys Pro Glu Gly
   50 55 60
   Cys Ala Asp Ala Cys Val Ile Met Pro Asn Glu Asn Gln Ser Ile Arg
   65 70 75 80
   Thr Val Ile Ser Gly Ser Ala Glu Asn Leu Ala Thr Leu Lys Ala Glu
   85 90 95
   Trp Glu Thr His Lys Arg Asn Val Asp Thr Leu Phe Ala Ser Gly Asn
   100 105 110
   Ala Gly Leu Gly Phe Leu Asp Pro Thr Ala Ala Ile Val Ser Ser Asp
   115 120 125
   Thr Thr Ala
   130
<210> 29
   <211> 131
   <212> PRT
   <213> Artificial Sequence
<220>
   <223> AP205 coat protein
<400> 29
   Met Ala Asn Lys Thr Met Gln Pro Ile Thr Ser Thr Ala Asn Lys Ile
   1 5 10 15
   Val Trp Ser Asp Pro Thr Arg Leu Ser Thr Thr Phe Ser Ala Ser Leu
   20 25 30
   Leu Arg Gln Arg Val Lys Val Gly Ile Ala Glu Leu Asn Asn Val Ser
   35 40 45
   Gly Gln Tyr Val Ser Val Tyr Lys Arg Pro Ala Pro Lys Pro Glu Gly
   50 55 60
   Cys Ala Asp Ala Cys Val Ile Met Pro Asn Glu Asn Gln Ser Ile Arg
   65 70 75 80
   Thr Val Ile Ser Gly Ser Ala Glu Asn Leu Ala Thr Leu Lys Ala Glu
   85 90 95
   Trp Glu Thr His Lys Arg Asn Val Asp Thr Leu Phe Ala Ser Gly Asn
   100 105 110
   Ala Gly Leu Gly Phe Leu Asp Pro Thr Ala Ala Ile Val Ser Ser Asp
   115 120 125
   Thr Thr Ala
   130
<210> 30
   <211> 3607
   <212> DNA
   <213> Artificial Sequence
<220>
   <223> plasmid pAP281-32
<400> 30
   cgagctcgcc cctggcttat cgaaattaat acgactcact atagggagac cggaattcga     60
   gctcgcccgg ggatcctcta gattaaccca acgcgtagga gtcaggccat ggcaaataag    120
   acaatgcaac cgatcacatc tacagcaaat aaaattgtgt ggtcggatcc aactcgttta    180
   tcaactacat tttcagcaag tctgttacgc caacgtgtta aagttggtat agccgaactg    240
   aataatgttt caggtcaata tgtatctgtt tataagcgtc ctgcacctaa accgaaggtc    300
   agatgcctgt gtcattatgc cgaatgaaaa ccaatccatt cgcacagtga tttcagggtc    360
   agccgaaaac ttggctacct taaaagcaga atgggaaact cacaaacgta acgttgacac    420
   actcttcgcg agcggcaacg ccggtttggg tttccttgac cctactgcgg ctatcgtatc    480
   gtctgatact actgcttaag cttgtattct atagtgtcac ctaaatcgta tgtgtatgat    540
   acataaggtt atgtattaat ggtagccgcg ttctaacgac aatatgtaca agcctaattg    600
   tgtagcatct ggcttactga agcagaccct atcatctctc tcgtaaactg ccgtcagagt    660
   cggttgggtt ggacagacct ctgagtttct ggtaacgccg ttccgcaccc cggaaatggt    720
   caccgaacca ttcagcaggg tcatcgctag ccagatcctc tacgccggac gcatcgtggc    780
   ccgcatcacc ggcgccacag gtgcggtgct ggcgcctata tcgccgacat caccgatggg    840
   gaagatcggg ctcgccactt cgggctcatg atcgctggtt tccgcctggg tatggtggca    900
   ggccccgtgg cccgggggac tgttgggcgc catctccttg catgcaccat tccttgcggc    960
   ggcggtgctc aacggcctca acctactact gggctgcttc ctaatgcagg agtcgcataa   1020
   gggagagcgt cgatatggtg cactctcagt acaatctgct ctgatgccgc atagttaagc   1080
   caactccgct atcgctacgt gactgggtca tggctgcgcc ccgacacccg ccaacacccg   1140
   ctgacgcgcc ctgacgggct tgtctgcttc cggcatccgc ttacagacaa gctgtgaccg 1200
   tctccgggag ctgcatgtgt cagaggtttt caccgtcatc accgaaacgc gcgaggcagc 1260
   ttgaagacga aagggcctcg tgatacgcct atttttatag gttaatgtca tgataataat 1320
   ggtttcttag acgtcaggtg gcacttttcg gggaaatgtg cgcggacccc ctattggttt 1380
   atttttctaa atacattcaa atatgtatcc gctcatgaga caataaccct gataaatgct 1440
   tcaataatat tgaaaaagga agagtatgag tattcaacat ttccgtgtcg cccttattcc 1500
   cttttttgcg gcattttgcc ttcctgtttt tgctcaccca gaaacgctgg tgaaagtaaa 1560
   agatgctgaa gatcagttgg gtgcacgagt gggttacatc gaactggatc tcaacagcgg 1620
   taagatcctt gagagttttc gccccgaaga acgttttcca atgatgagca cttttaaagt 1680
   tctgctatgt ggcgcggtat tatcccgtat tgacgccggg caagagcaac tcggtcgccg 1740
   catacactat tctcagaatg acttggttga gtactcacca gtcacagaaa agcatcttac 1800
   atggcatgac agtaagagaa ttatgcagtg ctgccataac catgagtgat aacactgcgg 1860
   ccaacttact tctgacaacg atcggaggac cgaaggagct aaccgctttt ttgcacaaca 1920
   tgggggatca tgtaactcgc cttgatcgtt gggaaccgga gctgaatgaa gccataccaa 1980
   acgacgagcg tgacaccacg atgcctgtag caatggcaac aacgttgcgc aaactattaa 2040
   ctggcgaact acttactcta gcttcccggc aacaattaat agactggatg gaggcggata 2100
   aagttgcagg accacttctg cgctcggccc ttccggctgg ctggtttatt gctgataaat 2160
   ctggagccgg tgagcgtggg tctcgcggta tcattgcagc actggggcca gatggtaagc 2220
   cctcccgtat cgtagttatc tacacgacgg ggagtcaggc aactatggat gaacgaaata 2280
   gacagatcgc tgagataggt gcctcactga ttaagcattg gtaactgtca gaccaagttt 2340
   actcatatat actttagatt gatttaaaac ttcattttta atttaaaagg atctaggtga 2400
   agatcctttt tgataatctc atgaccaaaa tcccttaacg tgagttttcg ttccactgag 2460
   cgtcagaccc cgtagaaaag atcaaaggat cttcttgaga tccttttttt ctgcgcgtaa 2520
   tctgctgctt gcaaacaaaa aaaccaccgc taccagcggt ggtttgtttg ccggatcaag 2580
   agctaccaac tctttttccg aaggtaactg gcttcagcag agcgcagata ccaaatactg 2640
   tccttctagt gtagccgtag ttaggccacc acttcaagaa ctctgtagca ccgcctacat 2700
   acctcgctct gctaatcctg ttaccagtgg ctgctgccag tggcgataag tcgtgtctta 2760
   ccgggttgga ctcaagacga tagttaccgg ataaggcgca gcggtcgggc tgaacggggg 2820
   gttcgtgcac acagcccagc ttggagcgaa cgacctacac cgaactgaga tacctacagc 2880
   gtgagcattg agaaagcgcc acgcttcccg aagggagaaa ggcggacagg tatccggtaa 2940
   gcggcagggt cggaacagga gagcgcacga gggagcttcc agggggaaac gcctggtatc 3000
   tttatagtcc tgtcgggttt cgccacctct gacttgagcg tcgatttttg tgatgctcgt 3060
   caggggggcg gagcctatgg aaaaacgcca gcaacgcggc ctttttacgg ttcctggcct 3120
   tttgctggcc ttttgctcac atgttctttc ctgcgttatc ccctgattct gtggataacc 3180
   gtattaccgc ctttgagtga gctgataccg ctcgccgcag ccgaacgacc gacggcgcag 3240
   cgagtcagtg agcgaggaag cggaagagcg cccaatacgc aaaccgcctc tccccgcgcg 3300
   ttggccgatt cattaatgca gctgtggtgt catggtcggt gatcgccagg gtgccgacgc 3360
   gcatctcgac tgcatggtgc accaatgctt ctggcgtcag gcagccatcg gaagctgtgg 3420
   tatggccgtg caggtcgtaa atcactgcat aattcgtgtc gctcaaggcg cactcccgtt 3480
   ctggataatg ttttttgcgg cgacatcata acggttctgg caaatattct gaaatgagct 3540
   ggtgacaatt aatcatcgaa ctagttaact agtacgcaag ttcacgtaaa aagggtatcg 3600
   cggaatt 3607
<210> 31
   <211> 28
   <212> PRT
   <213> Homo sapiens
<400> 31
   Gly Ser Ser Phe Leu Ser Pro Glu His Gln Arg Val Gln Gln Arg Lys
   1 5 10 15
   Glu Ser Lys Lys Pro Pro Ala Lys Leu Gln Pro Arg
   20 25
<210> 32
   <211> 28
   <212> PRT
   <213> Mus musculus
<400> 32
   Gly Ser Ser Phe Leu Ser Pro Glu His Gln Lys Ala Gln Gln Arg Lys
   1 5 10 15
   Glu Ser Lys Lys Pro Pro Ala Lys Leu Gln Pro Arg
   20 25
<210> 33
   <211> 5
   <212> PRT
   <213> Artificial Sequence
<220> .
   <223> Linker
<400> 33
   Gly Gly Lys Gly Gly
   1 5
<210> 34
   <211> 3
   <212> PRT
   <213> Artificial Sequence
<220>
   <223> N-terminal glycine linker
<220>
   <221> REPEAT
   <222> (1)..(1)
   <223> Glycine can be repeated from zero to five times
<220>
   <221> REPEAT
   <222> (3)..(3)
   <223> Glycine can be repeated from zero to twelve times
<400> 34
   Gly Cys Gly
   1
<210> 35
   <211> 9
   <212> PRT
   <213> Artificial sequence
<220>
   <223> N terminal glycine serine linkers
<220>
   <221> REPEAT
   <222> (1)..(1)
   <223> Glycine can be repeated from zero to five times
<220>
   <221> REPEAT
   <222> (3)..(3)
   <223> Glycine can be repeated from zero to ten times
<220>
   <221> REPEAT
   <222> (4)..(4)
   <223> serine can be repeated from zero to two times
<220>
   <221> REPEAT
   <222> (5)..(9)
   <223> These residues can be repeated from zero to three times as a group
<400> 35
   Gly Cys Gly Ser Gly Gly Gly Gly Ser
   1               5
<210> 36
   <211> 3
   <212> PRT
   <213> Artificial Sequence
<220>
   <223> C-terminal glycine linker
<220>
   <221> REPEAT
   <222> (1)..(1)
   <223> Glycine can be repeated from zero to twelve times
<220>
   <221> REPEAT
   <222> (3)..(3)
   <223> Glycine can be repeated from zero to five times
<400> 36
   Gly Cys Gly
   1
<210> 37
   <211> 10
   <212> PRT
   <213> Artificial Sequence
<220>
   <223> C terminal glycine serine linkers
<220>
   <221> REPEAT
   <222> (1)..(1)
   <223> Glycine can be repeated from zero to ten times
<220>
   <221> REPEAT
   <222> (2)..(2)
   <223> serine can be repeated from zero to two times
<220>
   <221> REPEAT
   <222> (3)..(7)
   <223> These residues can be repeated from zero to three times as a group
<220>
   <221> REPEAT
   <222> (8)..(8)
   <223> Glycine can be repeated from zero to eight times
<220>
   <221> REPEAT
   <222> (10)..(10)
   <223> Glycine can be repeated from zero to five times
<400> 37
   Gly Ser Gly Gly Gly Gly Ser Gly Cys Gly
   1 5 10
<210> 38
   <211> 5
   <212> PRT
   <213> Artificial Sequence
<220>
   <223> Glycine serine linker
<220>
   <221> REPEAT
   <222> (1)..(5)
   <223> These residues can be repeated any times as a group
<400> 38
   Gly Gly Gly Gly Ser
   1 5
<210> 39
   <211> 10
   <212> PRT
   <213> Artificial Sequence
<220>
   <223> N-terminal gamma1
<400> 39
   Cys Gly Asp Lys Thr His Thr Ser Pro Pro
   1 5 10
<210> 40
   <211> 10
   <212> PRT
   <213> Artificial sequence
<220>
   <223> C-terminal gamma 1
<400> 40
   Asp Lys Thr His Thr Ser Pro Pro Cys Gly
   1 5 10
<210> 41
   <211> 17
   <212> PRT
   <213> Artificial Sequence
<220>
   <223> N-terminal gamma 3
<400> 41
   Cys Gly Gly Pro Lys Pro Ser Thr Pro Pro Gly Ser Ser Gly Gly Ala
   1 5 10 15
   Pro
<210> 42
   <211> 18
   <212> PRT
   <213> Artificial Sequence
<220>
   <223> C-terminal gamma 3
<400> 42
   Pro Lys Pro Ser Thr Pro Pro Gly Ser Ser Gly Gly Ala Pro Gly Gly
   1 5 10 15
   Cys Gly
<210> 43
   <211> 6
   <212> PRT
   <213> Artificial Sequence
<220>
   <223> N-terminal glycine linker
<400> 43
   Gly Cys Gly Gly Gly Gly
   1 5
<210> 44
   <211> 6
   <212> PRT
   <213> Artificial Sequence
<220>
   <223> C-terminal glycine linker
<400> 44
   Gly Gly Gly Gly Cys Gly
   1 5
<210> 45
   <211> 6
   <212> PRT
   <213> Artificial Sequence
<220>
   <223> C-terminal glycine-lysine linker
<400> 45
   Gly Gly Lys Lys Gly Cys
   1 5
<210> 46
   <211> 6
   <212> PRT
   <213> Artificial Sequence
<220>
   <223> N-terminal glycine-lysine linker
<400> 46
   Cys Gly Lys Lys Gly Gly
   1 5
<210> 47
   <211> 4
   <212> PRT
   <213> Artificial sequence
<220>
   <223> C.terminal linker
<400> 47
   Gly Gly Cys Gly
   1
<210> 48
   <211> 27
   <212> PRT
   <213> Artificial Sequence
<220>
   <223> Grehlin precursor mutant
<400> 48
   Gly Ser Ser Phe Leu Ser Pro Glu His Gln Arg Val Gln Arg Lys Glu
   1               5                   10                  15
   Ser Lys Lys Pro Pro Ala Lys Leu Gln Pro Arg
               20                  25
<210> 49
   <211> 28
   <212> PRT
   <213> Canis familiaris
<400> 49
   Gly Ser Ser Phe Leu Ser Pro Glu His Gln Lys Leu Gln Gln Arg Lys
   1               5                   10                  15
   Glu Ser Lys Lys Pro Pro Ala Lys Leu Gln Pro Arg
               20                  25
<210> 50
   <211> 27
   <212> PRT
   <213> Artificial Sequence
<220>
   <223> Canis familiaris ghrelin mutant
<400> 50
   Gly Ser Ser Phe Leu Ser Pro Glu His Gln Lys Leu Gln Arg Lys Glu
   1               5                   10                  15
   Ser Lys Lys Pro Pro Ala Lys Leu Gln Pro Arg
               20                  25
<210> 51
   <211> 27
   <212> PRT
   <213> Artificial sequence
<220>
   <223> Mus musculus ghrelin mutant
<400> 51
   Gly Ser Ser Phe Leu Ser Pro Glu His Gln Lys Ala Gln Arg Lys Glu
   1               5                   10                  15
   Ser Lys Lys Pro Pro Ala Lys Leu Gln Pro Arg
<210> 52
   <211> 10
   <212> PRT
   <213> Homo sapiens
<400> 52
   Lys Lys Pro Pro Ala Lys Leu Gln Pro Arg
   1 5 10
<210> 53
   <211> 8
   <212> PRT
   <213> Homo sapiens
<400> 53
   Pro Pro Ala Lys Leu Gln Pro Arg
   1 5
<210> 54
   <211> 6
   <212> PRT
   <213> Homo sapiens
<400> 54
   Ala Lys Leu Gln Pro Arg
   1 5
<210> 55
   <211> 10
   <212> PRT
   <213> Homo sapiens
<400> 55
   Gly Ser Ser Phe Leu Ser Pro Glu His Gln
   1 5 10
<210> 56
   <211> 10
   <212> PRT
   <213> Homo sapiens
<400> 56
   Glu His Gln Arg Val Gln Gln Arg Lys Glu
   1 5 10
<210> 57
   <211> 57
   <212> DNA
   <213> Artificial sequence
<220>
   <223> AP205 ribosomal binding site
<400> 57
   tctagaattt tctgcgcacc catcccgggt ggcgcccaaa gtgaggaaaa tcacatg 57
<210> 58
   <211> 35
   <212> DNA
   <213> Artificial Sequence
<220>
   <223> Shine-Dalgarno sequence of vector pQb185
<400> 58
   tctagattaa cccaacgcgt aggagtcagg ccatg 35
<210> 59
   <211> 5
   <212> PRT
   <213> Homo sapiens
<400> 59
   Lys Leu Gln Pro Arg
   1 5
<210> 60
   <211> 13
   <212> PRT
   <213> Homo sapiens
<400> 60
   Gly Ser Ser Phe Leu Ser Pro Glu His Gln Arg Val Gln
   1 5 10
<210> 61
   <211> 15
   <212> PRT
   <213> Homo sapiens
<400> 61
   Gln Arg Lys Glu Ser Lys Lys Pro Pro Ala Lys Leu Gln Pro Arg
   1 5 10 15
<210> 62
   <211> 13
   <212> PRT
   <213> Canis familiaris
<400> 62
   Gly Ser Ser Phe Leu Ser Pro Glu His Gln Lys Leu Gln
   1 5 10
<210> 63
   <211> 15
   <212> PRT
   <213> Canis familiaris
<400> 63
   Gln Arg Lys Glu Ser Lys Lys Pro Pro Ala Lys Leu Gln Pro Arg
   1 5 10 15
<210> 64
   <211> 28
   <212> PRT
   <213> Artificial sequence
<220>
   <223> human ghrelin peptide mutant
<400> 64
   Cys Gly Ser Ser Phe Leu Ser Pro Glu His Gln Arg Val Gln Arg Lys
   1 5 10 15
   Glu Ser Lys Lys Pro Pro Ala Lys Leu Gln Pro Arg
   20 25
<210> 65
   <211> 29
   <212> PRT
   <213> Artificial Sequence
<220>
   <223> human ghrelin peptide 24-51 mutant
<400> 65
   Cys Gly Ser Ser Phe Leu Ser Pro Glu His Gln Arg Val Gln Gln Arg
   1 5 10 15
   Lys Glu Ser Lys Lys Pro Pro Ala Lys Leu Gln Pro Arg
   20 25
<210> 66
   <211> 28
   <212> PRT
   <213> Artificial sequence
<220>
   <223> human ghrelin peptide mutant
<400> 66
   Gly Ser Ser Phe Leu Ser Pro Glu His Gln Arg Val Gln Arg Lys Glu
   1 5 10 15
   Ser Lys Lys Pro Pro Ala Lys Leu Gln Pro Arg Cys
   20 25
<210> 67
   <211> 29
   <212> PRT
   <213> Artificial Sequence
<220>
   <223> human ghrelin peptide 24-51 mutant
<400> 67
   Gly Ser Ser Phe Leu Ser Pro Glu His Gln Arg Val Gln Gln Arg Lys
   1 5 10 15
   Glu Ser Lys Lys Pro Pro Ala Lys Leu Gln Pro Arg Cys
   20 25
<210> 68
   <211> 14
   <212> PRT
   <213> Artificial Sequence
<220>
   <223> human ghrelin peptide 24-36 mutant
<400> 68
   Gly Ser Ser Phe Leu Ser Pro Glu His Gln Arg Val Gln Cys
   1 5 10
<210> 69
   <211> 16
   <212> PRT
   <213> Artificial Sequence
<220>
   <223> human ghrelin peptide 37-51 mutant
<400> 69
   Cys Gln Arg Lys Glu Ser Lys Lys Pro Pro Ala Lys Leu Gln Pro Arg
   1 5 10 15
<210> 70
   <211> 9
   <212> PRT
   <213> Artificial sequence
<220>
   <223> human ghrelin peptide 44-51 mutant
<400> 70
   Cys Pro Pro Ala Lys Leu Gln Pro Arg
   1 5
<210> 71
   <211> 29
   <212> PRT
   <213> Artificial sequence
<220>
   <223> dog ghrelin peptide 24-51 mutant
<400> 71
   Cys Gly Ser Ser Phe Leu Ser Pro Glu His Gln Lys Leu Gln Gln Arg
   1 5 10 15
   Lys Glu Ser Lys Lys Pro Pro Ala Lys Leu Gln Pro Arg
   20 25
<210> 72
   <211> 28
   <212> PRT
   <233> Artificial sequence
<220>
   <223> dog ghrelin peptide mutant
<400> 72
   Cys Gly Ser Ser Phe Leu Ser Pro Glu His Gln Lys Leu Gln Arg Lys
   1 5 10 15
   Glu Ser Lys Lys Pro Pro Ala Lys Leu Gln Pro Arg
   20 25
<210> 73
   <211> 29
   <212> PRT
   <213> Artificial Sequence
<220>
   <223> dog ghrelin peptide24-51 mutant
<400> 73
   Gly Ser Ser Phe Leu Ser Pro Glu His Gln Lys Leu Gln Gln Arg Lys
   1               5                   10                  15
   Glu Ser Lys Lys Pro Pro Ala Lys Leu Gln Pro Arg Cys
               20                  25
<210> 74
   <211> 28
   <212> PRT
   <213> Artificial sequence
<220>
   <223> dog ghrelin mutant
<400> 74
   Gly Ser Ser Phe Leu Ser Pro Glu His Gln Lys Leu Gln Arg Lys Glu
   1               5                   10                  15
   Ser Lys Lys Pro Pro Ala Lys Leu Gln Pro Arg Cys
               20                  25
<210> 75
   <211> 14
   <212> PRT
   <213> Artificial Sequence
<220>
   <223> dog ghrelin peptide 24-36 mutant
<400> 75
   Gly Ser Ser Phe Leu Ser Pro Glu His Gln Lys Leu Gln Cys
   1               5                   10
<210> 76
   <211> 11
   <212> PRT
   <213> Artificial Sequence
<220>
   <223> human ghrelin peptide 31-40 mutant
<400> 76
   Cys Glu His Gln Arg Val Gln Gln Arg Lys Glu
   1               5                   10
<210> 77
   <211> 29
   <212> PRT
   <213> Artificial sequence
<220>
   <223> mouse ghrelin peptide 24-51 mutant
<400> 77
   Cys Gly Ser Ser Phe Leu Ser Pro Glu His Gln Lys Ala Gln Gln Arg
   1               5                   10                  15
   Lys Glu Ser Lys Lys Pro Pro Ala Lys Leu Gln Pro Arg
               20                  25
<210> 78
   <211> 37
   <212> DNA
   <213> Artificial Sequence
<220>
   <223> oligonucleotide primer
<400> 78
   ggtaacatcg gtcgagatgg aaaacaaact ctggtcc 37
<210> 79
   <211> 37
   <212> DNA
   <213> Artificial Sequence
<220>
   <223> oligonucleotide primer
<400> 79
   ggaccagagt ttgttttcca tctcgaccga tgttacc 37
<210> 80
   <211> 22
   <212> DNA
   <213> Artificial Sequence
<220>
   <223> oligonucleotide primer
<400> 80
   agctcgcccg gggatcctct ag 22
<210> 81
   <211> 40
   <212> DNA
   <213> Artificial Sequence
<220>
   <223> oligonucleotide primer
<400> 81
   cgatgcattt catccttagt tatcaatacg ctgggttcag 40
<210> 82
   <211> 36
   <212> DNA
   <213> Artificial Sequence
<220>
   <223> oligonucleotide primer
<400> 82
   ggcaaaatta gagactgtta ctttaggtaa gatcgg 36
<210> 83
   <211> 36
   <212> DNA
   <213> Artificial sequence
<220>
   <223> oligonucleotide primer
<400> 83
   ccgatcttac ctaaagtaac agtctctaat tttgcc 36
<210> 84
   <211> 33
   <212> DNA
   <213> Artificial Sequence
<220>
   <223> oligonucleotide primer
<400> 84
   ggccatggca cgactcgaga ctgttacttt agg 33
<210> 85
   <211> 19
   <212> DNA
   <213> Artificial Sequence
<220>
   <223> oligonucleotide primer
<400> 85
   gatttaggtg acactatag 19
<210> 86
   <211> 37
   <212> DNA
   <213> Artificial Sequence
<220>
   <223> oligonucleotide primer
<400> 86
   gatggacgtc aaactctggt cctcaatccg cgtgggg 37
<210> 87
   <211> 37
   <212> DNA
   <213> Artificial Sequence
<220>
   <223> oligonucleotide primer
<400> 87
   ccccacgcgg attgaggacc agagtttgac gtccatc 37
<210> 88
   <211> 31
   <212> DNA
   <213> Artificial sequence
<220>
   <223> EcoRIHBcAg(s) primer
<400> 88
   ccggaattca tggacattga cccttataaa g 31
<210> 89
   <211> 51
   <212> DNA
   <213> Artificial Sequence
<220>
   <223> Lys-HBcAg(as) primer
<400> 89
   cctagagcca cctttgccac catcttctaa attagtaccc acccaggtag c 51
<210> 90
   <211> 48
   <212> DNA
   <213> Artificial sequence
<220>
   <223> Lys-HBcAg(s) primer
<400> 90
   gaagatggtg gcaaaggtgg ctctagggac ctagtagtca gttatgtc 48
<210> 91
   <211> 38
   <212> DNA
   <213> Artificial sequence
<220>
   <223> HBcAg(1-149)Hind(as) primer
<400> 91
   cgcgtcccaa gcttctaaac aacagtagtc tccggaag 38
<210> 92
   <211> 37
   <212> DNA
   <213> Artificial sequence
<220>
   <223> 48as primer
<400> 92
   gtgcagtatg gtgaggtgag gaatgctcag gagactc 37
<210> 93
   <211> 37
   <212> DNA
   <213> Artificial sequence
<220>
   <223> 48s primer
<400> 93
   gagtctcctg agcattcctc acctcaccat actgcac 37
<210> 94
   <211> 33
   <212> DNA
   <213> Artificial sequence
<220>
   <223> 107as primer
<400> 94
   cttccaaaag tgagggaaga aatgtgaaac cac 33
<210> 95
   <211> 47
   <212> DNA
   <213> Artificial Sequence
<220>
   <223> HBcAg149hind-as
<400> 95
   cgcgtcccaa gcttctaaac aacagtagtc tccggaagcg ttgatag 47
<210> 96
   <211> 33
   <212> DNA
   <213> Artificial Sequence
<220>
   <223> 107s primer
<400> 96
   gtggtttcac atttcttccc tcacttttgg aag 33
<210> 97
   <211> 38
   <212> DNA
   <213> Artificial Sequence
<220>
   <223> HBcAgwtHindIIII primer
<400> 97
   cgcgtcccaa gcttctaaca ttgagattcc cgagattg 38
<210> 98
   <211> 10
   <212> PRT
   <213> Artificial Sequence
<220>
   <223> epitope CeH3
<400> 98
   Val Asn Leu Thr Trp Ser Arg Ala Ser Gly
   1               5                   10
<210> 99
   <211> 51
   <212> DNA
   <213> Artificial sequence
<220>
   <223> CeH3fwd primer
<220>
   <221> CDS
   <222> (1)..(51)
<400> 99
   gtt aac ttg acc tgg tct cgt gct tct ggt gca tcc agg gat cta gta      48
   Val Asn Leu Thr Trp Ser Arg Ala Ser Gly Ala Ser Arg Asp Leu Val
   1               5                   10                  15
   gtc                                                                  51
   Val
<210> 100
   <211> 17
   <212> PRT
   <213> Artificial Sequence
<220>
   <223> CeH3fwd primer
<400> 100
   Val Asn Leu Thr Trp Ser Arg Ala Ser Gly Ala Ser Arg Asp Leu Val
   1 5 10 15
   Val
<210> 101
   <211> 51
   <212> DNA
   <213> Artificial sequence
<220>
   <223> CeH3rev primer
<400> 101
   accagaagca cgagaccagg tcaagttaac atcttccaaa ttattaccca c 51
<210> 102
   <211> 7
   <212> PRT
   <213> Artificial sequence
<220>
   <223> CeH3rev primer peptide
<400> 102
   Asp Glu Leu Asn Asn Gly Val
   1 5
<210> 103
   <211> 31
   <212> DNA
   <213> Artificial Sequence
<220>
   <223> HBcAg-wt EcoRI fwd primer
<400> 103
   ccggaattca tggacattga cccttataaa g 31
<210> 104
   <211> 38
   <212> DNA
   <213> Artificial Sequence
<220>
   <223> HBcAg-wt Hind III rev primer
<400> 104
   cgcgtcccaa gcttctaaca ttgagattcc cgagattg 38
<210> 105
   <211> 30
   <212> PRT
   <213> Artificial sequence
<220>
   <223> GhrelC mutant
<400> 105
   Gly Ser Ser Phe Leu Ser Pro Glu His Gln Lys Ala Gln Gln Arg Lys
   1               5                   10                  15
   Glu Ser Lys Lys Pro Pro Ala Lys Leu Gln Pro Arg Gly Cys
               20                  25                  30
<210> 106
   <211> 28
   <212> PRT
   <213> Artificial sequence
<220>
   <223> cGhrQ14 mutant
<400> 106
   Cys Gly Ser Ser Phe Leu Ser Pro Glu His Gln Lys Ala Gln Arg Lys
   1               5                   10                  15
   Glu Ser Lys Lys Pro Pro Ala Lys Leu Gln Pro Arg
               20                  25
<210> 107
   <211> 29
   <212> PRT
   <213> Artificial sequence
<220>
   <223> GhrQ14C mutant
<400> 107
   Gly Ser Ser Phe Leu Ser Pro Glu His Gln Lys Ala Gln Arg Lys Glu
   1               5                   10                  15
   Ser Lys Lys Pro Pro Ala Lys Leu Gln Pro Arg Gly Cys
               20                  25
<210> 108
   <211> 11
   <212> PRT
   <213> Artificial Sequence
<220>
   <223> Ghrelin peptide mutant
<400> 108
   Cys Lys Lys Pro Pro Ala Lys Leu Gln Pro Arg
   1 5 10
<210> 109
   <211> 7
   <212> PRT
   <213> Artificial sequence
<220>
   <223> Ghrelin peptide mutant
<400> 109
   Cys Ala Lys Leu Gln Pro Arg
   1 5
<210> 110
   <211> 11
   <212> PRT
   <213> Artificial sequence
<220>
   <223> Ghrelin peptide mutant
<400> 110
   Gly Ser Ser Phe Leu Ser Pro Glu His Gln Cys
   1 5 10
<210> 111
   <211> 11
   <212> PRT
   <213> Homo sapiens
<400> 111
   Glu His Gln Arg Val Gln Gln Arg Lys Glu Ser
   1 5 10
<210> 112
   <211> 10
   <212> PRT
   <213> Mus musculus
<400> 112
   Glu His Gln Lys Ala Gln Gln Arg Lys Glu
   1 5 10
<210> 113
   <211> 11
   <212> PRT
   <213> Mus musculus
<400> 113
   Glu His Gln Lys Ala Gln Gln Arg Lys Glu Ser
   1 5 10
<210> 114
   <211> 10
   <212> PRT
   <213> Canis familiaris
<400> 114
   Glu His Gln Lys Leu Gln Gln Arg Lys Glu
   1 5 10
<210> 115
   <211> 11
   <212> PRT
   <213> Canis familiaris
<400> 115
   Glu His Gln Lys Leu Gln Gln Arg Lys Glu Ser
   1 5 10
<210> 116
   <211> 10
   <212> PRT
   <213> Homo sapiens
<400> 116
   Leu Ser Pro Glu His Gln Arg Val Gln Gln
   1 5 10
<210> 117
   <211> 10
   <212> PRT
   <213> Mus musculus
<400> 117
   Leu Ser Pro Glu His Gln Lys Ala Gln Gln
   1 5 10
<210> 118
   <211> 10
   <212> PRT
   <213> Canis familiaris
<400> 118
   Leu Ser Pro Glu His Gln Lys Leu Gln Gln
   1 5 10
<210> 119
   <211> 7
   <212> PRT
   <213> Homo sapiens
<400> 119
   Gly Ser Ser Phe Leu Ser Pro
   1 5
<210> 120
   <211> 29
   <212> PRT
   <213> Artificial Sequence
<220>
   <223> ghrelin peptide mutant
<400> 120
   Gly Ser Ser Phe Leu Ser Pro Glu His Gln Arg Val Gln Arg Lys Glu
   1 5 10 15
   Ser Lys Lys Pro Pro Ala Lys Leu Gln Pro Arg Gly Cys
   20 25
<210> 121
   <211> 30
   <212> PRT
   <213> Artificial Sequence
<220>
   <223> ghrelin peptide mutant
<400> 121
   Gly Ser Ser Phe Leu Ser Pro Glu His Gln Arg Val Gln Gln Arg Lys
   1               5                   10                  15
   Glu Ser Lys Lys Pro Pro Ala Lys Leu Gln Pro Arg Gly Cys
               20                  25                  30
<210> 122
   <211> 15
   <212> PRT
   <213> Artificial Sequence
<220>
   <223> ghrelin peptide mutant
<400> 122
   Gly Ser Ser Phe Leu Ser Pro Glu His Gln Arg Val Gln Gly Cys
   1               5                   10                  15
<210> 123
   <211> 30
   <212> PRT
   <213> Artificial Sequence
<220>
   <223> ghrelin peptide mutant
<400> 123
   Gly Ser Ser Phe Leu Ser Pro Glu His Gln Lys Leu Gln Gln Arg Lys
   1               5                   10                  15
   Glu Ser Lys Lys Pro Pro Ala Lys Leu Gln Pro Arg Gly Cys
               20                  25                  30
<210> 124
   <211> 29
   <212> PRT
   <213> Artificial sequence
<220>
   <223> ghrelin peptide mutant
<400> 124
   Gly Ser Ser Phe Leu Ser Pro Glu His Gln Lys Leu Gln Arg Lys Glu
   1               5                   10                  15
   Ser Lys Lys Pro Pro Ala Lys Leu Gln Pro Arg Gly Cys
               20                  25
<210> 125
   <211> 15
   <212> PRT
   <213> Artificial Sequence
<220>
   <223> ghrelin peptide mutant
<400> 125
   Gly Ser Ser Phe Leu Ser Pro Glu His Gln Lys Leu Gln Gly Cys
   1 5 10 15
<210> 126
   <211> 28
   <212> PRT
   <213> Artificial Sequence
<220>
   <223> mouse ghrelin peptide mutant
<400> 126
   Gly Ser Ser Phe Leu Ser Pro Glu His Gln Lys Ala Gln Arg Lys Glu
   1 5 10 15
   Ser Lys Lys Pro Pro Ala Lys Leu Gln Pro Arg Cys
   20 25
<210> 127
   <211> 29
   <212> PRT
   <213> Artificial sequence
<220>
   <223> mouse ghrelin peptide mutant
<400> 127
   Gly Ser Ser Phe Leu Ser Pro Glu His Gln Lys Ala Gln Arg Lys Glu
   1 5 10 15
   Ser Lys Lys Pro Pro Ala Lys Leu Gln Pro Arg Gly Cys
   20 25
<210> 128
   <211> 29
   <212> PRT
   <213> Artificial Sequence
<220>
   <223> mouse ghrelin peptide mutant
<400> 128
   Gly Ser Ser Phe Leu Ser Pro Glu His Gln Lys Ala Gln Gln Arg Lys
   1 5 10 15
   Glu Ser Lys Lys Pro Pro Ala Lys Leu Gln Pro Arg Cys
   20 25
<210> 129
   <211> 30
   <212> PRT
   <213> Artificial sequence
<220>
   <223> mouse ghrelin peptide mutant
<400> 129
   Gly Ser Ser Phe Leu Ser Pro Glu His Gln Lys Ala Gln Gln Arg Lys
   1 5 10 15
   Glu Ser Lys Lys Pro Pro Ala Lys Leu Gln Pro Arg Gly Cys
   20 25 30
<210> 130
   <211> 14
   <212> PRT
   <213> Artificial Sequence
<220>
   <223> mouse ghrelin peptide mutant
<400> 130
   Gly Ser Ser Phe Leu Ser Pro Glu His Gln Lys Ala Gln Cys
   1 5 10
<210> 131
   <211> 15
   <212> PRT
   <213> Artificial Sequence
<220>
   <223> mouse ghrelin peptide mutant
<400> 131
   Gly Ser Ser Phe Leu Ser Pro Glu His Gln Lys Ala Gln Gly Cys
   1 5 10 15
<210> 132
   <211> 13
   <212> PRT
   <213> Artificial Sequence
<220>
   <223> Ghrel24-33C
<400> 132
   Gly Gly Ser Ser Phe Leu Ser Pro Glu His Gln Gly Cys
   1 5 10
<210> 133
   <211> 11
   <212> PRT
   <213> Artificial Sequence
<220>
   <223> cGhrel42-51
<400> 133
   Cys Lys Lys Pro Pro Ala Lys Leu Gln Pro Arg
   1 5 10
<210> 134
   <211> 11
   <212> PRT
   <213> Artificial sequence
<220>
   <223> cGhrel31-40
<400> 134
   Cys Glu His Gln Lys Ala Gln Gln Arg Lys Glu
   1 5 10
<210> 135
   <211> 12
   <212> PRT
   <213> Artificial Sequence
<220>
   <223> cGhrel31-41
<400> 135
   Cys Glu His Gln Lys Ala Gln Gln Arg Lys Glu Ser
   1 5 10
<210> 136
   <211> 11
   <212> PRT
   <213> Artificial Sequence
<220>
   <223> cGhrel28-37
<400> 136
   Cys Leu Ser Pro Glu His Gln Lys Ala Gln Gln
   1 5 10
<210> 137
   <211> 12
   <212> PRT
   <213> Artificial Sequence
<220>
   <223> human ghrelin peptide mutant
<400> 137
   Cys Glu His Gln Arg Val Gln Gln Arg Lys Glu Ser
   1 5 10
<210> 138
   <211> 11
   <212> PRT
   <213> Artificial sequence
<220>
   <223> human ghrelin peptide mutant
<400> 138
   Cys Leu Ser Pro Glu His Gln Arg Val Gln Gln
   1 5 10
<210> 139
   <211> 25
   <212> DNA
   <213> Artificial Sequence
<220>
   <223> primer p1.44
<400> 139 25
   aaccatggca aataagccaa tgcaa 25
<210> 140
   <211> 30
   <212> DNA
   <213> Artificial sequence
<220>
   <223> primer p1.45
<400> 140
   aatctagaat tttctgcgca cccatcccgg 30
<210> 141
   <211> 31
   <212> DNA
   <213> Artificial sequence
<220>
   <223> primer p1.46
<400> 141
   aaaagcttaa gcagtagtat cagacgatac g 31
<210> 142
   <211> 43
   <212> DNA
   <213> Artificial sequence
<220>
   <223> primer p1.47
<400> 142
   gagtgatcca actcgtttat caactacatt ttcagcaagt ctg 43
<210> 143
   <211> 43
   <212> DNA
   <213> Artificial Sequence
<220>
   <223> primer p1.48
<400> 143
   cagacttgct gaaaatgtag ttgataaacg agttggatca ctc 43
<210> 144
   <211> 117
   <212> PRT
   <213> homo sapiens
<400> 144
   Met Pro Ser Pro Gly Thr Val Cys Ser Leu Leu Leu Leu Gly Met Leu
   1               5                   10                  15
   Trp Leu Asp Leu Ala Met Ala Gly Ser Ser Phe Leu Ser Pro Glu His
               20                  25                  30
   Gln Arg Val Gln Gln Arg Lys Glu Ser Lys Lys Pro Pro Ala Lys Leu
           35                  40                  45
   Gln Pro Arg Ala Leu Ala Gly Trp Leu Arg Pro Glu Asp Gly Gly Gln
       50                  55                  60
   Ala Glu Gly Ala Glu Asp Glu Leu Glu Val Arg Phe Asn Ala Pro Phe
   65                  70                  75                  80
   Asp Val Gly Ile Lys Leu Ser Gly Val Gln Tyr Gln Gln His Ser Gln
                   85                  90                  95
   Ala Leu Gly Lys Phe Leu Gln Asp Ile Leu Trp Glu Glu Ala Lys Glu
               100                 105                 110
   Ala Pro Ala Asp Lys
           115
<210> 145
   <211> 117
   <212> PRT
   <213> Canis familiaris
<400> 145
   Met Pro Ser Pro Gly Thr Val Cys Ser Leu Leu Leu Leu Gly Met Leu
   1 5 10 15
   Trp Leu Asp Leu Ala Met Ala Gly Ser Ser Phe Leu Ser Pro Glu His
   20 25 30
   Gln Lys Leu Gln Gln Arg Lys Glu Ser Lys Lys Pro Pro Ala Lys Leu
   35 40 45
   Gln Pro Arg Ala Leu Ala Gly Trp Leu Arg Pro Glu Asp Gly Gly Gln
   50 55 60
   Ala Glu Gly Ala Glu Asp Glu Leu Glu Val Arg Phe Asn Ala Pro Phe
   65 70 75 80
   Asp Val Gly Ile Lys Leu Ser Gly Val Gln Tyr Gln Gln His Ser Gln
   85 90 95
   Ala Leu Gly Lys Phe Leu Gln Asp Ile Leu Trp Glu Glu Ala Lys Glu
   100 105 110
   Ala Pro Ala Asp Lys
   115
<210> 146
   <211> 117
   <212> PRT
   <213> Mus musculus
<400> 146
   Met Pro Ser Pro Gly Thr Val Cys Ser Leu Leu Leu Leu Gly Met Leu
   1               5                   10                  15
   Trp Leu Asp Leu Ala Met Ala Gly Ser Ser Phe Leu Ser Pro Glu His
               20                  25                  30
   Gln Lys Ala Gln Gln Arg Lys Glu Ser Lys Lys Pro Pro Ala Lys Leu
           35                  40                  45
   Gln Pro Arg Ala Leu Ala Gly Trp Leu Arg Pro Glu Asp Gly Gly Gln
       50                  55                  60
   Ala Glu Gly Ala Glu Asp Glu Leu Glu Val Arg Phe Asn Ala Pro Phe
   65                  70                  75                  80
   Asp Val Gly Ile Lys Leu Ser Gly Val Gln Tyr Gln Gln His Ser Gln
                   85                  90                  95
   Ala Leu Gly Lys Phe Leu Gln Asp Ile Leu Trp Glu Glu Ala Lys Glu
               100                 105                 110
   Ala Pro Ala Asp Lys
           115